# EUROPEAN PATENT SPECIFICATION

(11) **EP 4 556 433 B1**
(45) Date of publication and mention of the grant of the patent: **25.11.2026**
(21) Application number: 25151767.8
(22) Date of filing: 22.02.2018
(51) Int. Cl.: B82Y 10/00, G06F 21/62, C12Q 1/68, G11C 11/54, G11C 13/00, G16B 50/30, G16B 50/40

(54) **NUCLEIC ACID BASED DATA STORAGE**
NUKLEINSÄUREBASIERTE DATENSPEICHERUNG
STOCKAGE DE DONNÉES À BASE D'ACIDE NUCLÉIQUE

(30) Priority: 22.02.2017 US 201762462284 P
(43) Date of publication of application: 21.05.2025
(62) Document number(s) of the earlier application(s) in accordance with Art. 76 EPC: 18756866.2 / 3 586 255
(73) Proprietor: Atlas Data Storage, Inc., Dover, Delaware 19901 (US)
(72) Inventor: PECK, Bill James, Santa Clara, CA 95051 (US); ZEITOUN, Ramsey Ibrahim, San Francisco, CA 94107 (US)
(74) Representative: Brand Murray Fuller LLP

(56) References cited:
- WO-A1-2017/011492
- WO-A1-2017/053450
- WO-A2-2015/021080
- GEORGE DVORSKY: "Living Bacteria Can Now Store Data", GIZMODO, 10 June 2016 (2016-06-10), pages 1 - 3, XP055537138, Retrieved from the Internet <URL:https://gizmodo.com/living-bacteria-can-now-store-data-1781773517> [retrieved on 20181220]
- YAZDI S M HOSSEIN TABATABAEI ET AL: "DNA-Based Storage: Trends and Methods", IEEE TRANSACTIONS ON MOLECULAR, BIOLOGICAL AND MULTI-SCALE COMMUNICATIONS, IEEE, vol. 1, no. 3, 2 March 2016 (2016-03-02), pages 230 - 248, XP011605293, DOI: 10.1109/TMBMC.2016.2537305

## Description

### CROSS-REFERENCE

This application claims the benefit of U.S. Provisional Application No. 62/462,284 filed on February 22, 2017.

### BACKGROUND

Biomolecule based information storage systems, *e*.*g*., DNA-based, have a large storage capacity and stability over time. However, there is a need for scalable, automated, highly accurate and highly efficient systems for biomolecules for information storage. In addition, there is a need for protecting the security of such information. Further biomolecule based information storage systems are known from publications WO 2017/011492 A1, WO 2017/053450 A1, George Dvorsky: "Living Bacteria Can Now Store Data", 10 June 2016, XP055537138 and Yazdi S M Hossein TabaTabaei et al: "DNA-Based Storage: Trends and Methods", IEEE, vol.1, no.3, 2 March 2016, XP011605293.

### BRIEF SUMMARY

The present invention is set out in independent claim 1. Preferred aspects are defined in dependent claims 2-14. In the following, only embodiments or examples comprising all the features of claim 1 are falling under the scope of protection of the present invention.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:
**Figure 1** illustrates an exemplary workflow for nucleic acid-based data storage.
**Figure 2** illustrates an exemplary workflow for storage for bioencryption.
**Figure 3** illustrates an exemplary workflow for retrieval following bioencryption.
**Figures 4A-4B** depict a bioencryption method using a Cas enzyme.
**Figures 5A-5C** depict various oligonucleotide sequence design schemes.
**Figures 6A-6C** depict various oligonucleotide sequence design schemes.
**Figures 7A-7B** depict a barcode design scheme.
**Figure 8** illustrates a plate configured for oligonucleotide synthesis comprising 24 regions, or sub-fields, each having an array of 256 clusters.
**Figure 9** illustrates a closer view of the sub-field in **FIG. 8** having 16 x16 of clusters, each cluster having 121 individual loci.
**Figure 10** illustrates a detailed view of the cluster in **FIG. 8****,** where the cluster has 121 loci.
**Figure 11A** illustrates a front view of a plate with a plurality of channels.
**Figure 11B** illustrates a sectional view of plate with a plurality of channels.
**Figures 12A-12B** depict a continuous loop and reel-to-reel arrangements for flexible structures.
**Figures 13A-13C** depict a zoom in of a flexible structure, having flat features (loci), channels, or wells, respectively.
**Figure 14A** illustrates a zoom in of features on a structure described herein.
**Figures 14B-14C** illustrate markings on structures described herein.
**Figure 15** illustrates an oligonucleotide synthesis material deposition device.
**Figure 16** illustrates an oligonucleotide synthesis workflow.
**Figure 17** illustrates an example of a computer system.
**Figure 18** is a block diagram illustrating architecture of a computer system.
**Figure 19** is a diagram demonstrating a network configured to incorporate a plurality of computer systems, a plurality of cell phones and personal data assistants, and Network Attached Storage (NAS).
**Figure 20** is a block diagram of a multiprocessor computer system using a shared virtual address memory space.

### DETAILED DESCRIPTION

### Definitions

Unless defined otherwise, all technical and scientific terms used herein have the same meaning as is commonly understood by one of ordinary skill in the art to which these inventions belong.

Throughout this disclosure, various embodiments are presented in a range format. It should be understood that the description in range format is merely for convenience and brevity and should not be construed as an inflexible limitation on the scope of any embodiments. Accordingly, the description of a range should be considered to have specifically disclosed all the possible subranges as well as individual numerical values within that range to the tenth of the unit of the lower limit unless the context clearly dictates otherwise. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual values within that range, for example, 1.1, 2, 2.3, 5, and 5.9. This applies regardless of the breadth of the range. The upper and lower limits of these intervening ranges may independently be included in the smaller ranges, and are also encompassed within the invention, subject to any specifically excluded limit in the stated range. Where the stated range includes one or both of the limits, ranges excluding either or both of those included limits are also included in the invention, unless the context clearly dictates otherwise.

The terminology used herein is for the purpose of describing particular embodiments only and is not intended to be limiting of any embodiment. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items.

Unless specifically stated or obvious from context, as used herein, the term "about" in reference to a number or range of numbers is understood to mean the stated number and numbers +/- 10% thereof, or 10% below the lower listed limit and 10% above the higher listed limit for the values listed for a range.

The term "oligonucleotide" as used herein is used interchangeably with "oligonucleic acid." The terms "oligonucleotide" and "oligonucleic acid" encompass double- or triple-stranded nucleic acids, as well as single-stranded molecules.

### Nucleic Acid Based Information Storage

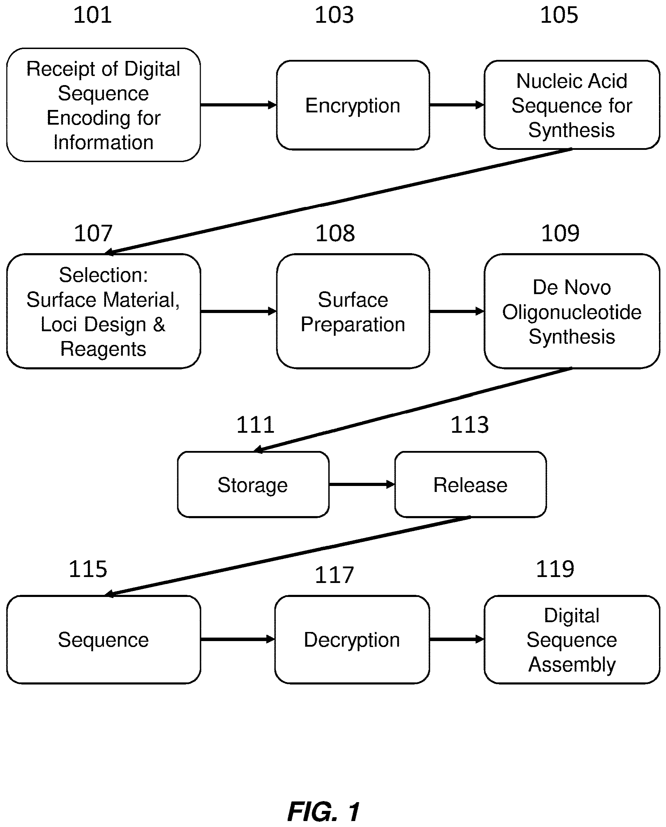

Provided herein are devices, compositions, systems, and methods for nucleic acid-based information (data) storage. An exemplary workflow is provided in **FIG. 1****.** In a first step, a digital sequence encoding an item of information (i.e., digital information in a binary code for processing by a computer) is received **101.** An encryption **103** scheme is applied to convert the digital sequence from a binary code to a nucleic acid sequence **105.** A surface material for nucleic acid extension, a design for loci for nucleic acid extension (aka, arrangement spots), and reagents for nucleic acid synthesis are selected **107.** The surface of a structure is prepared for nucleic acid synthesis **108.** De novo oligonucleotide synthesis is performed **109.** The synthesized oligonucleotides are stored **111** and available for subsequent release **113,** in whole or in part. Once released, the oligonucleotides, in whole or in part, are sequenced **115,** and subject to decryption **117** to convert the nucleic sequence back to the digital sequence. The digital sequence is then assembled **119** to obtain an alignment encoding for the original item of information.

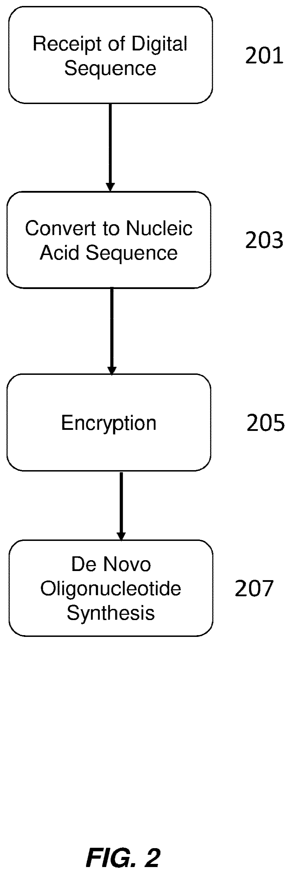

Further provided herein are methods and systems for secured DNA-based information storage including receipt of one or more digital sequences encoding for at least one item of information **201,** conversion of the one or more digital sequences to a nucleic acid sequence **203,** encryption of the nucleic acid sequence **205,** and de novo oligonucleotide synthesis of the encrypted nucleic acid sequence **207.** *See* **FIG. 2****.**

Provided herein are devices, compositions, systems, and methods for nucleic acid-based information storage, wherein machine instructions are received for conversion from a digital sequence to a nucleic acid sequence, bioencryption, biodecryption, or a combination of any of these steps. Machine instructions may be received for desired items of information for conversion and for one or more types of bioencryption selected from a list of options, for example, without limitation, enzymatic based (e.g., CRISPR/Cas complex or restriction enzyme digest), electromagnetic radiation based (e.g., photolysis or photodetection), chemical cleavage (e.g, gaseous ammonia or methylamine treatment to cleave Thymidine-succinyl hexamide CED phosphoramidite (CLP-2244 from ChemGenes)), and affinity based (e.g., a sequence tag for hybridization, or incorporation of modified nucleotides with enhanced affinity to a capture reagent) forms of bioencryption. Following receipt of a particular bioencryption selection, a program module performs the step of converting the item of information to nucleic acid sequence and applying design instructions for design of a bioencrypted version of the sequence, before providing synthesis instructions to a material deposition device for de novo synthesis of oligonucleotides. In some instances, machine instructions for selection of one or more species within a category of bioencryption are provided.

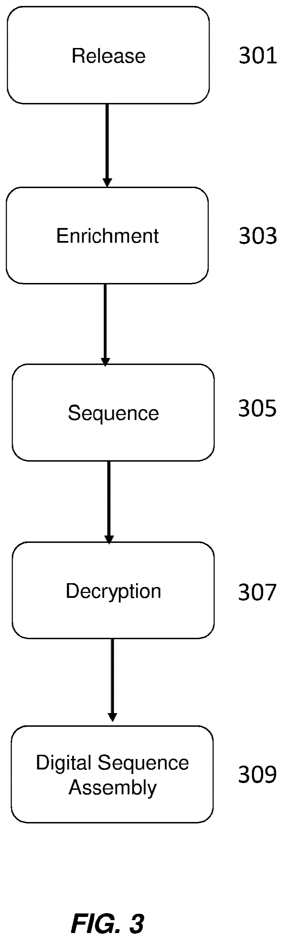

Further provided herein are methods and systems for secured DNA-based information retrieval including release of oligonucleotides from a surface **301,** enrichment of desired oligonucleotides **303,** sequencing of the oligonucleotides **305,** decryption of the nucleic acid sequence **307,** and assembly of one or more digital sequences encoding for an item of information ***309.** See* **FIG. 3****.**

Machine instructions as described herein may also be provided for biodecryption. Biodecryption may comprise receipt of machine instructions. Such instructions may include one or more formats of biodecryption selected from a list of options, for example, without limitation, enzymatic based (e.g., CRISPR/Cas complex or restriction enzyme digest), electromagnetic radiation based (e.g., photolysis or photodetection), chemical cleavage (e.g, gaseous ammonia or methylamine treatment to cleave Thymidine-succinyl hexamide CED phosphoramidite (CLP-2244 from ChemGenes)), and affinity based (e.g., nucleic acid sequences for hybridization, or incorporation of modified nucleotides with enhanced affinity to a capture reagent) forms of biodecryption of the oligonucleotides. Following receipt of a particular biodecryption selection, a program module performs the step releasing the modulatory agent(s) for enrichment of the oligonucleotides. Following enrichment, the oligonucleotides are sequenced, optionally aligned to a longer nucleic acid sequence, and converted to a digital sequence corresponding to an item of information. In some instances, machine instructions for selection of one or more species within a category of biodecryption are provided.

### Items of Information

Optionally, an early step of a DNA data storage process disclosed herein includes obtaining or receiving one or more items of information in the form of an initial code (e.g., digital sequence). Items of information include, without limitation, text, audio and visual information. Exemplary sources for items of information include, without limitation, books, periodicals, electronic databases, medical records, letters, forms, voice recordings, animal recordings, biological profiles, broadcasts, films, short videos, emails, bookkeeping phone logs, internet activity logs, drawings, paintings, prints, photographs, pixelated graphics, and software code. Exemplary biological profile sources for items of information include, without limitation, gene libraries, genomes, gene expression data, and protein activity data. Exemplary formats for items of information include, without limitation, .txt, .PDF, .doc, .docx, .ppt, .pptx, .xls, .xlsx, .rtf, .jpg, .gif, .psd, .bmp, .tiff, .png, and .mpeg. The amount of individual file sizes encoding for an item of information, or a plurality of files encoding for items of information, in digital format include, without limitation, up to 1024 bytes (equal to 1 KB), 1024 KB (equal to 1MB), 1024 MB (equal to 1 GB), 1024 GB (equal to 1TB), 1024 TB (equal to 1PB), 1 exabyte, 1 zettabyte, 1 yottabyte, 1 xenottabyte or more. In some instances, an amount of digital information is at least or about 1 gigabyte (GB). In some instances, the amount of digital information is at least or about 1, 2, 3, 4, 5, 6, 7, 8, 9, 10, 20, 50, 100, 200, 300, 400, 500, 600, 700, 800, 900, 1000 or more than 1000 gigabytes. In some instances, the amount of digital information is at least or about 1 terabyte (TB). In some instances, the amount of digital information is at least or about 1, 2, 3, 4, 5, 6, 7, 8, 9, 10, 20, 50, 100, 200, 300, 400, 500, 600, 700, 800, 900, 1000 or more than 1000 terabytes. In some instances, the amount of digital information is at least or about 1 petabyte (PB). In some instances, the amount of digital information is at least or about 1, 2, 3, 4, 5, 6, 7, 8, 9, 10, 20, 50, 100, 200, 300, 400, 500, 600, 700, 800, 900, 1000 or more than 1000 petabytes.

### Encryption

### Biological Encryption and Decryption

Described herein are devices, compositions, systems, and methods comprising biological encryption (aka "bioencryption") following receipt of a digital sequence encoding an item of information. In addition to individual forms of bioencryption and biodecryption described herein, also provided herein are processes for incorporating the selection of one or more classes or species of masking a biological sequence into a workflow for information storage and/or retrieval.

Provided herein are devices, compositions, systems, and methods of target enrichment of a nucleic acid sequence of interest from a larger population of nucleic acid sequences comprising biological encryption. In some instances, biological encryption is used to enrich a target signal from noise. In some instances, the target signal is a nucleic acid sequence of interest. In some instances, biological encryption comprises introducing the nucleic acid sequence of interest into a larger population of nucleic acid sequences with known sequences. The known nucleic acid sequences can be referred to as encryption nucleic acid sequences. In some instances, the encryption nucleic acids are decrypted. In some instances, decryption of the known nucleic acid sequences results in an increase in signal-to-noise ratio of the nucleic acid sequence of interest.

Provided herein are devices, compositions, systems, and methods comprising incorporation of biological molecule encryption in an information storage and/or retrieval workflow. Exemplary forms of bioencryption and biodecryption include, without limitation, enzymatic based, electromagnetic radiation based, chemical cleavage, and affinity based bioencryption and biodecryption.

Provided herein are devices, compositions, systems, and methods comprising application of nuclease complex activity based encryption. Exemplary nucleases include, without limitation, a Cas nuclease (CRISPR associated), a Zinc Finger Nuclease (ZFNs), a Transcription Activator-Like Effector Nucleases, an Argonaute nuclease, S1 Nuclease, mung bean nuclease, or a DNAse. Exemplary Cas nucleases include, without limitation, Cas1, CaslB, Cas2, Cas3, Cas4, Cas5, Cas6, Cas7, Cas8, Cas9, Cas10, Csy1 , Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx1S, Csf1, Csf2, CsO, Csf4, Cpf1, c2c1, and c2c3. In some instances, the Cas nuclease is Cas9. In some instances, a CRISPR/Cas complex provides for predetermined removal of one or more nucleic acid sequences. In some instances, enrichment steps described herein comprises depletion of abundant sequences by hybridization (DASH). In some instances, the DASH comprises application of a nuclease. For example, a nuclease such as Cas9, when bound to a CRISPR complex including a guide RNA ("gRNA") sequence, induces a stranded break such that a longer form of a nucleic acid sequence is no longer intact. In some instances, excised nucleic acids are unavailable for subsequent amplification following enrichment. In some instances, gRNA shepherds the Cas9 enzyme to a specific stretch of nucleic acids. In alternative arrangements, a gRNA has multiple sites for cleavage. A gRNA-based system allows for generation of an encryption code with high specificity and selectivity. For example, since a CRISPR/Cas9 based system uses 20 bp to identify a sequence to cleave, at least about 10^12 different possibilities are available for designing a predetermined gRNA sequence for decryption using a 4 base system. Following removal of extraneous (aka "junk") DNA, the predetermined oligonucleotides encoding for a target sequence are subject to downstream processing, e.g., amplification and sequencing, resulting in a final sequence without the extra (junk) sequence. In some instances, each oligonucleotide of the plurality of oligonucleotides is designed for modification (e.g., cleavage, base swapping, recombination) at multiple locations. For example, each oligonucleotide of the plurality of oligonucleotides is synthesized with complementary regions for binding to about 1, 2, 3, 4, 5, 6, 7, 8, 9, 10 or more gRNA sequences. In such an arrangement, each of the plurality of oligonucleotides is subject to cleavage, base swapping, recombination subsequent to nuclease (e.g., CRISPR/Cas) complex activity at 1, 2, 3, 4, 5, 6, 7, 8, 9, 10 or more locations.

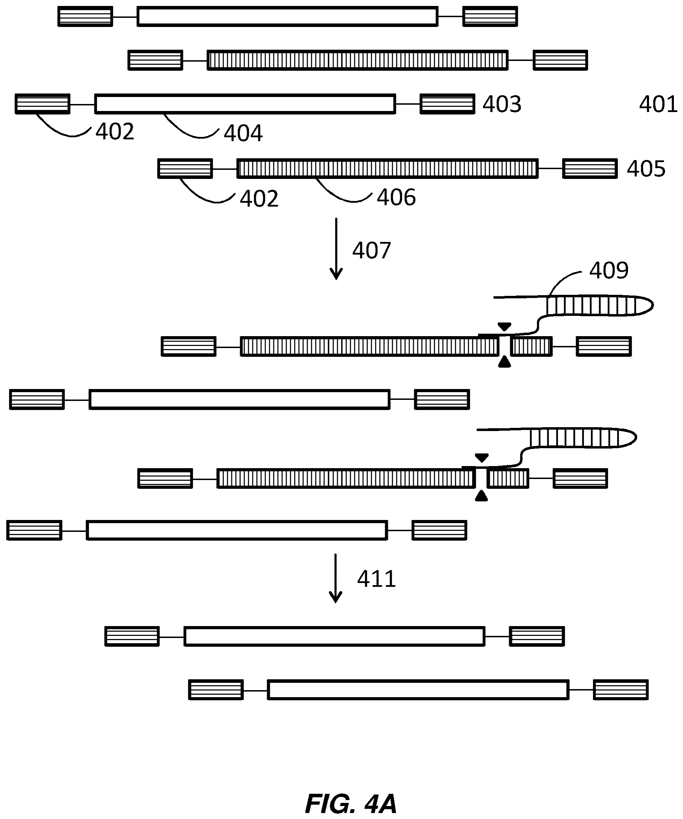

A first process for target enrichment for data encryption using CRISPR/Cas9 is illustrated in **FIG. 4A****.** A population of DNA sequences **401** comprises DNA information **403** and encrypted DNA **405.** The DNA information **403** and encrypted DNA **405** comprise adapter sequences **402** and DNA sequences **404, 406,** respectively. Guide RNAs **409** are added **407** to the population of DNA sequences **401.** The guide RNAs **409** are used to remove the encrypted DNA **405** by recognizing cleavage sequences encrypted in the encrypted DNA **405.** Following addition of the guide RNAs **409,** the encrypted DNA **405** is cleaved resulting in a nucleic acid sequence no longer intact. The encrypted DNA **405** are thus removed **411** from the population leaving the DNA information **403,** for example, when the encrypted DNA **405** comprising a nucleic acid sequence that is no longer intact is unable to be amplified.

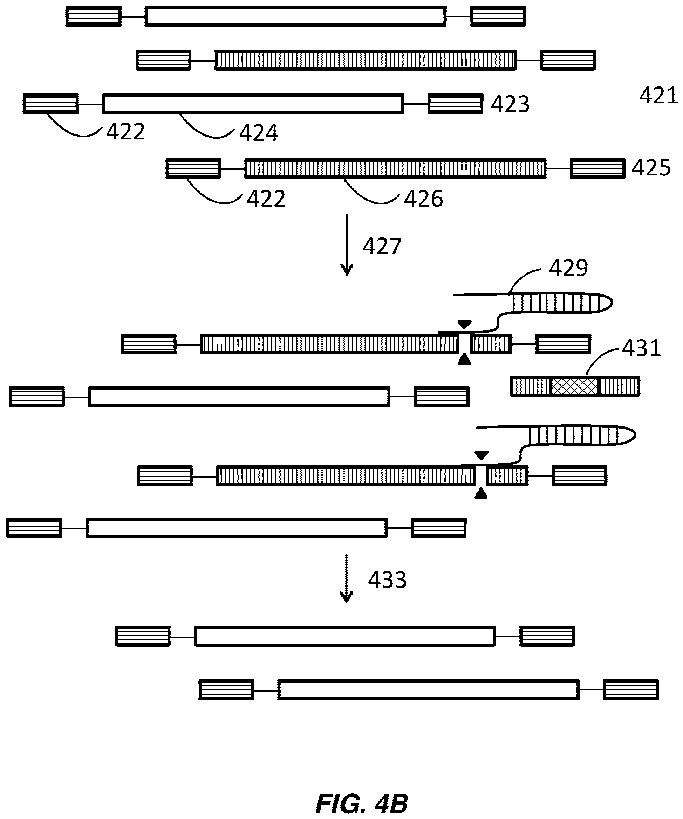

A second process for target enrichment for data encryption using CRISPR/Cas9 is illustrated in **FIG. 4B****.** A population of DNA sequences **421** comprises DNA information **423** and encrypted DNA **425.** The DNA information **423** and encrypted DNA **425** comprise adapter sequences **422** and DNA sequences **424, 426,** respectively. Guide RNAs **429** and donor DNA **431** are added **427** to the population of DNA sequences **421.** The guide RNAs **429** recognize an encrypted cleavage site in the encrypted DNA **425** and generate a cleavage site for insertion of the donor DNA **431.** Insertion of the donor DNA **431** results in an insertion or frameshift in the encrypted DNA **425.** In some instances, insertion of the donor DNA **431** results in introduction of a sequence tag for hybridization or incorporation of modified nucleotides with enhanced affinity to a capture reagent. For example, the donor DNA **431** is recognized by a fluorescent probe. In some instances, the donor DNA **431** introduces a sequence for electromagnetic radiation based (e.g., photolysis or photodetection) or chemical cleavage based (e.g, gaseous ammonia or methylamine treatment to cleave Thymidine-succinyl hexamide CED phosphoramidite (CLP-2244 from ChemGenes)) bioencryption and/or biodecryption. In some instances, the encrypted DNA **425** is no longer recognized for amplification, and only DNA information **423** is amplified resulting in enrichment of the DNA information **423.**

Devices, compositions, systems, and methods comprising application of nuclease complex activity based encryption as described herein may comprise base swapping or sequence swapping. For example, bioencryption and biodecryption using CRISPR/Cas comprises base swapping or sequence swapping. In some instances, bioencryption comprises a CRISPR/dCas9 where a disabled or "dead" Cas9 ("dCas9") no longer has a splicing function but, with the addition of another enzymatic activity, performs a different target molecule modifying function. For example, tethering a cytidine deaminase to dCas9 converts a C-G DNA base pair into T-A base pair. In an alternative dCas9 process, a different enzyme tethered to the dCas9 results in changing the base C into a T, or a G to an A in a target DNA.

Provided herein are devices, compositions, systems, and methods for bioencryption and biodecryption comprising application of a restriction enzyme. In some instances, the restriction enzyme targets an enzyme recognition site. In some instances, the enzyme recognition site is a specific nucleotide sequence. In some instances, the restriction enzyme cleaves the phosphate backbone at or near the enzyme recognition site. In some instances, cleavage of the recognition site results in a non-blunt end or a blunt end. In some instances, the restriction enzyme recognizes a nucleotide (e.g., A, T, G, C, U). In some instances, the restriction enzyme recognizes a modification such as, but not limited to, methylation, hydroxylation, or glycosylation. In some instances, the restriction enzyme results in fragmentation. In some instances, fragmentation produces fragments having 5' overhangs, 3' overhangs, blunt ends, or a combination thereof. In some instances, the fragments are selected, for example, based on size. In some instances, fragmentation by a restriction enzyme is followed by ligation. For example, fragmentation by a restriction enzyme is used to leave a predictable overhang, followed by ligation with one or more adapter oligonucleotides comprising an overhang complementary to the predictable overhang on a nucleic acid fragment. Exemplary restriction enzymes and their recognition sequences are provided in **Table 1.**

**Table 1. Restriction Enzymes**

| **Table 1** | |
|---|---|
| **Recognition Sequence** | **Enzyme** |
| AA/CGTT | AclI |
| A/AGCTT | HindIII HindIII-HF^{®} |
| AAT/ATT | SspI SspI-HF^{®} |
| /AATT | MluCI Tsp509I |
| A/CATGT | PciI |
| A/CCGGT | AgeI AgeI-HF^{®} |
| ACCTGC(4/8) | BspMI BfuAI |
| A/CCWGGT | SexAI |
| A/CGCGT | MluI MluI-HF^{®} |
| ACGGC(12/14) | BceAI |
| A/CGT | HpyCH4IV |
| ACN/GT | HpyCH4III |
| (10/15)ACNNNNGTAYC(12/7) (SEQ ID NO: 5) | BaeI |
| (9/12)ACNNNNNCTCC(10/7) (SEQ ID NO: 6) | BsaXI |
| A/CRYGT | AflIII |
| A/CTAGT | SpeI SpeI-HF^{®} |
| ACTGG(1/-1) | BsrI |
| ACTGGG(5/4) | BmrI |
| A/GATCT | BglII |
| AGC/GCT | AfeI |
| AG/CT | AluI |
| AGG/CCT | StuI |
| AGT/ACT | ScaI ScaI-HF^{®} |
| AT/CGAT | ClaI BspDI |
| ATCTATGTCGGGTGCGGAGAAAGAGGTAAT(-15/-19) (SEQ ID NO: 7) | PI-SceI |
| ATGCA/T | NsiI NsiI-HF^{®} |
| AT/TAAT | AseI |
| ATTT/AAAT | SwaI |
| (11/13)CAANNNNNGTGG(12/10) (SEQ ID NO: 8) | CspCI |
| C/AATTG | MfeI MfeI-HF^{®} |
| CACGAG(-5/-1) | BssSI BssSαI |
| CACGAG | Nb.BssSI |
| CACGTC(-3/-3) | BmgBI |
| CAC/GTG | PmlI |
| CACNNN/GTG | DraIII DraIII-HF^{®} |
| CACNN/NNGTG (SEQ ID NO: 9) | AleI |
| CAGCAG(25/27) | EcoP151 |
| CAG/CTG | PvuII PvuII-HF^{®} |
| CAGNNN/CTG | AlwNI |
| CAGTG(2/0) | BtsIMutI |
| NNCASTGNN/ | TspRI |
| CA/TATG | NdeI |
| CATG/ | NlaIII |
| C/ATG | CviAII |
| /CATG | FatI |
| CAYNN/NNRTG (SEQ ID NO: 10) | MslI |
| CC(12/16) | FspEI |
| CCANNNNN/NNNNTGG (SEQ ID NO: 11) | XcmI |
| CCANNNNN/NTGG (SEQ ID NO: 12) | BstXI |
| CCANNNN/NTGG (SEQ ID NO: 13) | PflMI |
| CCATC(4/5) | BccI |
| C/CATGG | NcoI NeoI-HF^{®} |
| CCCAGC(-5/-1) | BseYI |
| CCCGC(4/6) | FauI |
| CCC/GGG | SmaI |
| C/CCGGG | XmaI TspMI |
| (0/-1)CCD | Nt.CviPII |
| CCDG(10/14) | LpnPI |
| CCGC(-3/-1) | AciI |
| CCGC/GG | SacII |
| CCGCTC(-3/-3) | BsrBI |
| C/CGG | MspI HpaII |
| CC/NGG | ScrFI |
| /CCNGG | BssKI StyD4I |
| C/CNNGG | BsaJI |
| CCNNNNN/NNGG (SEQ ID NO: 14) | BslI |
| C/CRYGG | BtgI |
| CC/SGG | NeiI |
| C/CTAGG | AvrII |
| CCTC(7/6) | MnlI |
| CCTCAGC(-5/-2) | BbvCI |
| CCTCAGC | Nb.BbvCI |
| CCTCAGC(-5/-7) | Nt.BbvCI |
| CCTGCA/GG | SbfI SbfI-HF^{®} |
| CCTNAGC(-5/-2) | Bpu10I |
| CC/TNAGG | Bsu361 |
| CCTNN/NNNAGG (SEQ ID NO: 15) | EcoNI |
| CCTTC(6/5) | HpyAV |
| CC/WGG | BstNI |
| /CCWGG | PspGI |
| C/CWWGG | StyI StyI-HF^{®} |
| (10/12)CGANNNNNNTGC(12/10) (SEQ ID NO: 16) | BcgI |
| CGAT/CG | PvuI PvuI-HF^{®} |
| CG/CG | BstUI |
| C/GGCCG | EagI EagI-HF^{®} |
| CG/GWCCG | RsrII |
| CGRY/CG | BsiEI |
| C/GTACG | BsiWI BsiWI-HF^{®} |
| CGTCTC(1/5) | BsmBI |
| CGWCG/ | Hpy99I |
| CMG/CKG | MspA1I |
| CNNR(9/13) | MspJI |
| CR/CCGGYG | SgrAI |
| C/TAG | BfaI |
| CTCAG(9/7) | BspCNI |
| C/TCGAG | XhoI PaeR71 TliI |
| CTCTTC(1/4) | EarI |
| CTGAAG(16/14) | AcuI |
| CTGCA/G | PstI PstI-HF^{®} |
| CTGGAG(16/14) | BpmI |
| C/TNAG | DdeI |
| C/TRYAG | SfcI |
| C/TTAAG | AflII |
| CTTGAG(16/14) | BpuEI |
| C/TYRAG | SmlI |
| C/YCGRG | AvaI BsoBI |
| GAAGA(8/7) | MboII |
| GAAGAC(2/6) | Bbsl BbsI-HF^{®} |
| GAANN/NNTTC (SEQ ID NO: 17) | XmnI |
| GAATGC(1/-1) | BsmI |
| GAATGC | Nb.BsmI |
| G/AATTC | EcoRI EcoRI-HF^{®} |
| GACGC(5/10) | HgaI |
| GACGT/C | AatII |
| GAC/GTC | ZraI |
| GACN/NNGTC | Tth111I PflFI |
| GACNN/NNGTC (SEQ ID NO: 18) | PshAI |
| GACNNN/NNGTC (SEQ ID NO: 19) | AhdI |
| GACNNNN/NNGTC (SEQ ID NO: 20) | DrdI |
| GAG/CTC | Eco53kI |
| GAGCT/C | SacI SacI-HF^{®} |
| GAGGAG(10/8) | BseRI |
| GAGTC(4/5) | PleI |
| GAGTC(4/-5) | Nt.BstNBI |
| GAGTC(5/5) | MlyI |
| G/ANTC | HinfI |
| GAT/ATC | EcoRV EcoRV-HF^{®} |
| /GATC | MboI Sau3 AI DpnII BfuCI |
| GA/TC | DpnI |
| GATNN/NNATC (SEQ ID NO: 21) | BsaBI |
| G/AWTC | TfiI |
| GCAATG(2/0) | BsrDI |
| GCAATG | Nb.BsrDI |
| GCAGC(8/12) | BbvI |
| GCAGTG(2/0) | BtsI BtsαI |
| GCAGTG | Nb.BtsI |
| GCANNNN/NTGC (SEQ ID NO: 22) | BstAPI |
| GCATC(5/9) | SfaNI |
| GCATG/C | SphI SphI-HF^{®} |
| GCCC/GGGC | SrfI |
| GCCGAG(21/19) | NmeAIII |
| GCC/GGC | NaeI |
| G/CCGGC | NgoMIV |
| GCCNNNN/NGGC (SEQ ID NO: 23) | BglI |
| GCGAT/CGC | AsiSI |
| GCGATG(10/14) | BtgZI |
| G/CGC | HinP1I |
| GCG/C | HhaI |
| G/CGCGC | BssHII |
| GC/GGCCGC | NotI NotI-HF^{®} |
| GC/NGC | Fnu4HI |
| GCN/NGC | Cac8I |
| GCNNNNN/NNGC (SEQ ID NO: 24) | MwoI |
| G/CTAGC | NheI NheI-HF^{®} |
| GCTAG/C | BmtI BmtI-HF^{®} |
| GCTCTTC(1/4) | SapI BspQI |
| GCTCTTC(1/-7) | Nt.BspQI |
| GC/TNAGC | BlpI |
| G/CWGC | TseI ApeKI |
| GDGCH/C | Bsp1286I |
| GGATC(4/5) | AlwI |
| GGATC(4/-5) | Nt.AlwI |
| G/GATCC | BamHI BamHI-HF^{®} |
| GGATG(9/13) | FokI |
| GGATG(2/0) | BtsCI |
| GG/CC | HaeIII PhoI |
| GGCCGG/CC | FseI |
| GGCCNNNN/NGGCC (SEQ ID NO: 25) | SfiI |
| GG/CGCC | NarI |
| G/GCGCC | KasI |
| GGC/GCC | SfoI |
| GGCGC/C | PluTI |
| GG/CGCGCC | AscI |
| GGCGGA(11/9) | EciI |
| GGGAC(10/14) | BsmFI |
| GGGCC/C | ApaI |
| G/GGCCC | PspOMI |
| G/GNCC | Sau96I |
| GGN/NCC | NlaIV |
| GGTAC/C | KpnI KpnI-HF^{®} |
| G/GTACC | Acc65I |
| GGTCTC(1/5) | BsaI BsaI-HF^{®} |
| GGTGA(8/7) | HphI |
| G/GTNACC | BstEII BstEII-HF^{®} |
| G/GWCC | AvaII |
| G/GYRCC | BanI |
| GKGCM/C | BaeGI |
| GR/CGYC | BsaHI |
| GRGCY/C | BanII |
| GT/AC | RsaI |
| G/TAC | CviQI |
| GTA/TAC | BstZ17I |
| GTATAC | BstZ17I-HF^{®} |
| GTATCC(6/5) | BciVI |
| G/TCGAC | SalI SalI-HF^{®} |
| GTCTC(1/-5) | Nt.BsmAI |
| GTCTC(1/5) | BsmAI BcoDI |
| G/TGCAC | ApaLI |
| GTGCAG(16/14) | BsgI |
| GT/MKAC | AccI |
| GTN/NAC | Hpy166II |
| /GTSAC | Tsp45I |
| GTT/AAC | HpaI |
| GTTT/AAAC | PmeI |
| GTY/RAC | HincII |
| GWGCW/C | BsiHKAI |
| R/AATTY | ApoI ApoI-HF |
| RCATG/Y | NspI |
| R/CCGGY | BsrFI BsrFαI |
| R/GATCY | BstYI |
| RGCGC/Y | HaeII |
| RG/CY | CviKI-1 |
| RG/GNCCY | EcoO 1091 |
| RG/GWCCY | PpuMI |
| TAACTATAACGGTCCTAAGGTAGCGAA(-9/- 13) (SEQ ID NO: 26) | I-CeuI |
| TAC/GTA | SnaBI |
| TAGGGATAACAGGGTAAT(-9/-13) (SEQ ID NO: 27) | I-SceI |
| T/CATGA | BspHI |
| T/CCGGA | BspEI |
| TCCRAC(20/18) | MmeI |
| T/CGA | TaqαI |
| TCG/CGA | NruI NruI-HF^{®} |
| TCN/GA | Hpy188I |
| TC/NNGA | Hpy188III |
| T/CTAGA | XbaI |
| T/GATCA | BclI |
| TG/CA | HpyCH4V |
| TGC/GCA | FspI |
| TGGCAAACAGCTATTATGGGTATTATGGGT(-13/-17) (SEQ ID NO: 28) | PI-PspI |
| TGG/CCA | MscI |
| T/GTACA | BsrGI BsrGI-HF^{®} |
| T/TAA | MseI |
| TTAAT/TAA | PacI |
| TTA/TAA | PsiI |
| TT/CGAA | BstBI |
| TTT/AAA | DraI |
| VC/TCGAGB | PspXI |
| W/CCGGW | BsaWI |
| YAC/GTR | BsaAI |
| Y/GGCCR | EaeI |

Provided herein are devices, compositions, systems, and methods for bioencryption and biodecryption may comprise application of a repair enzyme. DNA repair enzymes, in some instances, are derived from a particular organism or virus or are non-naturally occurring variants thereof. Exemplary DNA repair enzymes include, but are not limited to, *E. coli* endonuclease IV, Tth endonuclease IV, human AP endonuclease, glycosylases, such as UDG, *E. coli 3-*methyladenine DNA glycoylase (AIkA) and human Aag, glycosylase/lyases, such as *E. coli* endonuclease III, *E. coli* endonuclease VIII, *E. coli* Fpg, human OGGl, and T4 PDG, and lyases. Exemplary additional DNA repair enzymes are listed in **Table 2.**

**Table 2. DNA Repair Enzymes.**

| **Gene Name** | **Activity** | **Accession Number** |
|---|---|---|
| UNG | Uracil-DNA glycosylase | NM_080911 |
| SMUG1 | Uracil-DNA glycosylase | NM_014311 |
| MBD4 | Removes U or T opposite G at CpG sequences | NM_003925 |
| TDG | Removes U, T or ethenoC opposite G | NM_003211 |
| OGG1 | Removes 8-oxoG opposite C | NM_016821 |
| MUTYH (MYH) | Removes A opposite 8-oxoG | NM_012222 |
| NTHL1 (NTH1) | Removes Ring-saturated or fragmented pyrimidines | NM_002528 |
| MPG | Removes 3-meA, ethenoA, hypoxanthine | NM_002434 |
| NEIL 1 | Removes thymine glycol | NM_024608 |
| NEIL2 | Removes oxidative products of pyrimidines | NM_145043 |
| XPC | Binds damaged DNA as complex with RAD23B, CETN2 | NM_004628 |
| RAD23B (HR23B) | Binds damaged DNA as complex with XPC, CETN2 | NM_002874 |
| CETN2 | Binds damaged DNA as complex with XPC, RAD23B | NM_004344 |
| RAD23A (HR23A) | Substitutes for HR23B | NM_005053 |
| XPA | Binds damaged DNA in preincision complex | NM_000380 |
| RPA1 | Binds DNA in preincision complex | NM_002945 |
| RPA2 | Binds DNA in preincision complex | NM_002946 |
| RPA3 | Binds DNA in preincision complex | NM_002947 |
| ERCC5 (XPG) | 3' incision | NM_000123 |
| ERCC1 | 5' incision subunit | NM_001983 |
| ERCC4 (XPF) | 5' incision subunit | NM_005236 |
| LIG1 | DNA joining | NM_000234 |
| CKN1(CSA) | Cockayne syndrome; Needed for transcription-coupled NER | NM_000082 |
| ERCC6 (CSB) | Cockayne syndrome; Needed for transcription-coupled NER | NM_000124 |
| XAB2 (HCNP) | Cockayne syndrome; Needed for transcription-coupled NER | NM_020196 |
| DDB1 | Complex defective in XP group E | NM_001923 |
| DDB2 | DDB1, DDB2 | NM_000107 |
| MMS19L (MMS19) | Transcription and NER | NM_022362 |
| FEN1 (DNase IV) | Flap endonuclease | NM_004111 |
| SPO11 | endonuclease | NM_012444 |
| FLJ35220 (ENDOV) | incision 3' of hypoxanthine and uracil | NM_173627 |
| FANCA | Involved in tolerance or repair of DNA crosslinks | NM_000135 |
| FANCB | Involved in tolerance or repair of DNA crosslinks | NM_152633 |
| FANCC | Involved in tolerance or repair of DNA crosslinks | NM_000136 |
| FANCD2 | Involved in tolerance or repair of DNA crosslinks | NM_033084 |
| FANCE | Involved in tolerance or repair of DNA crosslinks | NM_021922 |
| FANCF | Involved in tolerance or repair of DNA crosslinks | NM_022725 |
| FANCG (XRCC9) | Involved in tolerance or repair of DNA crosslinks | NM_004629 |
| FANCL | Involved in tolerance or repair of DNA crosslinks | NM_018062 |
| DCLRE1A (SNM1) | DNA crosslink repair | NM_014881 |
| DCLRE1B (SNM1B) | Related to SNM1 | NM_022836 |
| NEIL3 | Resembles NEIL1 and NEIL2 | NM_018248 |
| ATRIP (TREX1) | ATR-interacting protein 5' alternative ORF of the TREX1/ATRIP gene | NM_130384 |
| NTH | Removes damaged pyrimidines | NP_416150.1 |
| NEI | Removes damaged pyrimidines | NP_415242.1 |
| NFI | Deoxyinosine 3' endonuclease | NP_418426.1 |
| MUTM | Formamidopyrimidine DNA glycosylase | NP_418092.1 |
| UNG | Uracil-DNA glycosylase | NP_417075.1 |
| UVRA | DNA excision repair enzyme complex | NP_418482.1 |
| UVRB | DNA excision repair enzyme complex | NP_415300.1 |
| UVRC | DNA excision repair enzyme complex | NP_416423.3 |
| DENV | Pyrimidine dimer glycosylase | NP_049733.1 |

Provided herein are devices, compositions, systems, and methods for bioencryption and/or biodecryption comprising nucleic acid modification. In some instances, the nucleic acid modification impacts activity of nucleic acid sequences in a sequencing reaction. For example, the nucleic acid modification prevents the encrypted nucleic acid sequences from being amplified. In some instances, the nucleic acid modification comprises, but is not limited to, methylated bases, PNA (peptide nucleic acid) nucleotides, LNA (locked nucleic acid) nucleotides, and 2'-O-methyl-modified nucleotides. In some instances, the nucleic acid modification comprises a modified nucleobase that is not a cytosine, guanine, adenine or thymine. Non-limiting modified nucleobases include, without limitation, uracil, 3-meA (3-methyladenine), hypoxanthine, 8-oxoG (7,8-dihydro-8-oxoguanine), FapyG, FapyA, Tg (thymine glycol), hoU (hydroxyuracil), hmU (hydroxymethyluracil), fU (formyluracil), hoC (hydroxycytosine), fC (formylcytosine), 5-meC (5-methylcytosine), 6-meG (O6-methylguanine), 7-meG (N7-methylguanine), εC (ethenocytosine), 5-caC (5-carboxylcytosine), 2-hA, εA (ethenoadenine), 5-fU (5-fluorouracil), 3-meG (3-methylguanine), and isodialuric acid.

Provided herein are devices, compositions, systems, and methods for bioencryption comprising use of nucleic acid probe sequences. In some instances, nucleic acid probe sequences that are complementary to a portion of the nucleic acid sequences are then removed by a nuclease. For example, the nuclease is a duplex specific nuclease that recognizes a double stranded nucleic acid molecule formed between the nucleic acid probes and the nucleic acid sequences. In some instances, the nucleic acid probe allows for capturing and isolating nucleic acid sequences. In some instances, the nucleic acid probes comprise at least about 5, 10, 15, 20, 25, 30, 35, 40, 45, 50, 55, 60, 65, 70, 75, 80, 85, 90, 95, 100, or more than 100 nucleotides in length.

In some instances, nucleic acid sequences are identified using nucleic acid probes comprising a label such as, but not limited to, an affinity tag such as biotin, digoxigenin, Ni-Nitrilotriacetic acid, desthiobiotin, histidine, polyhistidine, myc, hemagglutinin (HA), FLAG, a fluorescence tag, a tandem affinity purification (TAP) tag, glutathione S transferase (GST), a polynucleotide, an aptamer, a polypeptide (e.g., an antigen or antibody), or derivatives thereof. In some instances, the label is detected by light absorption, fluorescence, chemiluminescence, electrochemiluminescence, mass, or charge. Non-limiting examples of fluorophores are Alexa-Fluor dyes (e.g., Alexa Fluor^{®} 350, Alexa Fluor^{®} 405, Alexa Fluor^{®} 430, Alexa Fluor^{®} 488, Alexa Fluor^{®} 500, Alexa Fluor^{®} 514, Alexa Fluor^{®} 532, Alexa Fluor^{®} 546, Alexa Fluor^{®} 555, Alexa Fluor^{®} 568, Alexa Fluor^{®} 594, Alexa Fluor^{®} 610, Alexa Fluor^{®} 633, Alexa Fluor^{®} 647, Alexa Fluor^{®} 660, Alexa Fluor^{®} 680, Alexa Fluor^{®} 700, and Alexa Fluor^{®} 750), APC, Cascade Blue, Cascade Yellow and R-phycoerythrin (PE), DyLight 405, DyLight 488, DyLight 550, DyLight 650, DyLight 680, DyLight 755, DyLight 800, FITC, Pacific Blue, PerCP, Rhodamine, Texas Red, Cy5, Cy5.5, and Cy7.

Provided herein are devices, compositions, systems, and methods for bioencryption and/or biodecryption comprising nucleic acid hybridization based binding. Nucleic acid probes comprising an affinity tag may be used. In some instances, the affinity tag allows for the nucleic acid sequences to be pulled down. For example, the affinity tag biotin is conjugated to nucleic acid probes that are complementary to the nucleic acid sequences and is pulled down using streptavidin. In some instances, the affinity tag comprises magnetically susceptible material, e.g., a magnet or magnetically susceptible metal. In some instances, the nucleic acid sequences are pulled down using a solid support such as streptavidin and immobilized on the solid support. In some instances, the nucleic acid sequences are pulled down in solution such as through beads. In some instances, the nucleic acid probes allow for exclusion based on size. For example, the nucleic acid probes result in the nucleic acid sequences having a size different from other nucleic acid sequences so that the nucleic acid sequences are removed by size-based depletion.

Devices, compositions, systems, and methods for bioencryption and/or biodecryption comprising nucleic acid hybridization based binding may comprise controlled amplification. In some instances, the nucleic acid hybridization based binding strategy is directed to controlled amplification, where a plurality of oligonucleotides synthesized have a similar region for a forward primer to bind, but the reverse primer region is not readily identifiable. In such an instance, a predetermined reverse primer would be required. In a first exemplary workflow, a pool of reverse primers with preselected regions to bind to each of the different synthesized oligonucleotides is generated and used in an extension amplification reaction (e.g., with a DNA polymerase) to amplify the oligonucleotides for downstream processing, e.g., further amplification or a DNA sequencing reaction. Optionally, each of the reverse primers comprises an adapter region comprising a common sequence to incorporate a universal reverse primer binding site by an extension amplification reaction (e.g., with a DNA polymerase). In such an arrangement, the downstream processing is simplified as only a single forward or reverse primer is required to amplify or sequence the plurality of oligonucleotides. In a second exemplary workflow, a plurality of oligonucleotides are synthesized, each having one or two regions comprising a hybridization motif that, while varied, has sufficient hybridization ability to a common primer to allow downstream processing of the plurality of oligonucleotides (e.g., amplification or sequencing reactions) utilizing a common primer for one of or both of 5' and 3' regions of each of the synthesized oligonucleotides. In some instances, the oligonucleotide population is designed to be hybridized to about 1, 2, 3, 4, 5, 6, 7, 8, 9, 10, 11, 12, 13, 14, 15, 16, 17, 18, 19, 20 or more nucleobases of the common primer.

Provided herein are devices, compositions, systems, and methods for bioencryption and/or biodecryption comprising use of electromagnetic radiation (EMR). In some instances, the electromagnetic radiation provides for cleavage or image capture-based detection of a nucleic acid sequence. In some instances, EMR is applied towards a surface at a wavelength from about 100 nm to about 400 nm, from about 100 nm to about 300 nm, or from about 100 nm to about 200 nm. In some instances, EMR is applied towards a surface at a wavelength from less than 0.01 nm. In some instances, EMR is applied towards a surface at a wavelength from about 10 nm to about 400 nm, about 400 nm to about 700 nm, or about 700 nm to about 100,000 nm. For example, EMR is applied at an ultraviolet (UV) wavelength, or a deep UV wavelength. In some instances, deep UV light is applied to a surface at a wavelength of about 172 nm to cleave a bound agent from the surface. In some instances, EMR is applied with a xenon lamp. Exposure distance is a measurement between the lamp and the surface. In some instances, the exposure distance is about 0.1 to 5 cm. In some instances, the exposure distance is about 0.5 to 2 cm. In some instances, the exposure distance is about 0.5, 1, 2, 3, 4, or 5 cm. In some instances, EMR is applied with a laser. Exemplary lasers and their wavelengths include but are not limited to Ar₂ (126 nm), Kr₂ (146 nm), F₂ (157 nm), Xe₂ (172 and 175 nm), ArF (193 nm). In some instances, the nucleic acid sequences comprise nucleobases that are photocleavable at a specific site. In some instances, the nucleic acid sequences comprise a modified nucleobase that is photocleavable. In some instances, the nucleic acid sequences are photocleavable by application of a specific wavelength of light. In some instances, the nucleic acid sequences are photocleavable by application of multiple wavelengths of light.

Provided herein are devices, compositions, systems, and methods for bioencryption and/or biodecryption comprising use of chemical lysis. In some instances, the nucleic acid sequences comprise nucleobases that are chemically cleavable at a specific site. In some instances, the nucleic acid sequences comprise a modified nucleobase that is chemically cleavable. In some instances, the modified nucleobase comprises a modification that is chemically cleavable. In some instances, chemical lysis is performed using an amine reagent. In some instances, the amine reagent is a liquid, a gas, an aqueous reagent, or an anhydrous reagent. Non-limiting examples of amine reagents are ammonium hydroxide, ammonia gas, a C₁-C₆ alkylamine, or methylamine.

Devices, compositions, systems, and methods for bioencryption as described herein may comprise conversion of the digital sequence to a nucleic acid sequence. In some instances, the nucleic acid sequence is a DNA sequence. In some instances, the DNA sequence is single stranded or double stranded. In some instances, the nucleic acid sequence is a RNA sequence. In some instances, the RNA sequence is single stranded or double stranded. In some instances, the nucleic acid sequence is encrypted in a larger population of nucleic acid sequences. In some instances, the larger population of nucleic acid sequences is a homogenous population or a heterogeneous population. In some instances, the population of nucleic acid sequences comprises DNA sequences. In some instances, the DNA sequences are single stranded or double stranded. In some instances, the population of nucleic acid sequences comprises RNA sequences. In some instances, the RNA sequences are single stranded or double stranded.

A number of nucleic acid sequences may be encrypted. In some instances, the number of nucleic acid sequences that are encrypted are about 10 sequences to about 1 million or more sequences. In some instances, a number of nucleic acid sequences that are encrypted are at least about 10, 50, 100, 200, 500, 1,000, 2,000, 4,000, 8,000, 10,000, 25,000, 30,000, 35,000, 40,000, 45,000, 50,000, 55,000, 60,000, 65,000, 70,000, 80,000, 90,000, 100,000, 200,000, 300,000, 400,000, 500,000, 600,000, 700,000, 800,000, 900,000, 1 million, or more than 1 million sequences. In some instances, a number of nucleic acid sequences that are encrypted is greater than 1 trillion.

In some instances, the nucleic acid sequences that are encrypted comprise at least 10, 25, 50, 75, 100, 125, 150, 175, 200, 225, 250, 300, or more than 300 bases in length. In some instances, the nucleic acid sequences that are encrypted comprises 10 bases to 25 bases, 10 bases to 50 bases, 10 bases to 75 bases, 10 bases to 100 bases, 10 bases to 125 bases, 10 bases to 150 bases, 10 bases to 175 bases, 10 bases to 200 bases, 10 bases to 225 bases, 10 bases to 250 bases, 10 bases to 300 bases, 25 bases to 50 bases, 25 bases to 75 bases, 25 bases to 100 bases, 25 bases to 125 bases, 25 bases to 150 bases, 25 bases to 175 bases, 25 bases to 200 bases, 25 bases to 225 bases, 25 bases to 250 bases, 25 bases to 300 bases, 50 bases to 75 bases, 50 bases to 100 bases, 50 bases to 125 bases, 50 bases to 150 bases, 50 bases to 175 bases, 50 bases to 200 bases, 50 bases to 225 bases, 50 bases to 250 bases, 50 bases to 300 bases, 75 bases to 100 bases, 75 bases to 125 bases, 75 bases to 150 bases, 75 bases to 175 bases, 75 bases to 200 bases, 75 bases to 225 bases, 75 bases to 250 bases, 75 bases to 300 bases, 100 bases to 125 bases, 100 bases to 150 bases, 100 bases to 175 bases, 100 bases to 200 bases, 100 bases to 225 bases, 100 bases to 250 bases, 100 bases to 300 bases, 125 bases to 150 bases, 125 bases to 175 bases, 125 bases to 200 bases, 125 bases to 225 bases, 125 bases to 250 bases, 125 bases to 300 bases, 150 bases to 175 bases, 150 bases to 200 bases, 150 bases to 225 bases, 150 bases to 250 bases, 150 bases to 300 bases, 175 bases to 200 bases, 175 bases to 225 bases, 175 bases to 250 bases, 175 bases to 300 bases, 200 bases to 225 bases, 200 bases to 250 bases, 200 bases to 300 bases, 225 bases to 250 bases, 225 bases to 300 bases, or 250 bases to 300 bases.

In some instances, nucleic acid sequences that are encrypted result in at least 10%, 20%, 30%, 40%, 50%, 60%, 70%, 80%, 90%, 95%, or more than 95% enrichment of a nucleic acid sequence of interest. In some instances, nucleic acid sequences that are encrypted result in about 10%, 20%, 30%, 40%, 50%, 60%, 70%, 80%, 90%, 95%, or more than 95% enrichment of a nucleic acid sequence of interest.

Devices, compositions, systems, and methods for bioencryption and/or biodecryption as described herein may comprise a DNA or RNA based system. Canonical DNA is a base 4 coding system, having four different nucleobases available: A, T, C or G (adenine, thymine, cytosine, and guanine). Thus, these 4 bases allow for a base 3 (using less than all), or a 4 base coding scheme. In addition, use of uracil (U), which is found in RNA, provides a fifth base and allows for a base 5 coding scheme. In addition, a modified nucleobase may be used for a nucleic acid base coding greater than 4. Nucleobases that are not canonical DNA nucleobases or modified nucleobases include, without limitation, uracil, 3-meA (3-methyladenine), hypoxanthine, 8-oxoG (7,8-dihydro-8-oxoguanine), FapyG, FapyA, Tg (thymine glycol), hoU (hydroxyuracil), hmU (hydroxymethyluracil), fU (formyluracil), hoC (hydroxycytosine), fC (formylcytosine), 5-meC (5-methylcytosine), 6-meG (O6-methylguanine), 7-meG (N7-methylguanine), εC (ethenocytosine), 5-caC (5-carboxylcytosine), 2-hA, εA (ethenoadenine), 5-fU (5-fluorouracil), 3-meG (3-methylguanine), hmC (hydroxymethylcytosine) and isodialuric acid. Further provided herein are coding schemes where machine instructions provide for conversion of digital information in the form of a binary sequence into an intermediate code prior to ultimately being converted to the final nucleic acid sequence.

In some instances, to store data in a sequence of DNA, the information is converted from the 1s and 0s of binary code into the code of A, T, G, and C bases of DNA. In some instances, items of information are first encoded in a digital information form. In some cases, the binary code of digital information is converted into a biomolecule-based (e.g., DNA-based) code while preserving the information that the code represents. This converted code (digital binary code to a biomolecule code) is referred to herein as resulting in a "predetermined" sequence with respect to the deposit of a biomolecule disclosed herein on a surface disclosed herein. The predetermined sequence may encode sequence for a plurality of oligonucleotides.

### Binary code conversion

Generally, the initial code is digital information, typically in the form of binary code employed by a computer. General purpose computers are electronic devices reading "on" or "off" states, represented by the numbers "0" and "1". This binary code is application for computers to read multiple types of items of information. In binary arithmetic, the number two is written as the number 10. For example, "10" indicates "one time the number, two and no more". The number "3," is written as "11" to mean "one times two and one more." The number "4" is written as "100," the number "5" as "101," "six" as "110," etc. An example of American Standard Code II (ASCII) for binary code is provided for the alphabet in lower and upper case in **Table 3.**

**Table 3. American Standard Code II (ASCII) for Binary Code**

| **Letter** | **ASCII Code** | **Binary** | | **Letter** | **ASCII Code** | **Binary** | | **No.** | **ASCII Code** | **Binary** |
|---|---|---|---|---|---|---|---|---|---|---|
| a | 97 | 1100001 | | A | 65 | 1000001 | | 0 | chr(0) | 00000000 |
| b | 98 | 1100010 | | B | 66 | 1000010 | | 1 | chr(1) | 00000001 |
| c | 99 | 1100011 | | C | 67 | 1000011 | | 2 | chr(2) | 00000010 |
| d | 100 | 1100100 | | D | 68 | 1000100 | | 3 | chr(3) | 00000011 |
| e | 101 | 1100101 | | E | 69 | 1000101 | | 4 | chr(4) | 00000100 |
| f | 102 | 1100110 | | F | 70 | 1000110 | | 5 | chr(5) | 00000101 |
| g | 103 | 1100111 | | G | 71 | 1000111 | | 6 | chr(6) | 00000110 |
| h | 104 | 1101000 | | H | 72 | 1001000 | | 7 | chr(7) | 00000111 |
| i | 105 | 1101001 | | I | 73 | 1001001 | | 8 | chr(8) | 00001000 |
| j | 106 | 1101010 | | J | 74 | 1001010 | | 9 | chr(9) | 00001001 |
| k | 107 | 1101011 | | K | 75 | 1001011 | | 10 | chr(10) | 00001010 |
| l | 108 | 1101100 | | L | 76 | 1001100 | | 11 | chr(11) | 00001011 |
| m | 109 | 1101101 | | M | 77 | 1001101 | | 12 | chr(12) | 00001100 |
| n | 110 | 1101110 | | N | 78 | 1001110 | | 13 | chr(13) | 00001101 |
| o | 111 | 1101111 | | O | 79 | 1001111 | | 14 | chr(14) | 00001110 |
| p | 112 | 1110000 | | P | 80 | 1010000 | | 15 | chr(15) | 00001111 |
| q | 113 | 1110001 | | Q | 81 | 1010001 | | 16 | chr(16) | 00010000 |
| r | 114 | 1110010 | | R | 82 | 1010010 | | 17 | chr(17) | 00010001 |
| s | 115 | 1110011 | | S | 83 | 1010011 | | 18 | chr(18) | 00010010 |
| t | 116 | 1110100 | | T | 84 | 1010100 | | 19 | chr(19) | 00010011 |
| u | 117 | 1110101 | | U | 85 | 1010101 | | 20 | chr(20) | 00010100 |
| v | 118 | 1110110 | | V | 86 | 1010110 | | 21 | chr(21) | 00010101 |
| w | 119 | 1110111 | | W | 87 | 1010111 | | 22 | chr(22) | 00010110 |
| x | 120 | 1111000 | | X | 88 | 1011000 | | 23 | chr(23) | 00010111 |
| y | 121 | 1111001 | | Y | 89 | 1011001 | | 24 | chr(24) | 00011000 |
| z | 122 | 1111010 | | Z | 90 | 1011010 | | 25 | chr(25) | 00011001 |
| | | | | | | | | 26 | chr(26) | 00011010 |
| | | | | | | | | 27 | chr(27) | 00011011 |
| | | | | | | | | 28 | chr(28) | 00011100 |
| | | | | | | | | 29 | chr(29) | 00011101 |
| | | | | | | | | 30 | chr(30) | 00011110 |

Provided herein are methods for converting information in the form of a first code, e.g., a binary sequence to a nucleic acid sequence. The process may involve direct conversion from a base 2 code (i.e., binary) to a base code that is higher. Exemplary base codes include 2, 3, 4, 5, 6, 7, 8, 9, 10 or more. **Table 4** illustrates an exemplary alignment between various base numbering schemes. A computer receiving machine instructions for conversion, can automatically convert sequence information from one code to another.

**Table 4. Alignment of Base Numbering Schemes**

| | | | | | | | | | | |
|---|---|---|---|---|---|---|---|---|---|---|
| Decimal | 0 | 1 | 2 | 3 | 4 | 5 | 6 | 7 | 8 | 9 |
| Quaternary | 0 | 1 | 2 | 3 | 10 | 11 | 12 | 13 | 20 | 21 |
| Octal | 0 | 1 | 2 | 3 | 4 | 5 | 6 | 7 | 10 | 11 |
| Ternary | 0 | 1 | 2 | 10 | 11 | 12 | 20 | 21 | 22 | 100 |
| Binary | 0 | 1 | 10 | 11 | 100 | 101 | 110 | 111 | 1000 | 1001 |

### Nucleic Acid Sequence

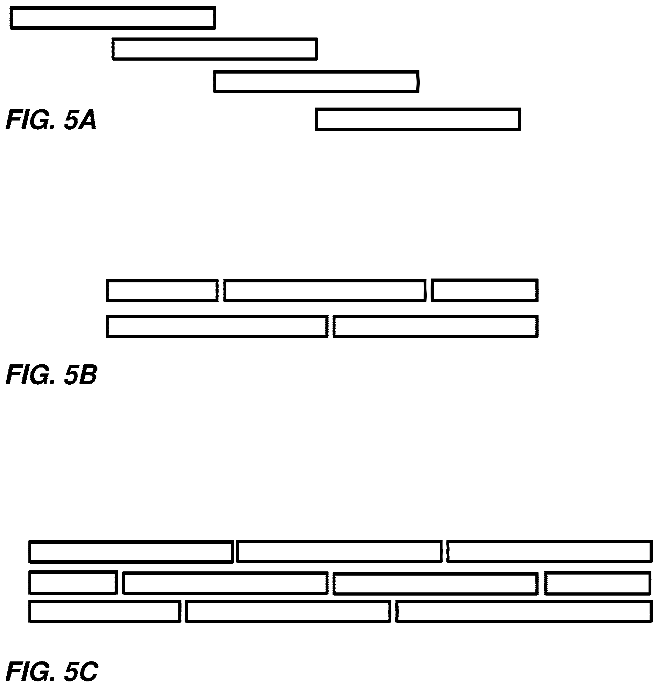

Provided herein are methods for designing a sequence for an oligonucleotide described herein such that the nucleic acid sequence encodes for at least part of an item of information. In some instances, each oligonucleotide sequence has design features to facilitate with sequence alignment during subsequent assembly steps and also to provide a means for error correction. In some arrangements, oligonucleotide sequences are designed such that overlap exits between each oligonucleotide sequence with another in the population. In some instances, each oligonucleotide sequence overlaps with a portion of just one other oligonucleotide sequence, **FIG. 5A****.** In an alternative arrangement, each oligonucleotide sequence region overlaps with two sequences such that 2 copies are generated for each sequence within a single oligonucleotide, **FIG. 5B****.** In yet another arrangement, each oligonucleotide sequence region overlaps with more than two sequences such that 3 copies are generated for each sequence within a single oligonucleotide, **FIG. 5C****.** Sequences for oligonucleotides described herein may encode for 10-2000, 10-500, 30-300, 50-250, or 75-200 bases in length. In some instances, each of the oligonucleotide sequence is at least 10, 15, 20, 25, 30, 50, 100, 150, 200, 500 or more bases in length.

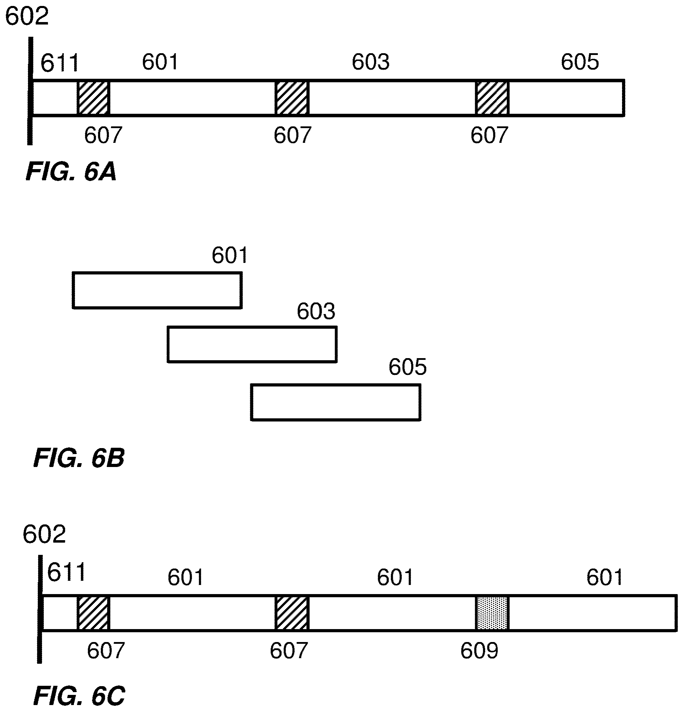

Provided herein are methods, systems and compositions wherein each oligonucleotide sequence described herein is designed to comprise a plurality of coding regions and a plurality of non-coding regions, **FIG. 6A****.** In such an arrangement, each coding region (e.g., **601, 603, 605)** encodes for at least a portion of an item of information. Optionally, each coding region in the same oligonucleotide encodes for a sequence from the same item of information, and an overlapping scheme is optionally employed as described herein, **FIG. 6B****.** In further instances, each coding region in the same oligonucleotide encodes for the same sequence, **FIG. 6C****.** Sequences for oligonucleotides described herein may encode for at least 1, 2, 3, 4, 5, 6, 7, 8, 9, 10, 11, 12, 13, 14, 15, 16, 17, 18, 19, 20 or more coding regions. Sequences for oligonucleotides described herein may encode for at least 1, 2, 3, 4, 5, 6, 7, 8, 9, 10, 11, 12, 13, 14, 15, 16, 17, 18, 19, 20 or more of the same coding region. In some instances, each of the multiple coding regions is 10-1000, 20-500, 30-300, 50-250, or 75-200 bases in length. In some instances, each of the multiple coding regions is at least 10, 15, 20, 25, 30, 50, 100, 150, 200 or more bases in length. In some instances, each oligonucleotide comprises a tether region **611** linking the molecule to the surface **602** of a structure.

In arrangements where multiple coding sequences are present in the same oligonucleotide, a cleavage region **607** is optionally present in between each coding region. The cleavage region **607** may be present at the junction between each coding region, or may be present within an adaptor region having a string of sequence between each coding region. A cleavage region **607** may encode for a sequence feature, once synthesized, which will break from the strand subsequent to application of a cleavage signal. The cleavage region **607** may encode for a restriction enzyme recognition site, a modified nucleic acid that is light sensitive and will break under application of electromagnetic radiation (e.g., oligodeoxynucleotide heteropolymers carrying base-sensitive S-pivaloylthioethyl (t-Bu-SATE) phosphotriester linkages sensitive to light wavelengths of >300 nm), or a modified nucleic acid that is sensitive to application of a certain chemical, e.g., Thymidine-succinyl hexamide CED phosphoramidite (CLP-2244 from ChemGenes) which breaks subsequent to application of ammonia gas. Because the design of a sequence to have a particular cleavage scheme may not be readily apparent from sequencing synthesized oligonucleotides, the cleavage scheme provides a means for adding a level of security to sequences encoded by the synthesized nucleic acid library. Sequences for oligonucleotides described herein may encode for at least 1, 2, 3, 4, 5, 6, 7, 8, 9, 10, 11, 12, 13, 14, 15, 16, 17, 18, 19, 20 or more cleavage regions. In some instances, each of the cleavage region encodes for 1-100, 1-50, 1-20, 1-10, 5-25, or 5-30 bases in length. In some instances, each of the cleavage region encodes for at least 1, 2, 3, 4, 5, 6, 7, 8, 9, 10, 11, 12, 13, 14, 15, 16, 17, 18, 19, 20, 25, 30, 40, 50 100 or more bases. In some arrangements, for each oligonucleotide, each coding region is identical and each cleavage region between each coding region is different. For example, a first cleavage region **607** is different from a second cleavage region **609.** In some arrangements, the cleavage region **607** closest to the surface **602** is identical to the next distal cleavage region **607.**

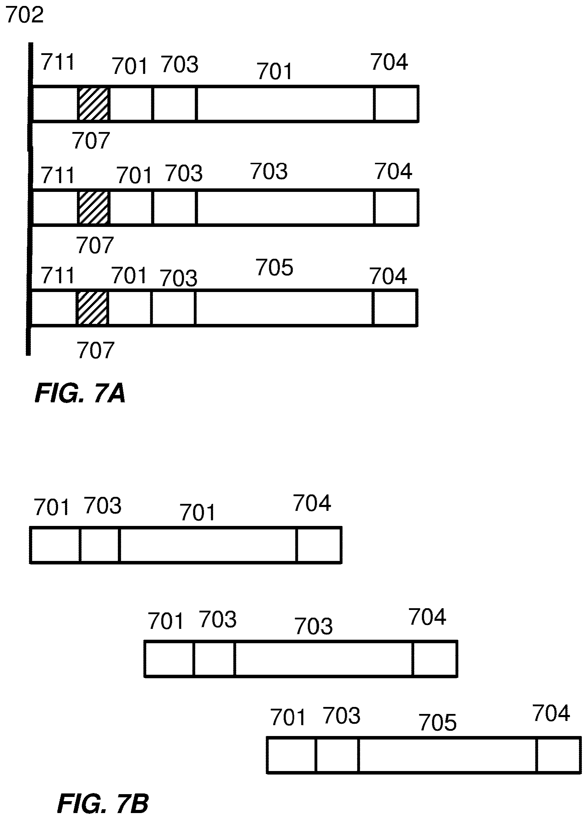

Barcodes are typically known nucleic acid sequences that allow some feature of a polynucleotide with which the barcode is associated to be identified. **FIGS. 7A-7B** provide an illustrative barcode arrangement. In **FIG. 7A****,** each coding region for a first oligonucleotide **701,** a second oligonucleotide **703,** and a third oligonucleotide **705,** has the following features (from surface **702** outward): a tether region **702,** a cleavage region **707,** a first primer binding region **701,** a barcode region **703,** a coding region **701, 703, 705,** and a second primer binding region **704.** The oligonucleotides may be amplified with the use of primers that recognize the first and/or second primer binding regions. Amplification may occur to oligonucleotides attached to the surface or released from the surface (i.e., via cleavage at the cleavage region **707).** After sequencing, the barcode region **703,** provides an indicator for identifying a characteristic associated with the coding region. In some instances, a barcode comprises a nucleic acid sequence that when joined to a target polynucleotide serves as an identifier of the sample from which the target polynucleotide was derived. Barcodes can be designed at suitable lengths to allow sufficient degree of identification, e.g., at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 ,36 ,37, 38, 39, 40, 41, 42, 43, 44, 45, 46, 47, 48, 49, 50, 51, 52, 53, 54, 55, or more bases in length. Multiple barcodes, such as 2, 3, 4, 5, 6, 7, 8, 9, 10, or more barcodes, may be used on the same molecule, optionally separated by non-barcode sequences. In some instances, barcodes are shorter than 10, 9, 8, 7, 6, 5, or 4 bases in length. In some instances, barcodes associated with some polynucleotides are of different lengths than barcodes associated with other polynucleotides. In general, barcodes are of sufficient length and comprise sequences that are sufficiently different to allow the identification of samples based on barcodes with which they are associated. In some arrangements, a barcode, and the sample source with which it is associated, can be identified accurately after the mutation, insertion, or deletion of one or more bases in the barcode sequence, such as the mutation, insertion, or deletion of 1, 2, 3, 4, 5, 6, 7, 8, 9, 10, or more bases. In some instances, each barcode in a plurality of barcodes differ from every other barcode in the plurality by at least three base positions, such as at least 3, 4, 5, 6, 7, 8, 9, 10, or more positions. Arrangements provided herein may include a barcode sequence that indicates the nucleic acid sequence encoding for a sequence in a particular region of a digital sequence. For example, a barcode sequence may indicate where in a large file a particular oligonucleotide sequence encodes. In some instances, a barcode sequence may indicate which file a particular oligonucleotide sequence is associated with. In some instances, a barcode sequence includes information associated with the conversion scheme for a particular sequence, providing an added layer of security.

Provided herein are oligonucleotide sequence design schemes where each oligonucleotide sequence in a population of oligonucleotide sequences is designed to have at least one region in common amongst oligonucleotide sequences in that population. For example, all oligonucleotides in the same population may comprise one or more primer regions. The design of sequence-specific primer regions allows for the selection of oligonucleotides to be amplified in selected batches from a large library of multiple oligonucleotides. Each oligonucleotide sequence may comprise at least 1, 2, 3, 4, 5, 6, 7, 8, 9, 10 or more primer binding sequences. A population of oligonucleotide sequences may comprise at least 1, 2, 3, 4, 5, 6, 7, 8, 9, 10, 25, 50, 100, 200, 500, 1000, 5000, 10000, 50000, 100000 or more non-identical binding sequences. Primer binding sequences may comprise 5-100, 10-75, 7-60, 8-60, 10-50, or 10-40 bases in length.

### Structures for oligonucleotide synthesis

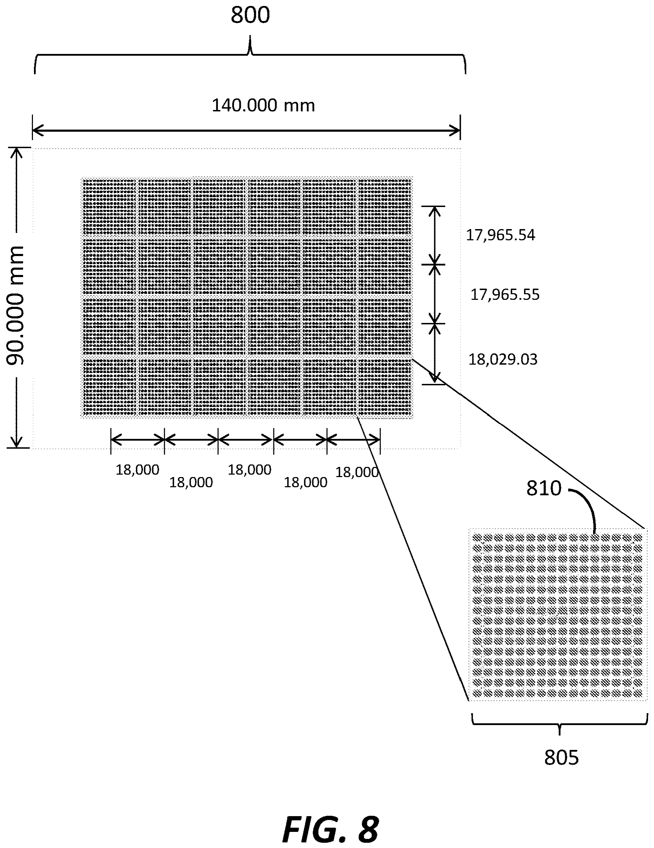

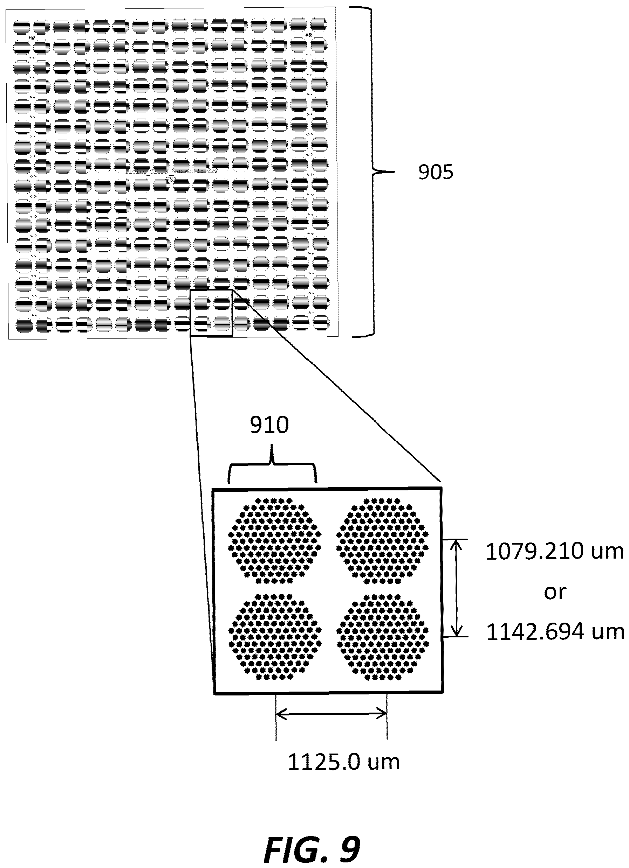

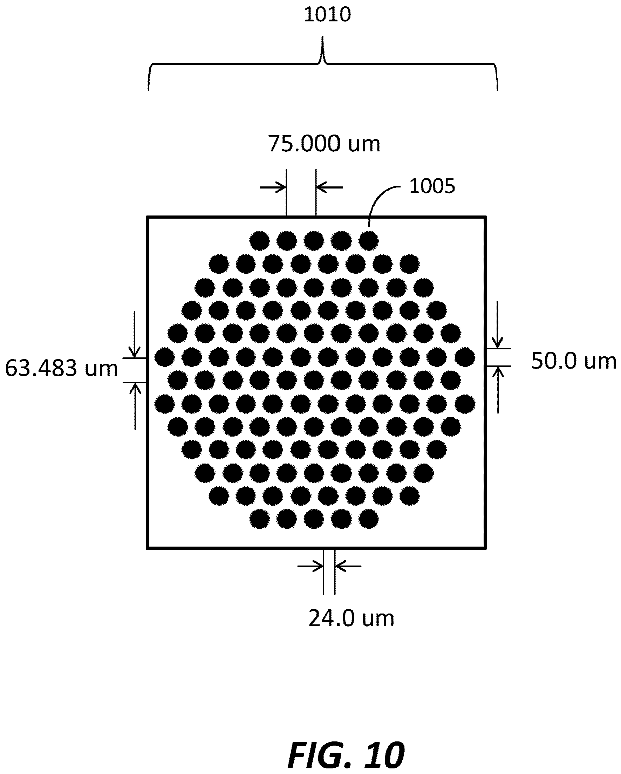

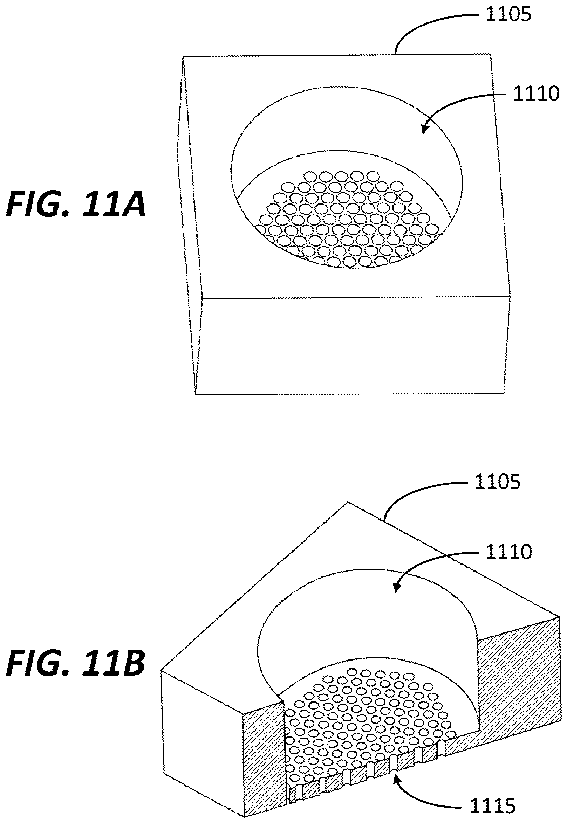

Provided herein are rigid or flexibles structures for oligonucleotide synthesis for use with devices, compositions, systems, and methods for bioencryption and/or biodecryption as described herein. In the case of rigid structures, provided herein are devices having structures (*e*.*g*., a plate) for the generation of a library of oligonucleotides. An exemplary structure **800** is illustrated in **FIG. 8****,** wherein the structure **800** has about the same size dimensions as a standard 96 well plate: 140 mm by 90 mm. The structure **800** comprises clusters grouped in 24 regions or sub-fields **805,** each sub-field **805** comprising an array of 256 clusters **810.** An expanded view of an exemplary sub-field **805** is shown in **FIG. 9****.** The structure as seen in **FIG. 8** and **FIG. 9** may be substantially planar. In the expanded view of four clusters **(****FIG. 9**), a single cluster **910,** has a Y axis cluster pitch (distance from center to center of adjacent clusters) of 1079.210 um or 1142.694 um, and an X axis cluster pitch of 1125 um. An illustrative cluster **1010** is depicted in **FIG. 10****,** where the Y axis loci pitch (distance from center to center of adjacent loci) is 63.483 um, and an X axis loci pitch is 75 um. The locus width at the longest part, *e*.*g*., diameter for a circular locus, is 50 um and the distance between loci is 24 um. The number of loci **1005** in the exemplary cluster in **FIG. 10** is 121. The loci (also referred to as "features"), may be flat, wells, or channels. An exemplary channel arrangement is illustrated in **FIGS. 11A-11B** where a plate **1105** is illustrated comprising a main channel **1110** and a plurality of channels **1115** connected to the main channel **1110.** The connection between the main channel **1110** and the plurality of channels **1115** provides for a fluid communication for flow paths from the main channel **1110** to the each of the plurality of channels **1115.** A plate **1105** described herein can comprise multiple main channels **1110.** The plurality of channels **1115** collectively forms a cluster within the main channel **1110.**

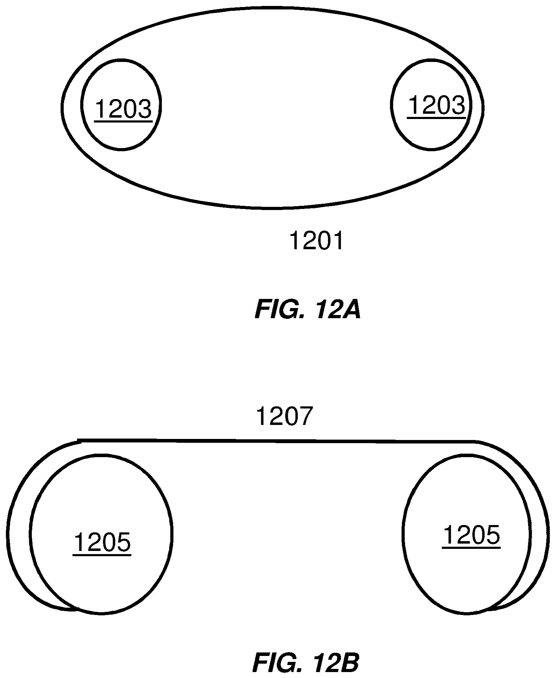

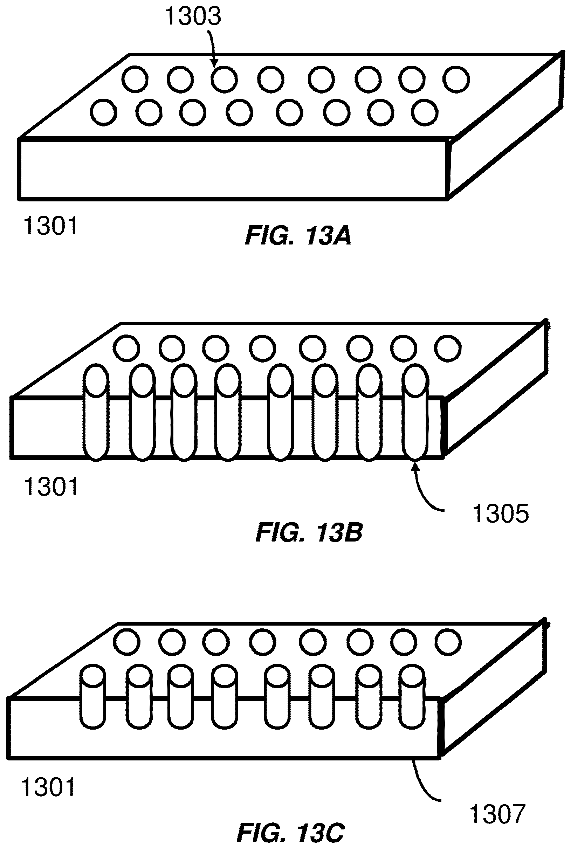

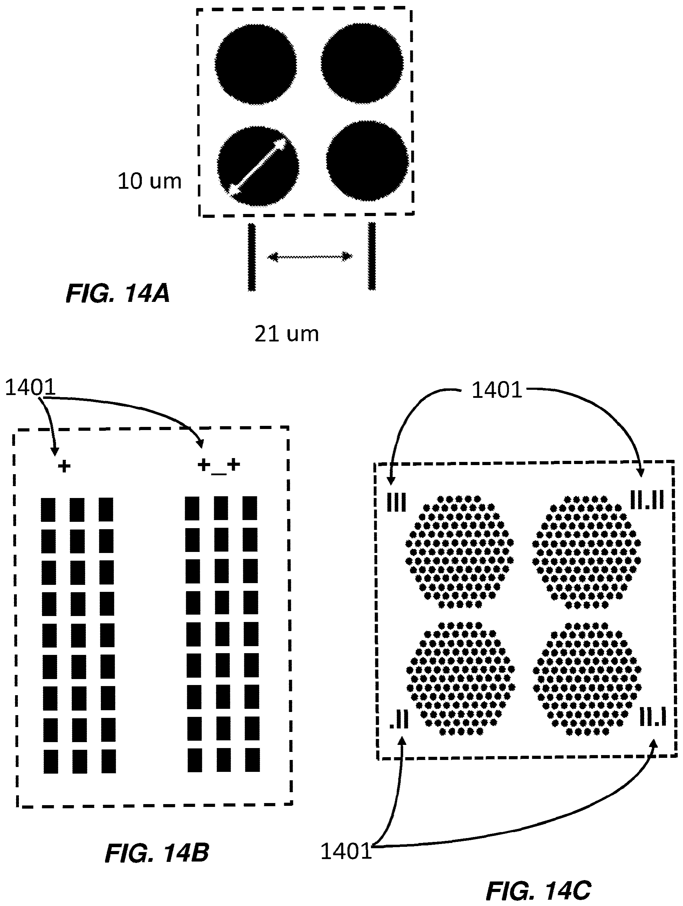

In the case of flexible structures, provided herein are devices wherein the flexible structure comprises a continuous loop **1201** wrapped around one or more fixed structures, e.g., a pair of rollers **1203** or a non-continuous flexible structure **1207** wrapped around separate fixed structures, e.g., a pair reels **1205**. *See* **FIGS. 12A-12B****.** Provided herein are flexible structures having a surface with a plurality of features (loci) for oligonucleotide extension. Each feature in a portion of the flexible structure **1301** may be a substantially planar feature **1303** (e.g., flat), a channel **1305,** or a well **1307.** *See* **FIGS. 13A-13C****.** In one exemplary arrangement, each feature of the structure has a width of about 10 um and a distance between the center of each structure of about 21 um. *See* **FIG. 14A****.** Features may comprise, without limitation, circular, rectangular, tapered, or rounded shapes.

Structures for oligonucleotide synthesis for use with devices, compositions, systems, and methods for bioencryption and/or biodecryption as described herein may comprise a channel. In some instances, a channel described herein has a width to depth (or height) ratio of 1 to 0.01, wherein the width is a measurement of the width at the narrowest segment of the microchannel. In some instances, a channel described herein has a width to depth (or height) ratio of 0.5 to 0.01, wherein the width is a measurement of the width at the narrowest segment of the microchannel. In some instances, a channel described herein has a width to depth (or height) ratio of about 0.01, 0.05, 0.1, 0.15, 0.16, 0.2, 0.5, or 1.

Provided herein are structures for polynucleotide synthesis comprising a plurality of discrete loci, channels, wells or protrusions for polynucleotide synthesis. Structures described herein may comprise a plurality of clusters, each cluster comprising a plurality of wells, loci or channels. Alternatively, described herein are structures that may comprise a homogenous arrangement of wells, loci or channels. In some instances, a structure described herein is provided comprising a plurality of channels corresponding to a plurality of features (loci) within a cluster, wherein the height or depth of the channel is from about 5 um to about 500 um, from about 5 um to about 400 um, from about 5 um to about 300 um, from about 5 um to about 200 um, from about 5 um to about 100 um, from about 5 um to about 50 um, or from about 10 um to about 50 um. In some cases, the height or depth of a channel is less than 100 um, less than 80 um, less than 60 um, less than 40 um or less than 20 um. In some cases, channel height or depth is about 10, 20, 30, 40, 50, 60, 70, 80, 90, 100, 200, 300, 400, 500 um or more. In some instances, the height or depth is at least 10, 25, 50, 75, 100, 200, 300, 400, 500, 600, 700, 800, 900, 1000, or more than 1000 nm. In some instances, the height or depth is in a range of about 10 nm to about 1000 nm, about 25 nm to about 900 nm, about 50 nm to about 800 nm, about 75 nm to about 700 nm, about 100 nm to about 600 nm, or about 200 nm to about 500. In some instances, the height or depth is in a range of about 50 nm to about 1 um.

Structures for oligonucleotide synthesis for use with devices, compositions, systems, and methods for bioencryption and/or biodecryption as described herein may comprise a feature. In some instances, the width of a feature (e.g., substantially planar feature, well, channel, locus, or protrusion) is from about 0.1 um to about 500 um, from about 0.5 um to about 500 um, from about 1 um to about 200 um, from about 1 um to about 100 um, from about 5 um to about 100 um, or from about 0.1 um to about 100 um, for example, about 90 um, 80 um, 70 um, 60 um, 50 um, 40 um, 30 um, 20 um, 10 um, 5 um, 1 um or 0.5 um. In some instances, the width of a feature (e.g., microchannel) is less than about 100 um, 90 um, 80 um, 70 um, 60 um, 50 um, 40 um, 30 um, 20 um or 10 um. In some instances, the width of a feature is at least 10, 25, 50, 75, 100, 200, 300, 400, 500, 600, 700, 800, 900, 1000, or more than 1000 nm. In some instances, the width of a feature is in a range of about 10 nm to about 1000 nm, about 25 nm to about 900 nm, about 50 nm to about 800 nm, about 75 nm to about 700 nm, about 100 nm to about 600 nm, or about 200 nm to about 500. In some instances, the width of a feature is in a range of about 50 nm to about 1000 nm. In some instances, the distance between the center of two adjacent features is from about 0.1 um to about 500 um, 0.5 um to about 500 um, from about 1 um to about 200 um, from about 1 um to about 100 um, from about 5 um to about 200 um, from about 5 um to about 100 um, from about 5 um to about 50 um, or from about 5 um to about 30 um, for example, about 20 um. In some instances, the total width of a feature is about 5um, 10 um, 20 um, 30 um, 40 um, 50 um, 60 um, 70 um, 80 um, 90 um, or 100 um. In some instances, the total width of a feature is about 1 um to 100 um, 30 um to 100 um, or 50 um to 70 um. In some instances, the distance between the center of two adjacent features is from about 0.5 um to about 2 um, from about 0.5 um to about 2 um, from about 0.75 um to about 2 um, from about 1 um to about 2 um, from about 0.2 um to about 1 um, from about 0.5 um to about 1.5 um, from about 0.5 um to about 0.8 um, or from about 0.5 um to about 1 um, for example, about 1 um. In some instances, the total width of a features is about 50 nm, 0.1 um, 0.2 um, 0.3 um, 0.4 um, 0.5 um, 0.6 um, 0.7 um, 0.8 um, 0.9 um, 1 um, 1.1 um, 1.2 um, 1.3 um, 1.4 um, or 1.5 um. In some instances, the total width of a feature is about 0.5 um to 2 um, 0.75 um to 1 um, or 0.9 um to 2 um.

In some instances, each feature supports the synthesis of a population of oligonucleotides having a different sequence than a population of oligonucleotides grown on another feature. Provided herein are surfaces which comprise at least 10, 100, 256, 500, 1000, 2000, 3000, 4000, 5000, 6000, 7000, 8000, 9000, 10000, 11000, 12000, 13000, 14000, 15000, 20000, 30000, 40000, 50000 or more clusters. Provided herein are surfaces which comprise more than 2,000; 5,000; 10,000; 20,000; 30,000; 50,000; 100,000; 200,000; 300,000; 400,000; 500,000; 600,000; 700,000; 800,000; 900,000; 1,000,000; 5,000,000; or 10,000,000 or more distinct features. In some cases, each cluster includes 1, 2, 3, 4, 5, 6, 7, 8, 9, 10, 20, 30, 40, 50, 60, 70, 80, 90, 100, 120, 130, 150, 200, 500 or more features. In some cases, each cluster includes 50 to 500, 50 to 200, 50 to 150, or 100 to 150 features. In some cases, each cluster includes 100 to 150 features. In exemplary arrangements, each cluster includes 109, 121, 130 or 137 features.

Provided herein are features having a width at the longest segment of 5 to 100 um. In some cases, the features have a width at the longest segment of about 30, 35, 40, 45, 50, 55 or 60 um. In some cases, the features are channels having multiple segments, wherein each segment has a center to center distance apart of 5 to 50 um. In some cases, the center to center distance apart for each segment is about 5, 10, 15, 20 or 25 um.

In some instances, the number of distinct oligonucleotides synthesized on the surface of a structure described herein is dependent on the number of distinct features available in the substrate. In some instances, the density of features within a cluster of a substrate is at least or about 1 feature per mm², 10 features per mm², 25 features per mm², 50 features per mm², 65 features per mm², 75 features per mm², 100 features per mm², 130 features per mm², 150 features per mm², 175 features per mm², 200 features per mm², 300 features per mm², 400 features per mm², 500 features per mm², 1,000 features per mm² or more. In some cases, a substrate comprises from about 10 features per mm² to about 500 mm², from about 25 features per mm² to about 400 mm², from about 50 features per mm² to about 500 mm², from about 100 features per mm² to about 500 mm², from about 150 features per mm² to about 500 mm², from about 10 features per mm² to about 250 mm², from about 50 features per mm² to about 250 mm², from about 10 features per mm² to about 200 mm², or from about 50 features per mm² to about 200 mm². In some instances, the distance between the centers of two adjacent features within a cluster is from about 10 um to about 500 um, from about 10 um to about 200 um, or from about 10 um to about 100 um. In some cases, the distance between two centers of adjacent features is greater than about 10 um, 20 um, 30 um, 40 um, 50 um, 60 um, 70 um, 80 um, 90 um or 100 um. In some cases, the distance between the centers of two adjacent features is less than about 200 um, 150 um, 100 um, 80 um, 70 um, 60 um, 50 um, 40 um, 30 um, 20 um or 10 um. In some cases, the distance between the centers of two adjacent features is less than about 10000 nm, 8000 nm, 6000 nm, 4000 nm, 2000 nm 1000 nm, 800 nm, 600 nm, 400 nm, 200 nm, 150 nm, 100 nm, 80 um, 70 nm, 60 nm, 50 nm, 40 nm, 30 nm, 20 nm or 10 nm. In some instances, each square meter of a structure described herein allows for at least about 10⁷, 10⁸, 10⁹, 10¹⁰, 10¹¹ features, where each feature supports one oligonucleotide. In some instances, 10⁹ oligonucleotides are supported on less than about 6, 5, 4, 3, 2 or 1 m² of a structure described herein.

Structures for oligonucleotide synthesis for use with devices, compositions, systems, and methods for bioencryption and/or biodecryption as described herein supports synthesis of a number of oligonucleotides. In some instances, a structure described herein provides support for the synthesis of more than 2,000; 5,000; 10,000; 20,000; 30,000; 50,000; 100,000; 200,000; 300,000; 400,000; 500,000; 600,000; 700,000; 800,000; 900,000; 1,000,000; 1,200,000; 1,400,000; 1,600,000; 1,800,000; 2,000,000; 2,500,000; 3,000,000; 3,500,000; 4,000,000; 4,500,000; 5,000,000; 10,000,000 or more non-identical oligonucleotides. In some cases, the structure provides support for the synthesis of more than 2,000; 5,000; 10,000; 20,000; 50,000; 100,000; 200,000; 300,000; 400,000; 500,000; 600,000; 700,000; 800,000; 900,000; 1,000,000; 1,200,000; 1,400,000; 1,600,000; 1,800,000; 2,000,000; 2,500,000; 3,000,000; 3,500,000; 4,000,000; 4,500,000; 5,000,000; 10,000,000 or more oligonucleotides encoding for distinct sequences. In some instances, at least a portion of the oligonucleotides have an identical sequence or are configured to be synthesized with an identical sequence. In some instances, the structure provides a surface environment for the growth of oligonucleotides having at least about 50, 60, 70, 75, 80, 85, 90, 95, 100, 110, 120, 130, 140, 150, 160, 175, 200, 225, 250, 275, 300, 325, 350, 375, 400, 425, 450, 475, 500 bases or more.

In some instances, oligonucleotides are synthesized on distinct features of a structure, wherein each feature supports the synthesis of a population of oligonucleotides. In some cases, each feature supports the synthesis of a population of oligonucleotides having a different sequence than a population of oligonucleotides grown on another locus. In some instances, the features of a structure are located within a plurality of clusters. In some instances, a structure comprises at least 10, 500, 1000, 2000, 3000, 4000, 5000, 6000, 7000, 8000, 9000, 10000, 11000, 12000, 13000, 14000, 15000, 20000, 30000, 40000, 50000 or more clusters. In some instances, a structure comprises more than 2,000; 5,000; 10,000; 100,000; 200,000; 300,000; 400,000; 500,000; 600,000; 700,000; 800,000; 900,000; 1,000,000; 1,100,000; 1,200,000; 1,300,000; 1,400,000; 1,500,000; 1,600,000; 1,700,000; 1,800,000; 1,900,000; 2,000,000; 300,000; 400,000; 500,000; 600,000; 700,000; 800,000; 900,000; 1,000,000; 1,200,000; 1,400,000; 1,600,000; 1,800,000; 2,000,000; 2,500,000; 3,000,000; 3,500,000; 4,000,000; 4,500,000; 5,000,000; or 10,000,000 or more distinct features. In some cases, each cluster includes 1, 2, 3, 4, 5, 6, 7, 8, 9, 10, 20, 30, 40, 50, 60, 70, 80, 90, 100, 120, 130, 150 or more features (loci). In some instances, each cluster includes 50 to 500, 100 to 150, or 100 to 200 features. In some instances, each cluster includes 109, 121, 130 or 137 features. In some instances, each cluster includes 5, 6, 7, 8, 9, 10, 11 or 12 features. In some instances, oligonucleotides from distinct features within one cluster have sequences that, when assembled, encode for a contiguous longer oligonucleotide of a predetermined sequence.

### Structure size

Structures for oligonucleotide synthesis for use with devices, compositions, systems, and methods for bioencryption and/or biodecryption as described herein comprise a variety of sizes. In some instances, a structure described herein is about the size of a standard 96 well plate, for example between about 100 and 200 mm by between about 50 and 150 mm. In some instances, a structure described herein has a diameter less than or equal to about 1000 mm, 500 mm, 450 mm, 400 mm, 300 mm, 250 nm, 200 mm, 150 mm, 100 mm or 50 mm. In some instances, the diameter of a substrate is between about 25 mm and 1000 mm, between about 25 mm and about 800 mm, between about 25 mm and about 600 mm, between about 25 mm and about 500 mm, between about 25 mm and about 400 mm, between about 25 mm and about 300 mm, or between about 25 mm and about 200. Non-limiting examples of substrate size include about 300 mm, 200 mm, 150 mm, 130 mm, 100 mm, 76 mm, 51 mm and 25 mm. In some instances, a substrate has a planar surface area of at least about 100 mm²; 200 mm²; 500 mm²; 1,000 mm²; 2,000 mm²; 5,000 mm²; 10,000 mm²; 12,000 mm²; 15,000 mm²; 20,000 mm²; 30,000 mm²; 40,000 mm²; 50,000 mm² or more. In some instances, the thickness of the substrate is between about 50 mm and about 2000 mm, between about 50 mm and about 1000 mm, between about 100 mm and about 1000 mm, between about 200 mm and about 1000 mm, or between about 250 mm and about 1000 mm. Non-limiting examples of substrate thickness include 275 mm, 375 mm, 525 mm, 625 mm, 675 mm, 725 mm, 775 mm and 925 mm. In some cases, the thickness of the substrate varies with diameter and depends on the composition of the substrate. For example, a structure comprising materials other than silicon may have a different thickness than a silicon structure of the same diameter. Structure thickness may be determined by the mechanical strength of the material used and the structure must be thick enough to support its own weight without cracking during handling. In some instances, a structure is more than about 1, 2, 3, 4, 5, 10, 15, 30, 40, 50 feet in any one dimension.

### Materials

Structures for oligonucleotide synthesis for use with devices, compositions, systems, and methods for bioencryption and/or biodecryption as described herein may be fabricated from a variety of materials. In certain instances, the materials from which the substrates/ solid supports of the disclosure are fabricated exhibit a low level of oligonucleotide binding. In some situations, material(s) that is (are) transparent to visible and/or UV light can be employed. Materials that are sufficiently conductive, e.g. those that can form uniform electric fields across all or a portion of the substrates/solids support described herein, can be utilized. In some instances, such materials may be connected to an electric ground. In some cases, the substrate or solid support can be heat conductive or insulated. The materials can be chemical resistant and heat resistant to support chemical or biochemical reactions such as a series of oligonucleotide synthesis reactions. For flexible materials, materials of interest can include: nylon, both modified and unmodified, nitrocellulose, polypropylene, and the like.

For rigid materials, specific materials of interest include: glass; fuse silica; silicon, plastics (for example polytetraflouroethylene, polypropylene, polystyrene, polycarbonate, and blends thereof, and the like); and metals (for example, gold, platinum, and the like). The structure can be fabricated from a material selected from the group consisting of silicon, polystyrene, agarose, dextran, cellulosic polymers, polyacrylamides, polydimethylsiloxane (PDMS), and glass. The substrates/solid supports or the microstructures, reactors therein may be manufactured with a combination of materials listed herein or any other suitable material known in the art.

The term "flexible" is used herein to refer to a structure that is capable of being bent, folded or similarly manipulated without breakage. In some cases, a flexible structure is bent at least 30 degrees around a roller. In some cases, a flexible structure is bent at least 180 degrees around a roller. In some cases, a flexible structure is bent at least 270 degrees around a roller. In some instances, a flexible structure is bent about 360 degrees around a roller. In some cases, the roller is less than about 10 cm, 5 cm, 3 cm, 2 cm or 1 cm in radius. In some instances, the flexible structure is bent and straightened repeatedly in either direction at least 100 times without failure (for example, cracking) or deformation at 20 °C. In some instances, a flexible structure described herein has a thickness that is amenable to rolling. In some cases, the thickness of the flexible structure described herein is less than about 50 mm, 10 mm, 1 mm, or 0.5 mm.

Exemplary flexible materials for structure described herein include, without limitation, nylon (unmodified nylon, modified nylon, clear nylon), nitrocellulose, polypropylene, polycarbonate, polyethylene, polyurethane, polystyrene, acetal, acrylic, acrylonitrile, butadiene styrene (ABS), polyester films such as polyethylene terephthalate, polymethyl methacrylate or other acrylics, polyvinyl chloride or other vinyl resin, transparent PVC foil, transparent foil for printers, poly(methyl methacrylate) (PMMA), methacrylate copolymers, styrenic polymers, high refractive index polymers, fluorine-containing polymers, polyethersulfone, polyimides containing an alicyclic structure, rubber, fabric, metal foils, and any combination thereof. Various plasticizers and modifiers may be used with polymeric substrate materials to achieve selected flexibility characteristics.

Flexible structures described herein may comprise a plastic material. In some instances, the flexible structure comprises a thermoplastic material. Non-limiting examples of thermoplastic materials include acrylic, acrylonitrile butadiene styrene, nylon, polylactic acid, polybenzimidazole, polycarbonate, polyether sulfone, polyetherether ketone, polyetherimide, polyethylene, polyphenylene oxide, polyphenylene sulfide, polypropylene, polystyrene, polyvinyl chloride, and polytetrafluoroethylene. In some instances, the substrate comprises a thermoplastic material in the polyaryletherketone (PEAK) family. Non-limiting examples of PEAK thermoplastics include polyetherketone (PEK), polyetherketoneketone (PEKK), poly(ether ether ketone ketone) (PEEKK), polyether ether ketone (PEEK), and polyetherketoneetherketoneketone (PEKEKK). In some instances, the flexible structure comprises a thermoplastic material compatible with toluene. In some instances, the flexibility of the plastic material is increased by the addition of a plasticizer. An example of a plasticizer is an ester-based plasticizer, such as phthalate. Phthalate plasticizers include bis(2-ethylhexyl) phthalate (DEHP), diisononly phthalate (DINP), di-n-butyl phthalate (DnBP, DBP), butyl benzyl phthalate (BBzP), diisodecyl phthalate (DIDP), dioctyl phthalate (DOP, DnOP), diisooctyl phthalate (DIOP), diethyl phthalate (DEP), diisobutyl phthalate (DIBP), and din-hexyl phthalate. In some instances, modification of the thermoplastic polymer through copolymerization or through the addition of non-reactive side chains to monomers before polymerization also increases flexibility.

Provided herein are flexible structures which may further comprise a fluoroelastomer. Materials having about 80% fluoroelastomers are designated as FKMs. Fluoroelastomers include perfluoro-elastomers (FFKMs) and tetrafluoroethylene/propylene rubbers (FEPM). Fluoroelastomers have five known types. Type 1 FKMs are composed of vinylidene fluoride (VDF) and hexafluoropropylene (HFP) and their fluorine content typically is around 66% by weight. Type 2 FKMs are composed of VDF, HFP, and tetrafluoroethylene (TFE) and typically have between about 68% and 69% fluorine. Type 3 FKMs are composed of VDF, TFE, and perfluoromethylvinylether (PMVE) and typically have between about 62% and 68% fluorine. Type 4 FKMs are composed of propylene, TFE, and VDF and typically have about 67% fluorine. Type 5 FKMs are composed of VDF, HFP, TFE, PMVE, and ethylene.

In some instances, a substrate disclosed herein comprises a computer readable material. Computer readable materials include, without limitation, magnetic media, reel-to-reel tape, cartridge tape, cassette tape, flexible disk, paper media, film, microfiche, continuous tape (e.g., a belt) and any media suitable for storing electronic instructions. In some cases, the substrate comprises magnetic reel-to-reel tape or a magnetic belt. In some instances, the substrate comprises a flexible printed circuit board.

Structures described herein may be transparent to visible and/or UV light. In some instances, structures described herein are sufficiently conductive to form uniform electric fields across all or a portion of a structure. In some instances, structures described herein are heat conductive or insulated. In some instances, the structures are chemical resistant and heat resistant to support a chemical reaction such as an oligonucleotide synthesis reaction. In some instances, the structure is magnetic. In some instances, the structures comprise a metal or a metal alloy.

Structures for oligonucleotide synthesis may be over 1, 2, 5, 10, 30, 50 or more feet long in any dimension. In the case of a flexible structure, the flexible structure is optionally stored in a wound state, e.g., in a reel. In the case of a large rigid structure, e.g., greater than 1 foot in length, the rigid structure can be stored vertically or horizontally.

### Encryption key markings on the structure's surface

Structures for oligonucleotide synthesis for use with devices, compositions, systems, and methods for bioencryption and/or biodecryption as described herein may comprise encryption markings. Provided herein are structures having markings **1401** wherein the markings provide information relating to the source item of information associated with a nearby population of oligonucleotides, an encryption scheme for decrypting the sequence of the nearby population of oligonucleotides, the copy number for the nearby population of oligonucleotides, or any combination thereof. *See, e.g.,* **FIGS. 14B-14C****.** The markings may be visible to the naked eye, or visible under a magnified view using a microscope. In some instances, the markings on the surface are only visible after a treatment condition to expose the marking, such as a heat, chemical or light treatment (e.g., UV or IR light to illuminate the marking). An example ink developed by heat includes, without limitation, cobalt chloride, (which turns blue when heated). Example inks developed by chemical reaction include, without limitation, phenolphthalein, copper sulfate, lead(II) nitrate, cobalt(II) chloride, and cerium oxalate developed by manganese sulfate and hydrogen peroxide.

### Surface Preparation

Structures for oligonucleotide synthesis for use with devices, compositions, systems, and methods for bioencryption and/or biodecryption as described herein may comprise a surface for oligonucleotide synthesis. Provided herein are methods to support the immobilization of a biomolecule on a substrate, where a surface of a structure described herein comprises a material and/or is coated with a material that facilitates a coupling reaction with the biomolecule for attachment. To prepare a structure for biomolecule immobilization, surface modifications may be employed that chemically and/or physically alter the substrate surface by an additive or subtractive process to change one or more chemical and/or physical properties of a substrate surface or a selected site or region of the surface. For example, surface modification involves (1) changing the wetting properties of a surface, (2) functionalizing a surface, i.e. providing, modifying or substituting surface functional groups, (3) defunctionalizing a surface, i.e. removing surface functional groups, (4) otherwise altering the chemical composition of a surface, e.g., through etching, (5) increasing or decreasing surface roughness, (6) providing a coating on a surface, e.g., a coating that exhibits wetting properties that are different from the wetting properties of the surface, and/or (7) depositing particulates on a surface. In some instances, the surface of a structure is selectively functionalized to produce two or more distinct areas on a structure, wherein at least one area has a different surface or chemical property that another area of the same structure. Such properties include, without limitation, surface energy, chemical termination, surface concentration of a chemical moiety, and the like.

In some instances, a surface of a structure disclosed herein is modified to comprise one or more actively functionalized surfaces configured to bind to both the surface of the substrate and a biomolecule, thereby supporting a coupling reaction to the surface. In some instances, the surface is also functionalized with a passive material that does not efficiently bind the biomolecule, thereby preventing biomolecule attachment at sites where the passive functionalization agent is bound. In some cases, the surface comprises an active layer only defining distinct features for biomolecule support.

In some instances, the surface is contacted with a mixture of functionalization groups which are in any different ratio. In some instances, a mixture comprises at least 2, 3, 4, 5 or more different types of functionalization agents. In some cases, the ratio of the at least two types of surface functionalization agents in a mixture is about 1:1, 1:2, 1:5, 1:10, 2:10, 3:10, 4:10, 5:10, 6:10, 7:10, 8:10, 9:10, or any other ratio to achieve a desired surface representation of two groups. In some instances, desired surface tensions, wettabilities, water contact angles, and/or contact angles for other suitable solvents are achieved by providing a substrate surface with a suitable ratio of functionalization agents. In some cases, the agents in a mixture are chosen from suitable reactive and inert moieties, thus diluting the surface density of reactive groups to a desired level for downstream reactions. In some instances, the mixture of functionalization reagents comprises one or more reagents that bind to a biomolecule and one or more reagents that do not bind to a biomolecule. Therefore, modulation of the reagents allows for the control of the amount of biomolecule binding that occurs at a distinct area of functionalization.

In some instances, a method for substrate functionalization comprises deposition of a silane molecule onto a surface of a substrate. The silane molecule may be deposited on a high energy surface of the substrate. In some instances the high surface energy region includes a passive functionalization reagent. Methods described herein provide for a silane group to bind the surface, while the rest of the molecule provides a distance from the surface and a free hydroxyl group at the end to which a biomolecule attaches. In some instances, the silane is an organofunctional alkoxysilane molecule. Non-limiting examples of organofunctional alkoxysilane molecules include dimethylchloro-octodecyl-silane, methyldichloro-octodecyl-silane, trichloro-octodecyl-silane, and trimethyl-octodecyl-silane, triethyl-octodecyl-silane. In some instances, the silane is an amino silane. Examples of amino silanes include, without limitation, 11-acetoxyundecyltriethoxysilane, n-decyltriethoxysilane, (3-aminopropyl)trimethoxysilane, (3-aminopropyl)triethoxysilane, glycidyloxypropyl/trimethoxysilane and N-(3-triethoxysilylpropyl)-4-hydroxybutyramide. In some instances, the silane comprises 11-acetoxyundecyltriethoxysilane, n-decyltriethoxysilane, (3-aminopropyl)trimethoxysilane, (3-aminopropyl)triethoxysilane, glycidyloxypropyl/trimethoxysilane, N-(3-triethoxysilylpropyl)-4-hydroxybutyramide, or any combination thereof. In some instances, an active functionalization agent comprises 11-acetoxyundecyltriethoxysilane. In some instances, an active functionalization agent comprises n-decyltriethoxysilane. In some cases, an active functionalization agent comprises glycidyloxypropyltriethoxysilane (GOPS). In some instances, the silane is a fluorosilane. In some instances, the silane is a hydrocarbon silane. In some cases, the silane is 3-iodo-propyltrimethoxysilane. In some cases, the silane is octylchlorosilane.

In some instances, silanization is performed on a surface through self-assembly with organofunctional alkoxysilane molecules. The organofunctional alkoxysilanes are classified according to their organic functions. Non-limiting examples of siloxane functionalizing reagents include hydroxyalkyl siloxanes (silylate surface, functionalizing with diborane and oxidizing the alcohol by hydrogen peroxide), diol (dihydroxyalkyl) siloxanes (silylate surface, and hydrolyzing to diol), aminoalkyl siloxanes (amines require no intermediate functionalizing step), glycidoxysilanes (3-glycidoxypropyl-dimethyl-ethoxysilane, glycidoxy-trimethoxysilane), mercaptosilanes (3-mercaptopropyl-trimethoxysilane, 3-4 epoxycyclohexyl-ethyltrimethoxysilane or 3-mercaptopropyl-methyl-dimethoxysilane), bicyclohepthenyl-trichlorosilane, butyl-aldehydr-trimethoxysilane, or dimeric secondary aminoalkyl siloxanes. Exemplary hydroxyalkyl siloxanes include allyl trichlorochlorosilane turning into 3-hydroxypropyl, or 7-oct-1-enyl trichlorochlorosilane turning into 8-hydroxyoctyl. The diol (dihydroxyalkyl) siloxanes include glycidyl trimethoxysilane-derived (2,3-dihydroxypropyloxy)propyl (GOPS). The aminoalkyl siloxanes include 3-aminopropyl trimethoxysilane turning into 3-aminopropyl (3-aminopropyltriethoxysilane, 3-aminopropyl-diethoxy-methylsilane, 3-aminopropyl-dimethyl-ethoxysilane, or 3-aminopropyl-trimethoxysilane). In some cases, the dimeric secondary aminoalkyl siloxanes is bis (3-trimethoxysilylpropyl) amine turning into bis(silyloxylpropyl)amine.

Active functionalization areas may comprise one or more different species of silanes, for example, 1, 2, 3, 4, 5, 6, 7, 8, 9, 10 or more silanes. In some cases, one of the one or more silanes is present in the functionalization composition in an amount greater than another silane. For example, a mixed silane solution having two silanes comprises a 99:1, 98:2, 97:3, 96:4, 95:5, 94:6, 93:7, 92:8, 91:9, 90:10, 89:11, 88:12, 87:13, 86:14, 85:15, 84:16, 83:17, 82:18, 81:19, 80:20, 75:25, 70:30, 65:35, 60:40, 55:45 ratio of one silane to another silane. In some instances, an active functionalization agent comprises 11-acetoxyundecyltriethoxysilane and n-decyltriethoxysilane. In some instances, an active functionalization agent comprises 11-acetoxyundecyltriethoxysilane and n-decyltriethoxysilane in a ratio from about 20:80 to about 1:99, or about 10:90 to about 2:98, or about 5:95.

In some instances, functionalization comprises deposition of a functionalization agent to a structure by any deposition technique, including, but not limiting to, chemical vapor deposition (CVD), atomic layer deposition (ALD), plasma enhanced CVD (PECVD), plasma enhanced ALD (PEALD), metal organic CVD (MOCVD), hot wire CVD (HWCVD), initiated CVD (iCVD), modified CVD (MCVD), vapor axial deposition (VAD), outside vapor deposition (OVD), physical vapor deposition (*e*.*g*., sputter deposition, evaporative deposition), and molecular layer deposition (MLD).

Any step or component in the following functionalization process be omitted or changed in accordance with properties desired of the final functionalized substrate. In some cases, additional components and/or process steps are added to the process workflows embodied herein. In some instances, a substrate is first cleaned, for example, using a piranha solution. An example of a cleaning process includes soaking a substrate in a piranha solution (*e*.*g*., 90% H₂SO₄, 10% H₂O₂) at an elevated temperature (*e.g.,* 120 °C) and washing (*e.g.,* water) and drying the substrate (*e.g.,* nitrogen gas). The process optionally includes a post piranha treatment comprising soaking the piranha treated substrate in a basic solution (*e.g.,* NH₄OH) followed by an aqueous wash (*e.g.,* water). In some instances, a surface of a structure is plasma cleaned, optionally following the piranha soak and optional post piranha treatment. An example of a plasma cleaning process comprises an oxygen plasma etch. In some instances, the surface is deposited with an active functionalization agent following by vaporization. In some instances, the substrate is actively functionalized prior to cleaning, for example, by piranha treatment and/or plasma cleaning.

The process for surface functionalization optionally comprises a resist coat and a resist strip. In some instances, following active surface functionalization, the substrate is spin coated with a resist, for example, SPR^{™} 3612 positive photoresist. The process for surface functionalization, in various instances, comprises lithography with patterned functionalization. In some instances, photolithography is performed following resist coating. In some instances, after lithography, the surface is visually inspected for lithography defects. The process for surface functionalization, in some instances, comprises a cleaning step, whereby residues of the substrate are removed, for example, by plasma cleaning or etching. In some instances, the plasma cleaning step is performed at some step after the lithography step.

In some instances, a surface coated with a resist is treated to remove the resist, for example, after functionalization and/or after lithography. In some cases, the resist is removed with a solvent, for example, with a stripping solution comprising N-methyl-2-pyrrolidone. In some cases, resist stripping comprises sonication or ultrasonication. In some instances, a resist is coated and stripped, followed by active functionalization of the exposed areas to create a desired differential functionalization pattern.

In some instances, the methods and compositions described herein relate to the application of photoresist for the generation of modified surface properties in selective areas, wherein the application of the photoresist relies on the fluidic properties of the surface defining the spatial distribution of the photoresist. Without being bound by theory, surface tension effects related to the applied fluid may define the flow of the photoresist. For example, surface tension and/or capillary action effects may facilitate drawing of the photoresist into small structures in a controlled fashion before the resist solvents evaporate. In some instances, resist contact points are pinned by sharp edges, thereby controlling the advance of the fluid. The underlying structures may be designed based on the desired flow patterns that are used to apply photoresist during the manufacturing and functionalization processes. A solid organic layer left behind after solvents evaporate may be used to pursue the subsequent steps of the manufacturing process. Structures may be designed to control the flow of fluids by facilitating or inhibiting wicking effects into neighboring fluidic paths. For example, a structure is designed to avoid overlap between top and bottom edges, which facilitates the keeping of the fluid in top structures allowing for a particular disposition of the resist. In an alternative example, the top and bottom edges overlap, leading to the wicking of the applied fluid into bottom structures. Appropriate designs may be selected accordingly, depending on the desired application of the resist.

In some instances, a structure described herein has a surface that comprises a material having a thickness of at least or at least about 0.1 nm, 0.5 nm, 1 nm, 2 nm, 5 nm, 10 nm or 25 nm that comprises a reactive group capable of binding nucleosides. Exemplary surfaces include, without limitation, glass and silicon, such as silicon dioxide and silicon nitride. In some cases, exemplary surfaces include nylon and PMMA.

In some instances, electromagnetic radiation in the form of UV light is used for surface patterning. In some instances, a lamp is used for surface patterning, and a mask mediates exposure locations of the UV light to the surface. In some instances, a laser is used for surface patterning, and a shutter opened/closed state controls exposure of the UV light to the surface. The laser arrangement may be used in combination with a flexible structure that is capable of moving. In such an arrangement, the coordination of laser exposure and flexible structure movement is used to create patterns of one or more agents having differing nucleoside coupling capabilities.

### Material Deposition Systems

Provided herein are systems and devices for the deposition and storage of biomolecules on a structure described herein. In some instances, the biomolecules are oligonucleotides that store encoded information in their sequences. In some instances, the system comprises a surface of a structure to support biomolecule attachment and/or a device for application of a biomolecule to the surface of the substrate. In an example, the device for biomolecule application is an oligonucleotide synthesizer. In some instances, the system comprises a device for treating the substrate with a fluid, for example, a flow cell. In some instances, the system comprises a device for moving the substrate between the application device and the treatment device. For instances where the substrate is a reel-to-reel tape, the system may comprise two or more reels that allow for access of different portions of the substrate to the application and optional treatment device at different times.

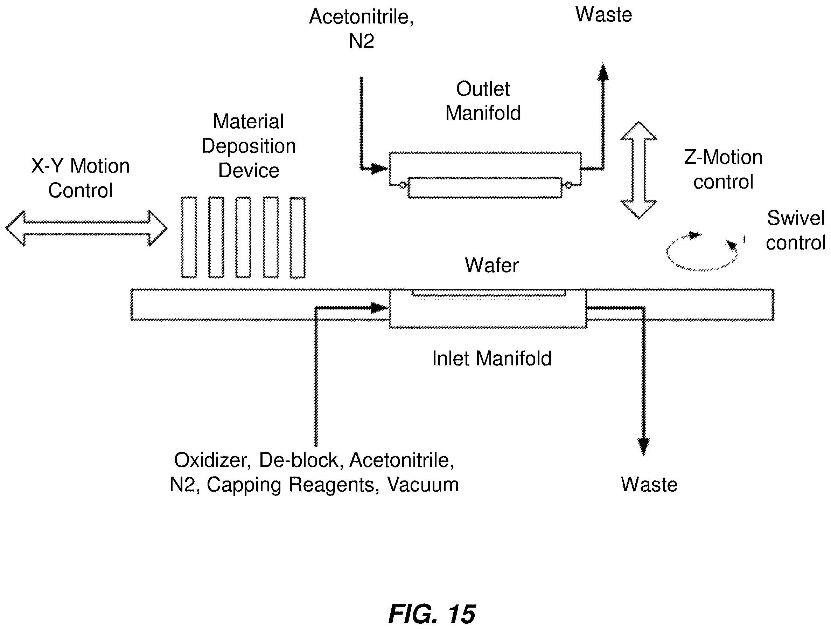

A first example of an oligonucleotide material deposition system for oligonucleotide synthesis is shown in **FIG. 15****.** The system includes a material deposition device that moves in the X-Y direction to align with the location of the substrate. The material deposition device can also move in the Z direction to seal with the substrate, forming a resolved reactor. A resolved reactor is configured to allow for the transfer of fluid, including oligonucleotides and/or reagents, from the substrate to a capping element and/or vice versa. As shown in **FIG. 15****,** fluid may pass through either or both the substrate and the capping element and includes, without limitation, coupling reagents, capping reagents, oxidizers, de-blocking agents, acetonitrile and nitrogen gas. Examples of devices that are capable of high resolution droplet deposition include the printhead of inkjet printers and laser printers. The devices useful in the systems and methods described herein achieve a resolution from about 100 dots per inch (DPI) to about 50,000 DPI; from about 100 DPI to about 20,000 DPI; from about 100 DPI to about 10,000 DPI; from about 100 DPI to about 5,000 DPI; from about 1,000 DPI to about 20,000 DPI; or from about 1,000 DPI to about 10,000 DPI. In some instances, the devices have a resolution at least about 1,000; 2,000; 3,000; 4,000; 5,000; 10,000; 12,000 DPI, or 20,000 DPI. The high resolution deposition performed by the device is related to the number and density of each nozzle that corresponds to a feature of the substrate.

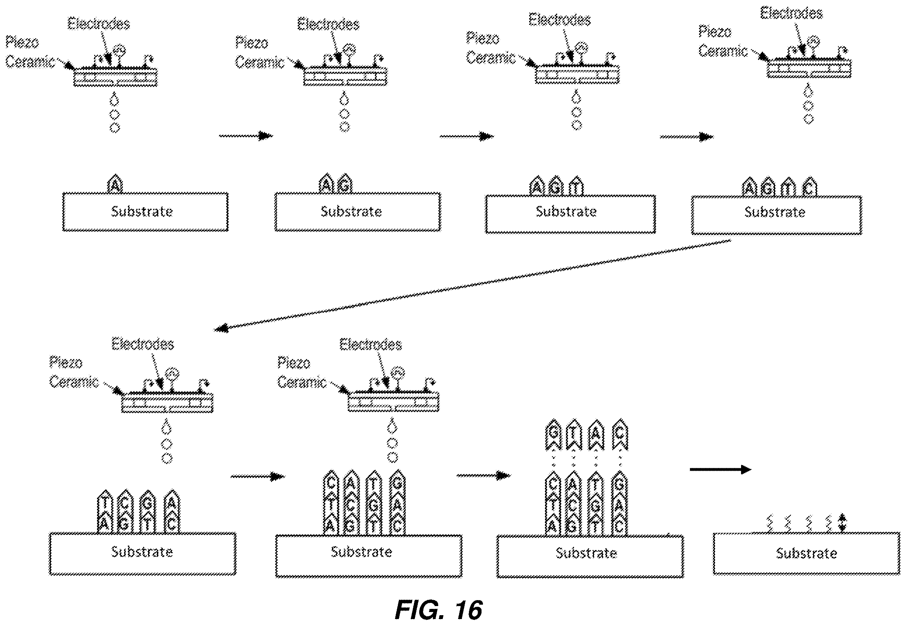

An exemplary process workflow for de novo synthesis of an oligonucleotide on a substrate using an oligonucleotide synthesizer is shown in **FIG. 16****.** Droplets comprising oligonucleotide synthesis reagents are released from the material deposition device to the substrate in a stepwise manner, wherein the material deposition device has a piezo ceramic material and electrodes to convert electrical signals into a mechanical signal for releasing the droplets. The droplets are released to specific locations on the surface of the substrate one nucleobase at a time to generate a plurality of synthesized oligonucleotides having predetermined sequences that encode data. In some cases, the synthesized oligonucleotides are stored on the substrate. Nucleic acid reagents may be deposited on the substrate surface in a non-continuous, or drop-on-demand method. Examples of such methods include the electromechanical transfer method, electric thermal transfer method, and electrostatic attraction method. In the electromechanical transfer method, piezoelectric elements deformed by electrical pulses cause the droplets to be ejected. In the electric thermal transfer method, bubbles are generated in a chamber of the device, and the expansive force of the bubbles causes the droplets to be ejected. In the electrostatic attraction method, electrostatic force of attraction is used to eject the droplets onto the substrate. In some cases, the drop frequency is from about 5 KHz to about 500 KHz; from about 5 KHz to about 100 KHz; from about 10 KHz to about 500 KHz; from about 10 KHz to about 100 KHz; or from about 50 KHz to about 500 KHz. In some cases, the frequency is less than about 500 KHz, 200 KHz, 100 KHz, or 50 KHz.

The size of the droplets dispensed correlates to the resolution of the device. In some instances, the devices deposit droplets of reagents at sizes from about 0.01 pl to about 20 pl, from about 0.01 pl to about 10 pl, from about 0.01 pl to about 1 pl, from about 0.01 pl to about 0.5 pl, from about 0.01 pl to about 0.01 pl, or from about 0.05 pl to about 1 pl. In some instances, the droplet size is less than about 1 pl, 0.5 pl, 0.2 pl, 0.1 pl, or 0.05 pl. The size of droplets dispensed by the device is correlated to the diameters of deposition nozzles, wherein each nozzle is capable of depositing a reagent onto a feature of the substrate. In some instances, a deposition device of an oligonucleotide synthesizer comprises from about 100 to about 10,000 nozzles; from about 100 to about 5,000 nozzles; from about 100 to about 3,000 nozzles; from about 500 to about 10,000 nozzles; or from about 100 to about 5,000 nozzles. In some cases, the deposition device comprises greater than 1,000; 2,000; 3,000; 4,000; 5,000; or 10,000 nozzles. In some instances, each material deposition device comprises a plurality of nozzles, where each nozzle is optionally configured to correspond to a feature on a substrate. Each nozzle may deposit a reagent component that is different from another nozzle. In some instances, each nozzle deposits a droplet that covers one or more features of the substrate. In some instances, one or more nozzles are angled. In some instances, multiple deposition devices are stacked side by side to achieve a fold increase in throughput. In some cases, the gain is 2x, 4x, 8x or more. An example of a deposition device is Samba Printhead (Fujifilm). A Samba Printhead may be used with the Samba Web Administration Tool (SWAT).

The number of deposition sites may be increased by using and rotating the same deposition device by a certain degree or saber angle. By rotating the deposition device, each nozzle is jetted with a certain amount of delay time corresponding to the saber angle. This unsynchronized jetting creates a cross talk among the nozzles. Therefore, when the droplets are jetting at a certain saber angle different from 0 degrees, the droplet volume from the nozzle could be different.

In some arrangements, the configuration of an oligonucleotide synthesis system allows for a continuous oligonucleotide synthesis process that exploits the flexibility of a substrate for traveling in a reel-to-reel type process. This synthesis process operates in a continuous production line manner with the substrate travelling through various stages of oligonucleotide synthesis using one or more reels to rotate the position of the substrate. In an exemplary embodiment, an oligonucleotide synthesis reaction comprises rolling a substrate: through a solvent bath, beneath a deposition device for phosphoramidite deposition, through a bath of oxidizing agent, through an acetonitrile wash bath, and through a deblock bath. Optionally, the tape is also traversed through a capping bath. A reel-to-reel type process allows for the finished product of a substrate comprising synthesized oligonucleotides to be easily gathered on a take-up reel, where it can be transported for further processing or storage.

In some arrangements, oligonucleotide synthesis proceeds in a continuous process as a continuous flexible tape is conveyed along a conveyor belt system. Similar to the reel-to-reel type process, oligonucleotide synthesis on a continuous tape operates in a production line manner, with the substrate travelling through various stages of oligonucleotide synthesis during conveyance. However, in a conveyor belt process, the continuous tape revisits an oligonucleotide synthesis step without rolling and unrolling of the tape, as in a reel-to-reel process. In some arrangements, oligonucleotide synthesis steps are partitioned into zones and a continuous tape is conveyed through each zone one or more times in a cycle. For example, an oligonucleotide synthesis reaction may comprise (1) conveying a substrate through a solvent bath, beneath a deposition device for phosphoramidite deposition, through a bath of oxidizing agent, through an acetonitrile wash bath, and through a block bath in a cycle; and then (2) repeating the cycles to achieve synthesized oligonucleotides of a predetermined length. After oligonucleotide synthesis, the flexible substrate is removed from the conveyor belt system and, optionally, rolled for storage. Rolling may be around a reel, for storage.

In an exemplary arrangement, a flexible substrate comprising thermoplastic material is coated with nucleoside coupling reagent. The coating is patterned into features such that each feature has diameter of about 10 um, with a center-to-center distance between two adjacent features of about 21 um. In this instance, the feature size is sufficient to accommodate a sessile drop volume of 0.2 pl during an oligonucleotide synthesis deposition step. In some cases, the feature density is about 2.2 billion features per m² (1 feature / 441 x 10⁻¹² m²). In some cases, a 4.5 m² substrate comprise about 10 billion features, each with a 10 um diameter.

A material deposition device described herein may comprise about 2,048 nozzles that each deposit about 100,000 droplets per second at 1 nucleobase per droplet. For each deposition device, at least about 1.75 x 10¹³ nucleobases are deposited on the substrate per day. In some instances, 100 to 500 nucleobase oligonucleotides are synthesized. In some cases, 200 nucleobase oligonucleotides are synthesized. Optionally, over 3 days, at a rate of about 1.75 x 10¹³ bases per day, at least about 262.5 x 10⁹ oligonucleotides are synthesized.

In some arrangements, a device for application of one or more reagents to a substrate during a synthesis reaction is configured to deposit reagents and /or nucleotide monomers for nucleoside phosphoramidite based synthesis. Reagents for oligonucleotide synthesis include reagents for oligonucleotide extension and wash buffers. As non-limiting examples, the device deposits cleaning reagents, coupling reagents, capping reagents, oxidizers, de-blocking agents, acetonitrile, gases such as nitrogen gas, and any combination thereof. In addition, the device optionally deposits reagents for the preparation and/or maintenance of substrate integrity. In some instances, the oligonucleotide synthesizer deposits a drop having a diameter less than about 200 um, 100 um, or 50 um in a volume less than about 1000, 500, 100, 50, or 20 pl. In some cases, the oligonucleotide synthesizer deposits between about 1 and 10000, 1 and 5000, 100 and 5000, or 1000 and 5000 droplets per second.

In some arrangements, during oligonucleotide synthesis, the substrate is positioned within and/or sealed within a flow cell. The flow cell may provide continuous or discontinuous flow of liquids such as those comprising reagents necessary for reactions within the substrate, for example, oxidizers and/or solvents. The flow cell may provide continuous or discontinuous flow of a gas, such as nitrogen, for drying the substrate typically through enhanced evaporation of a volatile substrate. A variety of auxiliary devices are useful to improve drying and reduce residual moisture on the surface of the substrate. Examples of such auxiliary drying devices include, without limitation, a vacuum source, depressurizing pump and a vacuum tank. In some cases, an oligonucleotide synthesis system comprises one or more flow cells, such as 2, 3, 4, 5, 6, 7, 8, 9, 10, or 20 and one or more substrates, such as 2, 3, 4, 5, 6, 7, 8, 9, 10 or 20. In some cases, a flow cell is configured to hold and provide reagents to the substrate during one or more steps in a synthesis reaction. In some instances, a flowcell comprises a lid that slides over the top of a substrate and can be clamped into place to form a pressure tight seal around the edge of the substrate. An adequate seal includes, without limitation, a seal that allows for about 1, 2, 3, 4, 5, 6, 7, 8, 9 or 10 atmospheres of pressure. In some cases, the lid of the flow cell is opened to allow for access to an application device such as an oligonucleotide synthesizer. In some cases, one or more steps of an oligonucleotide synthesis method are performed on a substrate within a flow cell, without the transport of the substrate.

In some arrangements, a device for treating a substrate with a fluid comprises a spray bar. Nucleotide monomers may be applied onto a substrate surface then a spray bar sprays the substrate surface with one or more treatment reagents using spray nozzles of the spray bar. In some arrangements, the spray nozzles are sequentially ordered to correlate with different treatment steps during oligonucleotide synthesis. The chemicals used in different process steps may be changed in the spray bar to readily accommodate changes in a synthesis method or between steps of a synthesis method. In some instances, the spray bar continuously sprays a given chemistry on a surface of a substrate as the substrate moves past the spray bar. In some cases, the spray bar deposits over a wide area of a substrate, much like the spray bars used in lawn sprinklers. In some instances, the spray bar nozzles are positioned to provide a uniform coat of treatment material to a given area of a substrate.

In some instances, an oligonucleotide synthesis system comprises one or more elements useful for downstream processing of synthesized oligonucleotides. As an example, the system comprises a temperature control element such as a thermal cycling device. In some instances, the temperature control element is used with a plurality of resolved reactors to perform nucleic acid assembly such as PCA and/or nucleic acid amplification such as PCR.

### De Novo Oligonucleotide Synthesis

Provided herein are systems and methods for oligonucleotide synthesis of a high density of oligonucleotides on a substrate in a short amount of time for use with devices, compositions, systems, and methods for bioencryption and/or biodecryption as described herein. In some instances, the substrate is a flexible substrate. In some instances, at least about 10¹⁰, 10¹¹, 10¹², 10¹³, 10¹⁴, or 10¹⁵ bases are synthesized in one day. In some instances, at least about 10 x 10⁸, 10 x 10⁹, 10 x 10¹⁰, 10 x 10¹¹, or 10 x 10¹² oligonucleotides are synthesized in one day. In some cases, each oligonucleotide synthesized comprises at least about 20, 50, 100, 200, 300, 400 or 500 nucleobases. In some cases, these bases are synthesized with a total average error rate of less than about 1 in 100; 200; 300; 400; 500; 1000; 2000; 5000; 10000; 15000; 20000 bases. In some instances, these error rates are for at least 50%, 60%, 70%, 80%, 90%, 95%, 98%, 99%, 99.5%, or more of the oligonucleotides synthesized. In some instances, these at least 90%, 95%, 98%, 99%, 99.5%, or more of the oligonucleotides synthesized do not differ from a predetermined sequence for which they encode. In some instances, the error rate for synthesized oligonucleotides on a substrate using the methods and systems described herein is less than about 1 in 200. In some instances, the error rate for synthesized oligonucleotides on a substrate using the methods and systems described herein is less than about 1 in 1,000. In some instances, the error rate for synthesized oligonucleotides on a substrate using the methods and systems described herein is less than about 1 in 2,000. In some instances, the error rate for synthesized oligonucleotides on a substrate using the methods and systems described herein is less than about 1 in 3,000. In some instances, the error rate for synthesized oligonucleotides on a substrate using the methods and systems described herein is less than about 1 in 5,000. Individual types of error rates include mismatches, deletions, insertions, and/or substitutions for the oligonucleotides synthesized on the substrate. The term "error rate" refers to a comparison of the collective amount of synthesized oligonucleotide to an aggregate of predetermined oligonucleotide sequences. In some instances, synthesized oligonucleotides disclosed herein comprise a tether of 12 to 25 bases. In some instances, the tether comprises 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 or more bases.

A suitable method for oligonucleotide synthesis on a substrate of this disclosure is a phosphoramidite method comprising the controlled addition of a phosphoramidite building block, i.e. nucleoside phosphoramidite, to a growing oligonucleotide chain in a coupling step that forms a phosphite triester linkage between the phosphoramidite building block and a nucleoside bound to the substrate. In some instances, the nucleoside phosphoramidite is provided to the substrate activated. In some instances, the nucleoside phosphoramidite is provided to the substrate with an activator. In some instances, nucleoside phosphoramidites are provided to the substrate in a 1.5, 2, 3, 4, 5, 6, 7, 8, 9, 10, 11, 12, 13, 14, 15, 16, 17, 18, 19, 20, 25, 30, 35, 40, 50, 60, 70, 80, 90, 100-fold excess or more over the substrate-bound nucleosides. In some instances, the addition of nucleoside phosphoramidite is performed in an anhydrous environment, for example, in anhydrous acetonitrile. Following addition and linkage of a nucleoside phosphoramidite in the coupling step, the substrate is optionally washed. In some instances, the coupling step is repeated one or more additional times, optionally with a wash step between nucleoside phosphoramidite additions to the substrate. In some instances, an oligonucleotide synthesis method used herein comprises 1, 2, 3 or more sequential coupling steps. Prior to coupling, in many cases, the nucleoside bound to the substrate is de-protected by removal of a protecting group, where the protecting group functions to prevent polymerization. A common protecting group is 4,4'-dimethoxytrityl (DMT).

Following coupling, phosphoramidite oligonucleotide synthesis methods optionally comprise a capping step. In a capping step, the growing oligonucleotide is treated with a capping agent. A capping step generally serves to block unreacted substrate-bound 5'-OH groups after coupling from further chain elongation, preventing the formation of oligonucleotides with internal base deletions. Further, phosphoramidites activated with 1H-tetrazole often react, to a small extent, with the O6 position of guanosine. Without being bound by theory, upon oxidation with I₂ /water, this side product, possibly via O6-N7 migration, undergoes depurination. The apurinic sites can end up being cleaved in the course of the final deprotection of the oligonucleotide thus reducing the yield of the full-length product. The O6 modifications may be removed by treatment with the capping reagent prior to oxidation with I₂/water. In some instances, inclusion of a capping step during oligonucleotide synthesis decreases the error rate as compared to synthesis without capping. As an example, the capping step comprises treating the substrate-bound oligonucleotide with a mixture of acetic anhydride and 1-methylimidazole. Following a capping step, the substrate is optionally washed.

Following addition of a nucleoside phosphoramidite, and optionally after capping and one or more wash steps, the substrate bound growing nucleic acid may be oxidized. The oxidation step comprises oxidizing the phosphite triester into a tetracoordinated phosphate triester, a protected precursor of the naturally occurring phosphate diester internucleoside linkage. In some instances, oxidation of the growing oligonucleotide is achieved by treatment with iodine and water, optionally in the presence of a weak base such as a pyridine, lutidine, or collidine. Oxidation is sometimes carried out under anhydrous conditions using tert-Butyl hydroperoxide or (1S)-(+)-(10-camphorsulfonyl)-oxaziridine (CSO). In some methods, a capping step is performed following oxidation. A second capping step allows for substrate drying, as residual water from oxidation that may persist can inhibit subsequent coupling. Following oxidation, the substrate and growing oligonucleotide is optionally washed. In some instances, the step of oxidation is substituted with a sulfurization step to obtain oligonucleotide phosphorothioates, wherein any capping steps can be performed after the sulfurization. Many reagents are capable of the efficient sulfur transfer, including, but not limited to, 3-(Dimethylaminomethylidene)amino)-3H-1,2,4-dithiazole-3-thione, DDTT, 3H-1,2-benzodithiol-3-one 1,1-dioxide, also known as Beaucage reagent, and N,N,N'N'-Tetraethylthiuram disulfide (TETD).

In order for a subsequent cycle of nucleoside incorporation to occur through coupling, a protected 5' end of the substrate bound growing oligonucleotide must be removed so that the primary hydroxyl group can react with a next nucleoside phosphoramidite. In some instances, the protecting group is DMT and deblocking occurs with trichloroacetic acid in dichloromethane. Conducting detritylation for an extended time or with stronger than recommended solutions of acids may lead to increased depurination of solid support-bound oligonucleotide and thus reduce the yield of the desired full-length product. Methods and compositions described herein provide for controlled deblocking conditions limiting undesired depurination reactions. In some instances, the substrate bound oligonucleotide is washed after deblocking. In some cases, efficient washing after deblocking contributes to synthesized oligonucleotides having a low error rate.

Methods for the synthesis of oligonucleotides on the substrates described herein typically involve an iterating sequence of the following steps: application of a protected monomer to a surface of a substrate feature to link with either the surface, a linker or with a previously deprotected monomer; deprotection of the applied monomer so that it can react with a subsequently applied protected monomer; and application of another protected monomer for linking. One or more intermediate steps include oxidation and/or sulfurization. In some instances, one or more wash steps precede or follow one or all of the steps.

In some instances, oligonucleotides are synthesized with photolabile protecting groups, where the hydroxyl groups generated on the surface are blocked by photolabile-protecting groups. When the surface is exposed to UV light, such as through a photolithographic mask, a pattern of free hydroxyl groups on the surface may be generated. These hydroxyl groups can react with photoprotected nucleoside phosphoramidites, according to phosphoramidite chemistry. A second photolithographic mask can be applied and the surface can be exposed to UV light to generate second pattern of hydroxyl groups, followed by coupling with 5'-photoprotected nucleoside phosphoramidite. Likewise, patterns can be generated and oligomer chains can be extended. Without being bound by theory, the lability of a photocleavable group depends on the wavelength and polarity of a solvent employed and the rate of photocleavage may be affected by the duration of exposure and the intensity of light. This method can leverage a number of factors such as accuracy in alignment of the masks, efficiency of removal of photo-protecting groups, and the yields of the phosphoramidite coupling step. Further, unintended leakage of light into neighboring sites can be minimized. The density of synthesized oligomer per spot can be monitored by adjusting loading of the leader nucleoside on the surface of synthesis.

The surface of the substrate that provides support for oligonucleotide synthesis may be chemically modified to allow for the synthesized oligonucleotide chain to be cleaved from the surface. In some instances, the oligonucleotide chain is cleaved at the same time as the oligonucleotide is deprotected. In some cases, the oligonucleotide chain is cleaved after the oligonucleotide is deprotected. In an exemplary scheme, a trialkoxysilyl amine such as (CH3CH2O)3Si-(CH2)2-NH2 is reacted with surface SiOH groups of a substrate, followed by reaction with succinic anhydride with the amine to create an amide linkage and a free OH on which the nucleic acid chain growth is supported. Cleavage includes gas cleavage with ammonia or methylamine. In some instances, once released from the surface, oligonucleotides are assembled into larger nucleic acids that are sequenced and decoded to extract stored information.

Oligonucleotides may be designed to collectively span a large region of a predetermined sequence that encodes for information. In some instances, larger oligonucleotides are generated through ligation reactions to join the synthesized oligonucleotides. One example of a ligation reaction is polymerase chain assembly (PCA). In some instances, at least a portion of the oligonucleotides are designed to include an appended region that is a substrate for universal primer binding. For PCA reactions, the presynthesized oligonucleotides include overlaps with each other (*e.g.,* 4, 20, 40 or more bases with overlapping sequence). During the polymerase cycles, the oligonucleotides anneal to complementary fragments and then are filled in by polymerase. Each cycle thus increases the length of various fragments randomly depending on which oligonucleotides find each other. Complementarity amongst the fragments allows for forming a complete large span of double-stranded DNA. In some cases, after the PCA reaction is complete, an error correction step is conducted using mismatch repair detecting enzymes to remove mismatches in the sequence. Once larger fragments of a target sequence are generated, they can be amplified. For example, in some cases, a target sequence comprising 5' and 3' terminal adapter sequences is amplified in a polymerase chain reaction (PCR) which includes modified primers that hybridize to the adapter sequences. In some cases, the modified primers comprise one or more uracil bases. The use of modified primers allows for removal of the primers through enzymatic reactions centered on targeting the modified base and/or gaps left by enzymes which cleave the modified base pair from the fragment. What remains is a double-stranded amplification product that lacks remnants of adapter sequence. In this way, multiple amplification products can be generated in parallel with the same set of primers to generate different fragments of double-stranded DNA.

Error correction may be performed on synthesized oligonucleotides and/or assembled products. An example strategy for error correction involves site-directed mutagenesis by overlap extension PCR to correct errors, which is optionally coupled with two or more rounds of cloning and sequencing. In certain instances, double-stranded nucleic acids with mismatches, bulges and small loops, chemically altered bases and/or other heteroduplexes are selectively removed from populations of correctly synthesized nucleic acids. In some instances, error correction is performed using proteins/enzymes that recognize and bind to or next to mismatched or unpaired bases within double-stranded nucleic acids to create a single or double-strand break or to initiate a strand transfer transposition event. Non-limiting examples of proteins/enzymes for error correction include endonucleases (T7 Endonuclease I, E. coli Endonuclease V, T4 Endonuclease VII, mung bean nuclease, Cell, E. coli Endonuclease IV, UVDE), restriction enzymes, glycosylases, ribonucleases, mismatch repair enzymes, resolvases, helicases, ligases, antibodies specific for mismatches, and their variants. Examples of specific error correction enzymes include T4 endonuclease 7, T7 endonuclease 1, S1, mung bean endonuclease, MutY, MutS, MutH, MutL, cleavase, CELI, and HINF1. In some cases, DNA mismatch-binding protein MutS (*Thermus aquaticus*) is used to remove failure products from a population of synthesized products. In some instances, error correction is performed using the enzyme Correctase. In some cases, error correction is performed using SURVEYOR endonuclease (Transgenomic), a mismatch-specific DNA endonuclease that scans for known and unknown mutations and polymorphisms for heteroduplex DNA.

### Release, Extraction and Assembly

Provided herein are method and devices for replicable information storage. In some instances, multiple copies of the same coding region, the oligonucleotide, the same cluster, the same portion of a structure comprising oligonucleotides, or the entire structure comprising oligonucleotides are synthesized. Where multiple copies of the same oligonucleotide are synthesized, each of the oligonucleotides may be attached to distinct regions of the surface. The distinct regions may be separated by breaking or cutting. Alternatively, each of the oligonucleotides may be present at a feature in the form of a spot, well or channel and individually accessible. For example, contacting the feature with a cleavage reagent and then water would free one copy of the oligonucleotide while leaving the other copies intact. Similarly, cleavage of oligonucleotides in an entire region or over an entire plate allows for accessing a fraction of a replicate population. Replicate populations may exist in separated reels, plates, belts, and the like. In the case of a flexible material, such as a tape, a replicate region may be cut and the remaining regions of the tape may be spliced back together. Alternatively, nucleic acid information of the synthesized and stored oligonucleotides may be obtained by performing amplification of oligonucleotides attached to the surface of the structure using primers and a DNA polymerase.

In some instances, an aqueous or gaseous transfer media is deposited onto one or a plurality of channels in a structure to transfer the oligonucleotides from the structure to a receiving unit. For example, a transfer media may pass through a channel in the structure to adhere to, collect and transfer an oligonucleotide from a channel in the structure to a receiving unit. In some instances, a charge conducting feature and an applied voltage are employed to attract or repel a transfer media to or through a channel in the structure. In some instances, a slip is employed to direct a transfer media into a channel in the structure. In some cases a pressure release is employed to direct a transfer media into or through a channel in the structure. In some cases a nozzle is employed to form a localized area of high pressure which forces a transfer media into or through a channel in the structure. In some instances, a pin is employed to transfer an oligonucleotide from a channel in the structure to a container to a receiving unit. In such instances, the pin may comprise agents to facilitate transfer media adhesion. In some cases a charge conducting feature is employed to attract or repel a transfer media to or through a channel in a structure, by forming a voltage potential between the conducting feature and the structure. In some cases, a pipette tip, or other capillary flow inducing structure, is used to transfer the fluid and oligonucleotides via capillary flow. In some instances, a container comprises one or more compartments that each receives a portion of the transfer media, and the one or more oligonucleotides therein, emitted from a single respective channel. In some instances, the container comprises a single compartment that receives one or more portions of the transfer media, each containing one or more oligonucleotides therein, emitted from a one or more structure channels.

### Sequencing

After extraction and/or amplification of oligonucleotides from the surface of the structure, suitable sequencing technology may be employed to sequence the oligonucleotides. In some cases, the DNA sequence is read on the substrate or within a feature of a structure. In some cases, the oligonucleotides stored on the substrate are extracted, optionally assembled into longer nucleic acids and then sequenced.

Oligonucleotides synthesized and stored on the structures described herein encode data that can be interpreted by reading the sequence of the synthesized oligonucleotides and converting the sequence into binary code readable by a computer. In some cases the sequences require assembly, and the assembly step may need to be at the nucleic acid sequence stage or at the digital sequence stage.

Provided herein are detection systems comprising a device capable of sequencing stored oligonucleotides, either directly on the structure and/or after removal from the main structure. In cases where the structure is a reel-to-reel tape of flexible material, the detection system comprises a device for holding and advancing the structure through a detection location and a detector disposed proximate the detection location for detecting a signal originated from a section of the tape when the section is at the detection location. In some instances, the signal is indicative of a presence of an oligonucleotide. In some instances, the signal is indicative of a sequence of an oligonucleotide (e.g., a fluorescent signal). In some instances, information encoded within oligonucleotides on a continuous tape is read by a computer as the tape is conveyed continuously through a detector operably connected to the computer. In some instances, a detection system comprises a computer system comprising an oligonucleotide sequencing device, a database for storage and retrieval of data relating to oligonucleotide sequence, software for converting DNA code of an oligonucleotide sequence to binary code, a computer for reading the binary code, or any combination thereof.

### Computer Systems

In various aspects, any of the systems described herein are operably linked to a computer and are optionally automated through a computer either locally or remotely. In various instances, the methods and systems of the disclsoure further comprise software programs on computer systems and use thereof. Accordingly, computerized control for the synchronization of the dispense/vacuum/refill functions such as orchestrating and synchronizing the material deposition device movement, dispense action and vacuum actuation are within the bounds of the disclosure. In some instances, the computer systems are programmed to interface between the user specified base sequence and the position of a material deposition device to deliver the correct reagents to specified regions of the substrate.

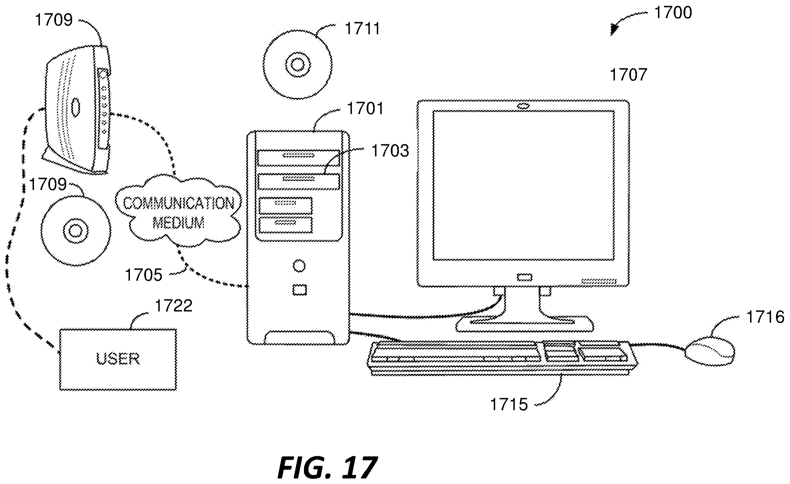

The computer system **1700** illustrated in **FIG. 17** may be understood as a logical apparatus that can read instructions from media **1711** and/or a network port **1705,** which can optionally be connected to server **1709** having fixed media **1712.** The system, such as shown in **FIG. 17** can include a CPU **1701,** disk drives **1703,** optional input devices such as keyboard **1715** and/or mouse **1716** and optional monitor **1707.** Data communication can be achieved through the indicated communication medium to a server at a local or a remote location. The communication medium can include any means of transmitting and/or receiving data. For example, the communication medium can be a network connection, a wireless connection or an internet connection. Such a connection can provide for communication over the World Wide Web. It is envisioned that data relating to the present disclosure can be transmitted over such networks or connections for reception and/or review by a party **1722.**

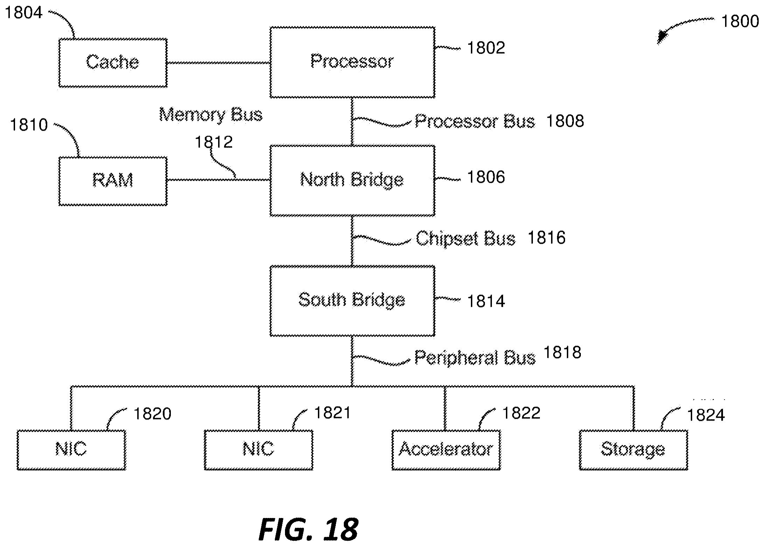

**FIG. 18** is a block diagram illustrating a first example architecture of a computer system **1800** that can be used in connection with example embodiments of the present disclosure. As depicted in **FIG. 18****,** the example computer system can include a processor **1802** for processing instructions. Non-limiting examples of processors include: Intel XeonTM processor, AMD OpteronTM processor, Samsung 32-bit RISC ARM 1176JZ(F)-S v1.0TM processor, ARM Cortex-A8 Samsung SSPC100TM processor, ARM Cortex-A8 Apple A4TM processor, Marvell PXA 930TM processor, or a functionally-equivalent processor. Multiple threads of execution can be used for parallel processing. In some instances, multiple processors or processors with multiple cores can also be used, whether in a single computer system, in a cluster, or distributed across systems over a network comprising a plurality of computers, cell phones, and/or personal data assistant devices.

As illustrated in **FIG. 18****,** a high speed cache **1804** can be connected to, or incorporated in, the processor **1802** to provide a high speed memory for instructions or data that have been recently, or are frequently, used by processor **1802.** The processor **1802** is connected to a north bridge **1806** by a processor bus **1808.** The north bridge **1806** is connected to random access memory (RAM) **1810** by a memory bus **1812** and manages access to the RAM **1810** by the processor **1802.** The north bridge **1806** is also connected to a south bridge **1814** by a chipset bus **1816.** The south bridge **1814** is, in turn, connected to a peripheral bus **1818.** The peripheral bus can be, for example, PCI, PCI-X, PCI Express, or other peripheral bus. The north bridge and south bridge are often referred to as a processor chipset and manage data transfer between the processor, RAM, and peripheral components on the peripheral bus **1818.** In some alternative architectures, the functionality of the north bridge can be incorporated into the processor instead of using a separate north bridge chip.

In some instances, system **1800** can include an accelerator card **1822** attached to the peripheral bus **1818.** The accelerator can include field programmable gate arrays (FPGAs) or other hardware for accelerating certain processing. For example, an accelerator can be used for adaptive data restructuring or to evaluate algebraic expressions used in extended set processing.

Software and data are stored in external storage **1824** and can be loaded into RAM **1810** and/or cache **1804** for use by the processor. The system **1800** includes an operating system for managing system resources; non-limiting examples of operating systems include: Linux, WindowsTM, MACOSTM, iOSTM, and other functionally-equivalent operating systems, as well as application software running on top of the operating system for managing data storage and optimization in accordance with example embodiments of the present disclosure.

In this example, system **1800** also includes network interface cards (NICs) **1820** and **1821** connected to the peripheral bus for providing network interfaces to external storage, such as Network Attached Storage (NAS) and other computer systems that can be used for distributed parallel processing.

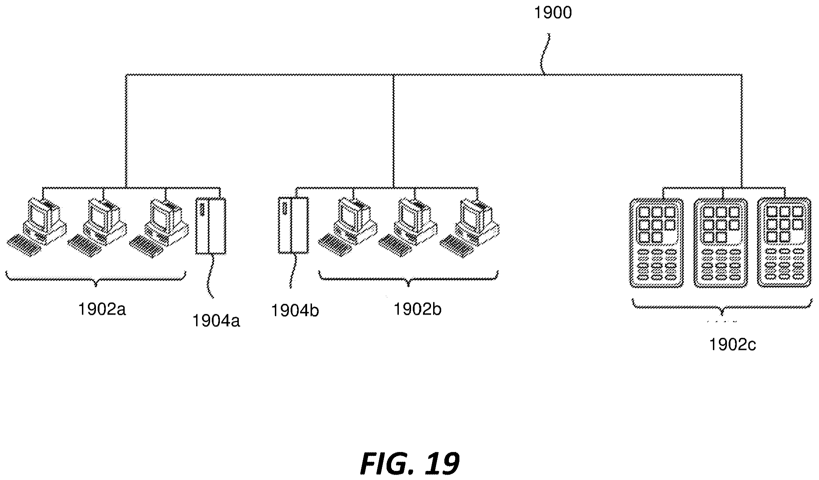

**FIG. 19** is a diagram showing a network **1900** with a plurality of computer systems **1902a,** and **1902b,** a plurality of cell phones and personal data assistants **1902c,** and Network Attached Storage (NAS) **1904a,** and **1904b.** In example embodiments, systems **1902a, 1902b,** and **1902c** can manage data storage and optimize data access for data stored in Network Attached Storage (NAS) **1904a** and **1904b.** A mathematical model can be used for the data and be evaluated using distributed parallel processing across computer systems **1902a,** and **1902b,** and cell phone and personal data assistant systems **1902c.** Computer systems **1902a,** and **1902b,** and cell phone and personal data assistant systems **1902c** can also provide parallel processing for adaptive data restructuring of the data stored in Network Attached Storage (NAS) **1904a** and **1904b.** **FIG. 19** illustrates an example only, and a wide variety of other computer architectures and systems can be used in conjunction with the various embodiments of the present disclosure. For example, a blade server can be used to provide parallel processing. Processor blades can be connected through a back plane to provide parallel processing. Storage can also be connected to the back plane or as Network Attached Storage (NAS) through a separate network interface.

In some example embodiments, processors can maintain separate memory spaces and transmit data through network interfaces, back plane or other connectors for parallel processing by other processors. In other instances, some or all of the processors can use a shared virtual address memory space.

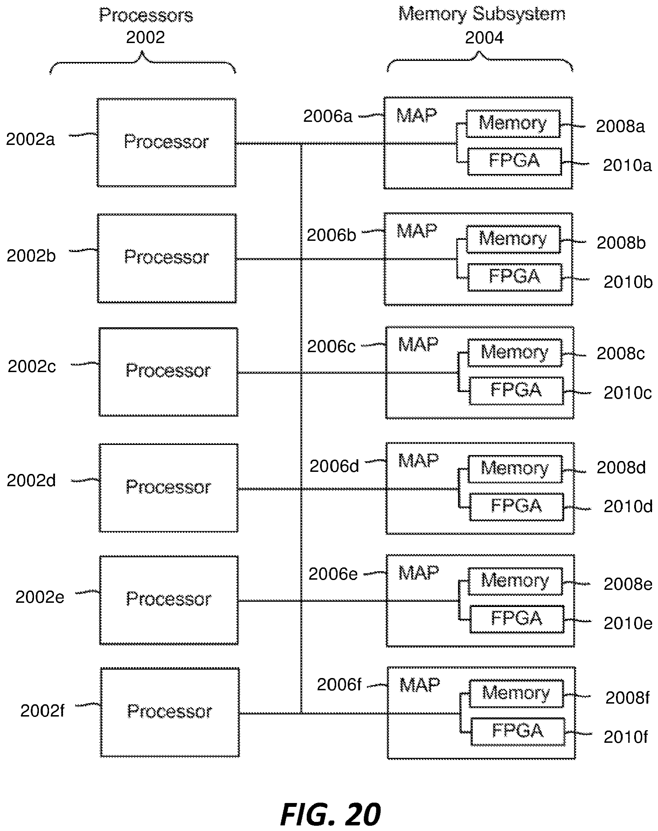

**FIG. 20** is a block diagram of a multiprocessor computer system **2000** using a shared virtual address memory space in accordance with an example embodiment. The system includes a plurality of processors **2002a-f** that can access a shared memory subsystem **2004.** The system incorporates a plurality of programmable hardware memory algorithm processors (MAPs) **2006a-f** in the memory subsystem **2004.** Each MAP **2006a-f** can comprise a memory **2008a-f** and one or more field programmable gate arrays (FPGAs) **2010a-f.** The MAP provides a configurable functional unit and particular algorithms or portions of algorithms can be provided to the FPGAs **2010a-f** for processing in close coordination with a respective processor. For example, the MAPs can be used to evaluate algebraic expressions regarding the data model and to perform adaptive data restructuring in example embodiments. In this example, each MAP is globally accessible by all of the processors for these purposes. In one configuration, each MAP can use Direct Memory Access (DMA) to access an associated memory **2008a-f,** allowing it to execute tasks independently of, and asynchronously from, the respective microprocessor **2002a-f.** In this configuration, a MAP can feed results directly to another MAP for pipelining and parallel execution of algorithms.

The above computer architectures and systems are examples only, and a wide variety of other computer, cell phone, and personal data assistant architectures and systems can be used in connection with example embodiments, including systems using any combination of general processors, co-processors, FPGAs and other programmable logic devices, system on chips (SOCs), application specific integrated circuits (ASICs), and other processing and logic elements. In some instances, all or part of the computer system can be implemented in software or hardware. Any variety of data storage media can be used in connection with example instances, including random access memory, hard drives, flash memory, tape drives, disk arrays, Network Attached Storage (NAS) and other local or distributed data storage devices and systems.

In example embodiments, the computer system can be implemented using software modules executing on any of the above or other computer architectures and systems. In other instances, the functions of the system can be implemented partially or completely in firmware, programmable logic devices such as field programmable gate arrays (FPGAs), system on chips (SOCs), application specific integrated circuits (ASICs), or other processing and logic elements. For example, the Set Processor and Optimizer can be implemented with hardware acceleration through the use of a hardware accelerator card, such as an accelerator card.

Provided herein are methods for storing information, comprising: converting an item of information in the form of at least one digital sequence to at least one nucleic acid sequence; providing a flexible structure having a surface; synthesizing a plurality of oligonucleotides having predetermined sequences collectively encoding for the at least one nucleic acid sequence, wherein the plurality of oligonucleotides comprises at least about 100,000 oligonucleotides, and wherein the plurality of oligonucleotides extends from the surface of the flexible structure; and storing the plurality of oligonucleotides. Further provided herein are methods wherein synthesizing comprises: depositing nucleosides on the surface at predetermined locations; and moving at least a portion of the flexible structure through a bath or emissions from a spray bar. Further provided herein are methods wherein the bath or emissions from a spray bar expose the surface of the structure to an oxidizing reagent or a deblocking reagent. Further provided herein are methods wherein synthesizing further comprises capping the nucleosides deposited on the surface. Further provided herein are methods wherein the nucleosides comprise a nucleoside phosphoramidite. Further provided herein are methods wherein the flexible structure comprises a reel-to-reel tape or a continuous tape. Further provided herein are methods wherein the flexible structure comprises a thermoplastic material. Further provided herein are methods wherein the thermoplastic material comprises a polyaryletherketone. Further provided herein are methods wherein the polyaryletherketone is polyetherketone, polyetherketoneketone, poly(ether ether ketone ketone), polyether ether ketone or polyetherketoneetherketoneketone. Further provided herein are methods wherein the flexible structure comprises nylon, nitrocellulose, polypropylene, polycarbonate, polyethylene, polyurethane, polystyrene, acetal, acrylic, acrylonitrile, butadiene styrene, polyethylene terephthalate, polymethyl methacrylate, polyvinyl chloride, transparent PVC foil, Poly(methyl methacrylate), styrenic polymer, fluorine-containing polymers, polyethersulfone or polyimide. Further provided herein are methods wherein each oligonucleotide of the plurality of oligonucleotides comprises from 50 to 500 bases in length. Further provided herein are methods wherein the plurality of oligonucleotides comprises at least about 10 billion oligonucleotides. Further provided herein are methods wherein at least about 1.75 x 10¹³ nucleobases are synthesized within 24 hours. Further provided herein are methods wherein at least about 262.5 x 10⁹ oligonucleotides are synthesized within 72 hours. Further provided herein are methods wherein the item of information is text information, audio information or visual information. Further provided herein are methods wherein the nucleosides comprise nucleoside phosphoramidite.

Provided herein are methods for storing information, comprising: converting an item of information in the form of at least one digital sequence to at least one nucleic acid sequence; providing a structure having a surface; synthesizing a plurality of oligonucleotides having predetermined sequences collectively encoding for the at least one nucleic acid sequence, wherein the plurality of oligonucleotides comprises at least about 100,000 oligonucleotides, wherein the plurality of oligonucleotides extends from the surface of the structure, and wherein synthesizing comprises: cleaning a surface of the structure; depositing nucleosides on the surface at predetermined locations; oxidizing, deblocking, and optionally capping the nucleosides deposited on the surface; wherein the cleaning, oxidizing, deblocking, and capping comprises moving at least a portion of the flexible structure through a bath or emissions from a spray bar; and storing the plurality of oligonucleotides. Further provided herein are methods wherein the nucleosides comprise nucleoside phosphoramidite.

The following examples are set forth to illustrate more clearly the principle and practice of embodiments disclosed herein to those skilled in the art and are not to be construed as limiting the scope of any claimed embodiments. Unless otherwise stated, all parts and percentages are on a weight basis.

### EXAMPLES

### EXAMPLE 1: Functionalization of a device surface

A device was functionalized to support the attachment and synthesis of a library of oligonucleotides. The device surface was first wet cleaned using a piranha solution comprising 90% H₂SO₄ and 10% H₂O₂ for 20 minutes. The device was rinsed in several beakers with DI water, held under a DI water gooseneck faucet for 5 min, and dried with N₂. The device was subsequently soaked in NH₄OH (1:100; 3 mL:300 mL) for 5 min, rinsed with DI water using a handgun, soaked in three successive beakers with DI water for 1 min each, and then rinsed again with DI water using the handgun. The device was then plasma cleaned by exposing the device surface to O₂. A SAMCO PC-300 instrument was used to plasma etch O₂ at 250 watts for 1 min in downstream mode.

The cleaned device surface was actively functionalized with a solution comprising N-(3-triethoxysilylpropyl)-4-hydroxybutyramide using a YES-1224P vapor deposition oven system with the following parameters: 0.5 to 1 torr, 60 min, 70 °C, 135 °C vaporizer. The device surface was resist coated using a Brewer Science 200X spin coater. SPR^{™} 3612 photoresist was spin coated on the device at 2500 rpm for 40 sec. The device was pre-baked for 30 min at 90 °C on a Brewer hot plate. The device was subjected to photolithography using a Karl Suss MA6 mask aligner instrument. The device was exposed for 2.2 sec and developed for 1 min in MSF 26A. Remaining developer was rinsed with the handgun and the device soaked in water for 5 min. The device was baked for 30 min at 100 °C in the oven, followed by visual inspection for lithography defects using a Nikon L200. A descum process was used to remove residual resist using the SAMCO PC-300 instrument to O₂ plasma etch at 250 watts for 1 min.

The device surface was passively functionalized with a 100 µL solution of perfluorooctyltrichlorosilane mixed with 10 µL light mineral oil. The device was placed in a chamber, pumped for 10 min, and then the valve was closed to the pump and left to stand for 10 min. The chamber was vented to air. The device was resist stripped by performing two soaks for 5 min in 500 mL NMP at 70 °C with ultrasonication at maximum power (9 on Crest system). The device was then soaked for 5 min in 500 mL isopropanol at room temperature with ultrasonication at maximum power. The device was dipped in 300 mL of 200 proof ethanol and blown dry with N₂. The functionalized surface was activated to serve as a support for oligonucleotide synthesis.

### EXAMPLE 2: Synthesis of a 50-mer sequence on an oligonucleotide synthesis device

A two dimensional oligonucleotide synthesis device was assembled into a flowcell, which was connected to a flowcell (Applied Biosystems (ABI394 DNA Synthesizer"). The two-dimensional oligonucleotide synthesis device was uniformly functionalized with N-(3-TRIETHOXYSILYLPROPYL)-4-HYDROXYBUTYRAMIDE (Gelest) and used to synthesize an exemplary oligonucleotide of 50 bp ("50-mer oligonucleotide") using oligonucleotide synthesis methods described herein.

The sequence of the 50-mer was as described in SEQ ID NO.: 1. 5'AGACAATCAACCATTTGGGGTGGACAGCCTTGACCTCTAGACTTCGGCAT##TTTTTTT TTT3' (SEQ ID NO.: 1), where # denotes Thymidine-succinyl hexamide CED phosphoramidite (CLP-2244 from ChemGenes), which is a cleavable linker enabling the release of oligonucleotides from the surface during deprotection.

The synthesis was done using standard DNA synthesis chemistry (coupling, capping, oxidation, and deblocking) according to the protocol in **Table 5** and an ABI synthesizer.

**Table 5: Synthesis Protocol**

| | **Table 5** | |
|---|---|---|
| **General DNA Synthesis Process Name** | **Process Step** | **Time (sec)** |
| **WASH** (Acetonitrile Wash Flow) | Acetonitrile System Flush | 4 |
| | Acetonitrile to Flowcell | 23 |
| | N2 System Flush | 4 |
| | Acetonitrile System Flush | 4 |
| **DNA BASE ADDITION** (Phosphoramidite + Activator Flow) | Activator Manifold Flush | 2 |
| | Activator to Flowcell | 6 |
| | Activator + Phosphoramidite to Flowcell | 6 |
| | Activator to Flowcell | 0.5 |
| | Activator + Phosphoramidite to Flowcell | 5 |
| | Activator to Flowcell | 0.5 |
| | Activator + Phosphoramidite to Flowcell | 5 |
| | Activator to Flowcell | 0.5 |
| | Activator + Phosphoramidite to Flowcell | 5 |
| | Incubate for 25sec | 25 |
| **WASH** (Acetonitrile Wash Flow) | Acetonitrile System Flush | 4 |
| | Acetonitrile to Flowcell | 15 |
| | N2 System Flush | 4 |
| | Acetonitrile System Flush | 4 |
| **DNA BASE ADDITION** (Phosphoramidite + Activator Flow) | Activator Manifold Flush | 2 |
| | Activator to Flowcell | 5 |
| | Activator + Phosphoramidite to Flowcell | 18 |
| | Incubate for 25sec | 25 |
| **WASH** (Acetonitrile Wash Flow) | Acetonitrile System Flush | 4 |
| | Acetonitrile to Flowcell | 15 |
| | N2 System Flush | 4 |
| | Acetonitrile System Flush | 4 |
| **CAPPING** (CapA+B, 1:1, Flow) | CapA+B to Flowcell | 15 |
| **WASH** (Acetonitrile Wash Flow) | Acetonitrile System Flush | 4 |
| | Acetonitrile to Flowcell | 15 |
| | Acetonitrile System Flush | 4 |
| **OXIDATION** (Oxidizer Flow) | Oxidizer to Flowcell | 18 |
| **WASH** (Acetonitrile Wash Flow) | Acetonitrile System Flush | 4 |
| | N2 System Flush | 4 |
| | Acetonitrile System Flush | 4 |
| | Acetonitrile to Flowcell | 15 |
| | Acetonitrile System Flush | 4 |
| | Acetonitrile to Flowcell | 15 |
| | N2 System Flush | 4 |
| | Acetonitrile System Flush | 4 |
| | Acetonitrile to Flowcell | 23 |
| | N2 System Flush | 4 |
| | Acetonitrile System Flush | 4 |
| **DEBLOCKING** (Deblock Flow) | Deblock to Flowcell | 36 |
| **WASH** (Acetonitrile Wash Flow) | Acetonitrile System Flush | 4 |
| | N2 System Flush | 4 |
| | Acetonitrile System Flush | 4 |
| | Acetonitrile to Flowcell | 18 |
| | N2 System Flush | 4.13 |
| | Acetonitrile System Flush | 4.13 |
| | Acetonitrile to Flowcell | 15 |

The phosphoramidite/activator combination was delivered similar to the delivery of bulk reagents through the flowcell. No drying steps were performed as the environment stays "wet" with reagent the entire time.

The flow restrictor was removed from the ABI 394 synthesizer to enable faster flow. Without flow restrictor, flow rates for amidites (0.1M in ACN), Activator, (0.25M Benzoylthiotetrazole ("BTT"; 30-3070-xx from GlenResearch) in ACN), and Ox (0.02M I2 in 20% pyridine, 10% water, and 70% THF) were roughly ~100uL/sec, for acetonitrile ("ACN") and capping reagents (1:1 mix of CapA and CapB, wherein CapA is acetic anhydride in THF/Pyridine and CapB is 16% 1-methylimidizole in THF), roughly ~200uL/sec, and for Deblock (3% dichloroacetic acid in toluene), roughly ~300uL/sec (compared to ~50uL/sec for all reagents with flow restrictor). The time to completely push out Oxidizer was observed, the timing for chemical flow times was adjusted accordingly and an extra ACN wash was introduced between different chemicals. After oligonucleotide synthesis, the chip was deprotected in gaseous ammonia overnight at 75 psi. Five drops of water were applied to the surface to assemble oligonucleotides. The assembled oligonucleotides were then analyzed on a BioAnalyzer small RNA chip (data not shown).

### EXAMPLE 3: Synthesis of a 100-mer sequence on an oligonucleotide synthesis device

The same process as described in Example 2 for the synthesis of the 50-mer sequence was used for the synthesis of a 100-mer oligonucleotide ("100-mer oligonucleotide"; 5' CGGGATCCTTATCGTCATCGTCGTACAGATCCCGACCCATTTGCTGTCCACCAGTCATG CTAGCCATACCATGATGATGATGATGATGAGAACCCCGCAT##TTTTTTTTTT3', where # denotes Thymidine-succinyl hexamide CED phosphoramidite (CLP-2244 from ChemGenes); SEQ ID NO.: 2) on two different silicon chips, the first one uniformly functionalized with N-(3-TRIETHOXYSILYLPROPYL)-4-HYDROXYBUTYRAMIDE and the second one functionalized with 5/95 mix of 11-acetoxyundecyltriethoxysilane and n-decyltriethoxysilane, and the oligonucleotides extracted from the surface were analyzed on a BioAnalyzer instrument (data not shown).

All ten samples from the two chips were further PCR amplified using a forward (5'ATGCGGGGTTCTCATCATC3'; SEQ ID NO.: 3) and a reverse (5'CGGGATCCTTATCGTCATCG3'; SEQ ID NO.: 4) primer in a 50uL PCR mix (25uL NEB Q5 mastermix, 2.5uL 10uM Forward primer, 2.5uL 10uM Reverse primer, 1uL oligonucleotide extracted from the surface, and water up to 50uL) using the following thermalcycling program:
98 °C, 30 sec
98 °C, 10 sec; 63 °C, 10 sec; 72 °C, 10 sec; repeat 12 cycles
72 °C, 2 min

The PCR products were also run on a BioAnalyzer (data not shown), demonstrating sharp peaks at the 100-mer position. Next, the PCR amplified samples were cloned, and Sanger sequenced. **Table 6** summarizes the results from the Sanger sequencing for samples taken from spots 1-5 from chip 1 and for samples taken from spots 6-10 from chip 2.

**Table 6: Sequencing Results**

| **Spot** | **Error rate** | **Cycle efficiency** |
|---|---|---|
| 1 | 1/763 bp | 99.87% |
| 2 | 1/824 bp | 99.88% |
| 3 | 1/780 bp | 99.87% |
| 4 | 1/429 bp | 99.77% |
| 5 | 1/1525 bp | 99.93% |
| 6 | 1/1615 bp | 99.94% |
| 7 | 1/531 bp | 99.81% |
| 8 | 1/1769 bp | 99.94% |
| 9 | 1/854 bp | 99.88% |
| 10 | 1/1451 bp | 99.93% |

Thus, the high quality and uniformity of the synthesized oligonucleotides were repeated on two chips with different surface chemistries. Overall, 89%, corresponding to 233 out of 262 of the 100-mers that were sequenced were perfect sequences with no errors.

**Table 7** summarizes error characteristics for the sequences obtained from the oligonucleotides samples from spots 1-10.

**Table 7: Error Characteristics**

| Sample ID/Spot no. | OSA_ 0046/1 | OSA_0 047/2 | OSA_0 048/3 | OSA_0 049/4 | OSA_0 050/5 | OSA_0 051/6 | OSA_0 052/7 | OSA_0 053/8 | OSA_0 054/9 | OSA_00 55/10 |
|---|---|---|---|---|---|---|---|---|---|---|
| Total Sequences | 32 | 32 | 32 | 32 | 32 | 32 | 32 | 32 | 32 | 32 |
| Sequencing Quality | 25 of 28 | 27 of 27 | 26 of 30 | 21 of 23 | 25 of 26 | 29 of 30 | 27 of 31 | 29 of 31 | 28 of 29 | 25 of 28 |
| Oligo Quality | 23 of 25 | 25 of 27 | 22 of 26 | 18 of 21 | 24 of 25 | 25 of 29 | 22 of 27 | 28 of 29 | 26 of 28 | 20 of 25 |
| ROI Match Count | 2500 | 2698 | 2561 | 2122 | 2499 | 2666 | 2625 | 2899 | 2798 | 2348 |
| ROI Mutation | 2 | 2 | 1 | 3 | 1 | 0 | 2 | 1 | 2 | 1 |
| ROI Multi Base Deletion | 0 | 0 | 0 | 0 | 0 | 0 | 0 | 0 | 0 | 0 |
| ROI Small Insertion | 1 | 0 | 0 | 0 | 0 | 0 | 0 | 0 | 0 | 0 |
| ROI Single Base Deletion | 0 | 0 | 0 | 0 | 0 | 0 | 0 | 0 | 0 | 0 |
| Large Deletion Count | 0 | 0 | 1 | 0 | 0 | 1 | 1 | 0 | 0 | 0 |
| Mutation: G>A | 2 | 2 | 1 | 2 | 1 | 0 | 2 | 1 | 2 | 1 |
| Mutation: T>C | 0 | 0 | 0 | 1 | 0 | 0 | 0 | 0 | 0 | 0 |
| ROI Error Count | 3 | 2 | 2 | 3 | 1 | 1 | 3 | 1 | 2 | 1 |
| ROI Error Rate | Err: ~1 in 834 | Err: ~1 in 1350 | Err: ~1 in 1282 | Err: ~1 in 708 | Err: ~1 in 2500 | Err: ~1 in 2667 | Err: ~1 in 876 | Err: ~1 in 2900 | Err: ~1 in 1400 | Err: ~1 in 2349 |
| ROI Minus Primer Error Rate | MP Err: ~1 in 763 | MP Err: ~1 in 824 | MP Err: ~1 in 780 | MP Err: ~1 in 429 | MP Err: ~1 in 1525 | MP Err: ~1 in 1615 | MP Err: ~1 in 531 | MP Err: ~1 in 1769 | MP Err: ~1 in 854 | MP Err: ~1 in 1451 |

### EXAMPLE 4: Highly accurate DNA-based information storage and assembly

Digital information was selected in the form of binary data totaling about 0.2 GB included content for the Universal Declaration of Human Rights in more than 100 languages, the top 100 books of Project Guttenberg and a seed database. The digital information was encrypted into a nucleic acid-based sequence and divided into strings. Over 10 million non-identical oligonucleotides, each corresponding to a string, were synthesized on a rigid silicon surface in a manner similar to that described in Example 2. Each non-identical oligonucleotide was under equal or less than 200 bases in length. The synthesized oligonucleotides were collected and sequenced and decoded back to digital code, with 100% accuracy for the source digital information, compared to the initial at least one digital sequence.

### EXAMPLE 5: Conversion of digital information to nucleic acid sequence

A computer txt file includes text information. A general purpose computer uses a software program having machine instructions for conversion of the sequence to base 3, 4, or 5 sequence, depending on instructions received. Each number in base 3 is assigned a nucleic acid (e.g., A=0, T=1, C=2). Each number in base 4 is assigned a nucleic acid (e.g., A=0, T=1, C=2, G=3). Alternatively, a base 5 quinary sequence is used, where each number in base 5 is assigned a nucleic acid (e.g., A=0, T=1, C=2, G=3, U=4). A sequence is generated as depicted in **Table 8**. Machine instructions are then provided for de novo synthesis of oligonucleotides encoding the nucleic acid sequence.

**Table 8. Sequence Conversion**

| Text | Jack went up the hill. |
|---|---|
| Binary sequence | 010010100110000101100011011010110010000001110111011001010110111 001110100001000000111010101110000001000000111010001101000011001 010010000001101000011010010110110001101100001011100000110100001 0100000110100001010 |
| Ternary sequence | 101010201100022101010021102012221200101112202210002122002210200 01111221210201120102111212220010111000100200102200222222110022222112 |
| Quaternary sequence | 102212011203122302001313121112321310020013111300020013101220121 10200122012211230123002320031002200310022 |
| Quinary sequence | 332214330133012303013123001030244433343300431224103020320210201 12342341100431241100334213 |

### EXAMPLE 6: Flexible surface having a high density of features

A flexible structure comprising thermoplastic material is coated with a nucleoside coupling reagent. The coating agent is patterned for a high density of features. A portion of the flexible surface is illustrated in **FIG. 14A****.** Each feature has a diameter of 10 um, with a center-to-center distance between two adjacent features of 21 um. The feature size is sufficient to accommodate a sessile drop volume of 0.2 pl during an oligonucleotide synthesis deposition step. The small feature dimensions allow for a high density of oligonucleotides to be synthesized on the surface of the substrate. The feature density is 2.2 billion features / m² (1 feature / 441 x 10⁻¹² m²). A 4.5 m² substrate is manufactured having 10 billion features, each with a 10 um diameter. The flexible structure is optionally placed in a continuous loop system, **FIG. 12A****,** or a reel-to-reel system, **FIG. 12B****,** for oligonucleotide synthesis.

### EXAMPLE 7: Oligonucleotide synthesis on a flexible structure

A flexible structure is prepared comprising a plurality of features on a thermoplastic flexible material. The structure serves as a support for the synthesis of oligonucleotides using an oligonucleotide synthesis device comprising a deposition device. The flexible structure is in the form of a flexible media much like a magnetic reel-to-reel tape.

De novo synthesis operates in a continuous production line manner with the structure travelling through a solvent bath and then beneath a stack of printheads where the phosphoramidites are printed on to a surface of the structure. The flexible structure with the sessile drops deposited on to the surface is rolled into a bath of oxidizing agent, then the tape emerges from the oxidizing bath and is immersed in an acetonitrile wash bath then submerged in a deblock bath. Optionally, the tape is traversed through a capping bath. In an alternative workflow, the flexible structure emerges from the oxidizing bath and is sprayed with acetonitrile in a wash step.

Alternatively, a spray bar is used instead of a liquid bath. In this process, the nucleotides are still deposited on the surface with an inkjet device but the flood steps are now done in a chamber with spray nozzles. For example, the deposition device has 2,048 nozzles that each deposits 100,000 droplets per second at 1 nucleobase per droplet. There is a sequential ordering of spray nozzles to mimic the ordering of the flood steps in standard phosphoramidite chemistry. This technique provides for easily changing the chemicals loaded in the spray bar to accommodate different process steps. Oligonucleotides are deprotected or cleaved in the same manner as described in Example 2.

For each deposition device, more than 1.75 x 10¹³ nucleobases are deposited on the structure per day. A plurality of 200 nucleobase oligonucleotides is synthesized. In 3 days, at a rate of 1.75 x 10¹³ bases per day, 262.5 x 10⁹ oligonucleotides are synthesized.

### EXAMPLE 8: Selection bioencryption

Machine instructions are received for desired items of information for conversion and for one or more categories of bioencryption selected from enzymatic based (e.g., CRISPR/Cas complex and restriction enzyme digest), electromagnetic radiation based (e.g., photolysis and photodetection), chemical cleavage (e.g, gaseous ammonia or methylamine treatment to cleave Thymidine-succinyl hexamide CED phosphoramidite (CLP-2244 from ChemGenes)), and affinity based (e.g., a sequence tag for hybridization, or incorporation of modified nucleotides with enhanced affinity to a capture reagent) forms of bioencryption. Following receipt of a particular bioencryption selection, a program module performs the step of converting the items of information to nucleic acid sequences and applying design instructions for design of a bioencrypted version of the sequence. A specific encryption subtype within the bioencryption category is selected. Synthesis instructions are then provided to a material deposition device for de novo synthesis of oligonucleotides.

### EXAMPLE 9: Selected biodecryrption

Machine instructions are provided for application of one or more categories of biodecryption selected from enzymatic based (e.g., CRISPR/Cas complex or restriction enzyme digest), electromagnetic radiation based (e.g., photolysis or photodetection), chemical cleavage based (e.g, gaseous ammonia or methylamine treatment to cleave Thymidine-succinyl hexamide CED phosphoramidite (CLP-2244 from ChemGenes)), and affinity based (e.g., a sequence tag for hybridization, or incorporation of modified nucleotides with enhanced affinity to a capture reagent) biodecryption. Following receipt of a particular biodecryption selection, a program module performs the step of releasing the modulatory agent(s) for enrichment of the oligonucleotides. Following enrichment, the oligonucleotides are sequenced, optionally aligned to a longer nucleic acid sequence, and converted to a digital sequence corresponding to an item of information.

### EXAMPLE 10: Biological encryption and decryption of a DNA sequence with CRISPR/Cas9

A digital sequence encoding for an item of information is received. The digital sequence is then converted to a nucleic acid sequence. The nucleic acid sequence is encrypted in a larger population of nucleic acid sequences. The encryption process involves adding "junk" regions for detection and removal by a CRISPR/Cas9 complex. Nucleic acid sequences are synthesized as in Examples 2-3.

The population of nucleic acid sequences comprising the encrypted nucleic acid sequences are mixed with Cas9 and gRNAs in Cas9 buffer and incubated at 37 °C for 2 hours. Cas9 is then inactivated and removed by purification. The purified sample is then analyzed by next generation sequencing.

### EXAMPLE 11: Biological encryption and decryption of a DNA sequence with CRISPR/Cas9 comprising sequence swapping

A digital sequence encoding for an item of information is received, the digital sequence is converted to nucleic acid sequences. The nucleic acid sequences are encrypted by addition of a specific sequence using CRISPR/Cas9 system and guide RNA sequence. Nucleic acid sequences are synthesized as in Examples 2-3.

The nucleic acid sequences are then mixed with fluorescent-tagged probes that are complementary to the swapped sequences. Nucleic acid sequences identified by the fluorescent-tagged probes are removed from the population.

### EXAMPLE 12: Biological encryption and decryption of a DNA sequence using restriction enzyme digest

A digital sequence encoding for an item of information is received, and the digital sequence is converted to nucleic acid sequences. A population of nucleic acid sequences is encrypted by addition of a specific sequence that is recognized by the restriction enzyme EcoRI. Nucleic acid sequences are synthesized, as in Examples 2-3, and stored.

The nucleic acid sequences are incubated with EcoRI. Encrypted nucleic acid sequences comprising the EcoRI recognition site are cleaved. Following cleavage of the encrypted nucleic acid sequences, sequences with complementary overhangs are hybridized and ligated to the released DNA. The ligated complex is then isolated and the purified sample is sequenced and the original digital information is assembled.

### EXAMPLE 13: Biological encryption and decryption of a DNA sequence using photolysis

A digital sequence encoding for an item of information is received, and the digital sequence is converted to nucleic acid sequences. A population of nucleic acid sequences is designed to include nucleobases that are photocleavable. Nucleic acid sequences are synthesized as in Examples 2-3, and stored.

UV-B irradiation of 280 nm is applied to the nucleic acid sequences. Encrypted nucleic acid sequences comprising the photocleavable site are cleaved and removed. The nucleic acid sequences are then collected sequenced. Alternatively, nucleic acid sequences are released from the surface of a structure, such as by ammonia gas cleavage, and then exposed to electromagnetic radiation to provide for breaks in the nucleotide sequences. Portions of the population are enriched, such as by pull down assay using beads having complementary capture probes bound thereto, PCR using primers selected to only amplify target sequence, or size exclusion chromatography. Enriched nucleic acids are then sequenced, converted to digital sequence, and an item of information is received.

### EXAMPLE 14: Biological encryption and decryption of a DNA sequence using chemical enrichment

A digital sequence encoding for an item of information is received, and the digital sequence is converted to nucleic acid sequences. A population of nucleic acid sequences is encrypted by addition of a specific sequence (e.g., Thymidine-succinyl hexamide CED phosphoramidite (CLP-2244 from ChemGenes)) that is chemically cleavable by ammonia gas. Nucleic acid sequences are synthesized as in Examples 2-3.

Ammonia gas is applied to the nucleic acid sequences. Encrypted nucleic acid sequences comprising the chemically cleavable sequence are released and enriched from the population using enrichment methods described herein. Enriched nucleic acids are then sequenced, converted to digital sequence, and an item of information is received.

### EXAMPLE 15: Biological encryption and decryption of a DNA sequence using nucleic acid probes comprising biotin

A digital sequence encoding for an item of information is received, and the digital sequence is converted to nucleic acid sequences. A population of nucleic acid sequences is encrypted by design of predetermined residues to comprise biotin containing nucleobases. Nucleic acid sequences are synthesized as in Examples 2-3.

The nucleic acid sequences are cleaved from a structure, and mixed with streptavidin containing beads. The nucleic acid sequences are then incubated with streptavidin magnetic beads. Nucleic acid sequences comprising biotin are pulled down by the magnetic beads. Enriched nucleic acids are then sequenced, converted to digital sequence, and an item of information is received.

### EXAMPLE 16: Biological encryption and decryption of a DNA sequence using photodetection

A digital sequence encoding for an item of information is received, and the digital sequence is converted to nucleic acid sequences. A population of nucleic acid sequences is encrypted by design to include specific sequence that is recognized by Alexa488-tagged nucleic acid probes. Nucleic acid sequences are synthesized as in Examples 2-3.

The nucleic acid sequences are released from a structure and mixed with Alexa488-tagged nucleic acid probes. The nucleic acid sequences are then sorted by fluorescence intensity. Nucleic acid sequences that are tagged with Alexa488-tagged nucleic acid probes are further analyzed. Probe bound nucleic acids are then sequenced, converted to digital sequence, and an item of information is received.

### EXAMPLE 17: Biological encryption and decryption of a DNA sequence using modified nucleotides

A digital sequence encoding for an item of information is received, the digital sequence is converted to nucleic acid sequences. A population of nucleic acid sequences is encrypted by designing for the addition of predetermined nucleobases comprising peptide nucleic acid (PNA) at predetermined locations and for the design of restriction enzyme recognitions sizes to excise PNA containing sections. Nucleic acid sequences are synthesized as in Examples 2-3.

The nucleic acid sequences are released, subject to restriction enzyme digestion, and then amplified by PCR. Nucleic acid sequences comprising PNAs are unable to be amplified. Enriched, amplified nucleic acids are then sequenced, converted to digital sequence, and the item of information is received.

### EXAMPLE 18: Biological encryption and decryption of a DNA sequence using CRISPR/Cas9 and chemical cleavage

A digital sequence encoding for an item of information is received, the digital sequence is converted to nucleic acid sequences. A population of nucleic acid sequences is encrypted by addition of a specific sequence using CRISPR/Cas9 and guide RNA sequence. The CRISPR/Cas9 system introduces a chemically cleavable site in the nucleic acid sequences at preselected locations. Nucleic acid sequences are synthesized as in Examples 2-3.

Ammonia gas is applied to the nucleic acid sequences. Encrypted nucleic acid sequences comprising the chemically cleavable site are cleaved and removed by size exclusion purification and analyzed by next generation sequencing.

While preferred embodiments of the present invention have been shown and described herein, it will be obvious to those skilled in the art that such embodiments are provided by way of example only. Numerous variations, changes, and substitutions will now occur to those skilled in the art without departing from the invention as set out in appended claims 1-14. It should be understood that various alternatives to the embodiments of the invention described herein may be employed in practicing the invention. It is intended that the following appended claims 1-14 define the scope of protection of the present invention.

## Claims

1. A system for retrieving information, the system comprising:
a storage unit comprising a plurality of oligonucleotides on a surface;
a deposition unit configured for applying an enzymatic based bioencryption format to biodecrypt the plurality of oligonucleotides (303), the enzymatic based bioencryption format configured for biodecryption by exposure of the plurality of oligonucleotides to a nuclease complex (307);
a sequencing unit configured for sequencing the plurality of oligonucleotides to obtain a nucleic acid sequence (305); and
a processor unit configured to convert the nucleic acid sequence to a digital sequence (309), wherein the digital sequence represents at least one item of information.

2. The system of claim 1, wherein the enzymatic bioencryption format is a first bioencryption format, the system further comprising an additional bioencryption format including at least one of an additional enzymatic based bioencryption, chemical based bioencryption, electromagnetic based bioencryption, or affinity based bioencryption.

3. The system of any claim of claims 1 or claim 2, wherein the nuclease complex includes a CRISPR/Cas complex.

4. The system of claim 1 or claim 2, wherein the enzymatic based bioencryption format includes ,one or more enzymes, the one or more enzymes including a repair enzyme and/or a restriction enzyme.

5. The system of claim 2, wherein the additional bioencryption format includes electromagnetic based bioencryption, and the deposition unit is configured to apply electromagnetic radiation to the plurality of oligonucleotides, wherein the electromagnetic radiation has a wavelength of about 0.01 nm to about 400 nm.

6. The system of claim 2, wherein the additional bioencryption format includes chemical based bioencryption, and the deposition unit is configured to apply a gas to the plurality of oligonucleotides, and the gas comprises ammonia, methylamine, or both.

7. The system of claim 2, wherein the additional bioencryption format includes affinity based bioencryption, and the deposition unit is configured to detect an affinity tag using light absorption, florescence, chemiluminescence, electrochemiluminescence, mass, or charge.

8. The system of claim 7, wherein affinity tag comprises biotin, digoxigenin, Ni-nitrilotriacetic acid, desthiobiotin, histidine, polyhistidine, myc, hemagglutinin, FLAG, a tandem affinity purification (TAP) tag, glutathione S transferase (GST), a polynucleotide, an aptamer, an antigen, or an antibody.

9. The system of claim 7, wherein the affinity tag comprises a magnetically susceptible material.

10. The system of any claim of claims 1-9, wherein the surface comprises encryption markings.

11. The system of any claim of claims 1-10, wherein the item of information includes a .txt file, a .pdf file, a .doc file, a docx file, a .ppt file, a pptx file, an .xls file, an .xlsx file, a .rtf file, a .jpg file, a .gif file, a .psd file, a .bmp file, a .tiff file, a .png file, and/or an .mpeg. file.

12. The system of any claim of claims 1-11, wherein the at least one item of information comprises audio or visual information.

13. The system of any claim of claims 1-12, wherein the at least one item of information comprises at least 1 gigabyte.

14. The system of any claim of claims 1-13, wherein the plurality of oligonucleotides comprises at least 100,000 oligonucleotides.

## Patentansprüche

1. System zum Abrufen von Informationen, wobei das System Folgendes umfasst:
eine Speichereinheit, die eine Mehrzahl von Oligonukleotiden auf einer Oberfläche umfasst;
eine Abscheidungseinheit, die dazu ausgebildet ist, ein enzymatisch basiertes Bioverschlüsselungsformat anzuwenden, um die Mehrzahl von Oligonukleotiden (303) zu bioentschlüsseln, wobei das enzymatisch basierte Bioverschlüsselungsformat zur Bioentschlüsselung durch Exposition der Mehrzahl von Oligonukleotiden gegenüber einem Nukleasekomplex (307) ausgebildet ist;
eine Sequenziereinheit, die zum Sequenzieren der Mehrzahl von Oligonukleotiden ausgebildet ist, um eine Nukleinsäuresequenz (305) zu erhalten; und
eine Prozessoreinheit, die dazu ausgebildet ist, die Nukleinsäuresequenz in eine digitale Sequenz (309) umzuwandeln, wobei die digitale Sequenz mindestens ein Informationselement darstellt.

2. System nach Anspruch 1, wobei das enzymatische Bioverschlüsselungsformat ein erstes Bioverschlüsselungsformat ist, wobei das System ferner ein zusätzliches Bioverschlüsselungsformat umfasst, das mindestens eine von einer zusätzlichen enzymatisch basierten Bioverschlüsselung, einer chemisch basierten Bioverschlüsselung, einer elektromagnetisch basierten Bioverschlüsselung oder einer affinitätsbasierten Bioverschlüsselung beinhaltet.

3. System nach einem der Ansprüche 1 oder 2, wobei der Nukleasekomplex einen CRISPR/Cas-Komplex beinhaltet.

4. System nach Anspruch 1 oder Anspruch 2, wobei das enzymatisch basierte Bioverschlüsselungsformat ein oder mehrere Enzyme beinhaltet, wobei das eine oder die mehreren Enzyme ein Reparaturenzym und/oder ein Restriktionsenzym beinhalten.

5. System nach Anspruch 2, wobei das zusätzliche Bioverschlüsselungsformat eine elektromagnetisch basierte Bioverschlüsselung beinhaltet und die Abscheidungseinheit dazu ausgebildet ist, elektromagnetische Strahlung auf die Mehrzahl von Oligonukleotiden einwirken zu lassen, wobei die elektromagnetische Strahlung eine Wellenlänge von etwa 0,01 nm bis etwa 400 nm aufweist.

6. System nach Anspruch 2, wobei das zusätzliche Bioverschlüsselungsformat eine chemisch basierte Bioverschlüsselung beinhaltet und die Abscheidungseinheit dazu ausgebildet ist, ein Gas auf die Mehrzahl von Oligonukleotiden einwirken zu lassen, und das Gas Ammoniak, Methylamin oder beides umfasst.

7. System nach Anspruch 2, wobei das zusätzliche Bioverschlüsselungsformat eine affinitätsbasierte Bioverschlüsselung beinhaltet und die Abscheidungseinheit dazu ausgebildet ist, einen Affinitäts-Tag mittels Lichtabsorption, Fluoreszenz, Chemilumineszenz, Elektrochemilumineszenz, Masse oder Ladung zu detektieren.

8. System nach Anspruch 7, wobei der Affinitäts-Tag Biotin, Digoxigenin, Ni-Nitrilotriessigsäure, Desthiobiotin, Histidin, Polyhistidin, Myc, Hämagglutinin, FLAG, einen Tandem-Affinitätsreinigungs-Tag (TAP-Tag), Glutathion-S-Transferase (GST), ein Polynukleotid, ein Aptamer, ein Antigen oder einen Antikörper umfasst.

9. System nach Anspruch 7, wobei der Affinitäts-Tag ein magnetisch suszeptibles Material umfasst.

10. System nach einem der Ansprüche 1 bis 9, wobei die Oberfläche Verschlüsselungsmarkierungen umfasst.

11. System nach einem der Ansprüche 1 bis 10, wobei das Informationselement eine .txt-Datei, eine .pdf-Datei, eine .doc-Datei, eine docx-Datei, eine .ppt-Datei, eine pptx-Datei, eine .xls-Datei, eine .xlsx-Datei, eine .rtf-Datei, eine .jpg-Datei, eine .gif-Datei, eine .psd-Datei, eine .bmp-Datei, eine .tiff-Datei, eine .png-Datei und/oder eine .mpeg.-Datei beinhaltet.

12. System nach einem der Ansprüche 1 bis 11, wobei das mindestens eine Informationselement Audioinformationen oder visuelle Informationen umfasst.

13. System nach einem der Ansprüche 1 bis 12, wobei das mindestens eine Informationselement mindestens 1 Gigabyte umfasst.

14. System nach einem der Ansprüche 1 bis 13, wobei die Mehrzahl von Oligonukleotiden mindestens 100.000 Oligonukleotide umfasst.

## Revendications

1. Système de récupération d'informations, le système comprenant :
une unité de stockage comprenant une pluralité d'oligonucléotides sur une surface ;
une unité de dépôt configurée pour appliquer un format de biochiffrement à base enzymatique afin de biodéchiffrer la pluralité d'oligonucléotides (303), le format de biochiffrement à base enzymatique étant configuré pour un biodéchiffrement par exposition de la pluralité d'oligonucléotides à un complexe de nucléase (307) ;
une unité de séquençage configurée pour séquencer la pluralité d'oligonucléotides afin d'obtenir une séquence d'acide nucléique (305) ; et
une unité processeur configurée pour convertir la séquence d'acide nucléique en une séquence numérique (309), dans lequel la séquence numérique représente au moins un élément d'information.

2. Système selon la revendication 1, dans lequel le format de biochiffrement enzymatique est un premier format de biochiffrement, le système comprenant en outre un format de biochiffrement supplémentaire incluant au moins l'un parmi un biochiffrement supplémentaire à base enzymatique, un biochiffrement à base chimique, un biochiffrement à base électromagnétique ou un biochiffrement à base d'affinité.

3. Système selon l'une quelconque des revendications 1 ou 2, dans lequel le complexe de nucléase inclut un complexe CRISPR/Cas.

4. Système selon la revendication 1 ou la revendication 2, dans lequel le format de biochiffrement à base enzymatique inclut une ou plusieurs enzymes, la ou les enzymes incluant une enzyme de réparation et/ou une enzyme de restriction.

5. Système selon la revendication 2, dans lequel le format de biochiffrement supplémentaire inclut un biochiffrement à base électromagnétique, et l'unité de dépôt est configurée pour appliquer un rayonnement électromagnétique à la pluralité d'oligonucléotides, dans lequel le rayonnement électromagnétique a une longueur d'onde d'environ 0,01 nm à environ 400 nm.

6. Système selon la revendication 2, dans lequel le format de biochiffrement supplémentaire inclut un biochiffrement à base chimique, et l'unité de dépôt est configurée pour appliquer un gaz à la pluralité d'oligonucléotides, et le gaz comprend de l'ammoniac, de la méthylamine ou les deux.

7. Système selon la revendication 2, dans lequel le format de biochiffrement supplémentaire inclut un biochiffrement à base d'affinité, et l'unité de dépôt est configurée pour détecter une étiquette d'affinité en utilisant l'absorption de lumière, la fluorescence, la chimiluminescence, l'électrochimiluminescence, la masse ou la charge.

8. Système selon la revendication 7, dans lequel l'étiquette d'affinité comprend de la biotine, de la digoxigénine, de l'acide Ni-nitrilotriacétique, de la desthiobiotine, de l'histidine, de la polyhistidine, myc, de l'hémagglutinine, FLAG, une étiquette de purification par affinité en tandem (TAP), de la glutathion S-transférase (GST), un polynucléotide, un aptamère, un antigène ou un anticorps.

9. Système selon la revendication 7, dans lequel l'étiquette d'affinité comprend un matériau magnétiquement susceptible.

10. Système selon l'une quelconque des revendications 1 à 9, dans lequel la surface comprend des marquages de chiffrement.

11. Système selon l'une quelconque des revendications 1 à 10, dans lequel l'élément d'information inclut un fichier .txt, un fichier .pdf, un fichier .doc, un fichier docx, un fichier .ppt, un fichier pptx, un fichier .xls, un fichier .xlsx, un fichier .rtf, un fichier .jpg, un fichier .gif, un fichier .psd, un fichier .bmp, un fichier .tiff, un fichier .png et/ou un fichier .mpeg.

12. Système selon l'une quelconque des revendications 1 à 11, dans lequel l'au moins un élément d'information comprend des informations audio ou visuelles.

13. Système selon l'une quelconque des revendications 1 à 12, dans lequel l'au moins un élément d'information comprend au moins 1 gigaoctet.

14. Système selon l'une quelconque des revendications 1 à 13, dans lequel la pluralité d'oligonucléotides comprend au moins 100 000 oligonucléotides.
